(19) Europäisches Patentamt
European Patent Office
Office européen des brevets

(11) EP 0 663 902 B1

(12) # EUROPÄISCHE PATENTSCHRIFT

(45) Veröffentlichungstag und Bekanntmachung des Hinweises auf die Patenterteilung:
**11.03.1998 Patentblatt 1998/11**

(21) Anmeldenummer: **93922944.9**

(22) Anmeldetag: **06.10.1993**

(51) Int Cl.[6]: **C07C 401/00**, A61K 31/59

(86) Internationale Anmeldenummer:
**PCT/EP93/02814**

(87) Internationale Veröffentlichungsnummer:
**WO 94/07853 (14.04.1994 Gazette 1994/09)**

(54) **25-CARBONSÄURE-DERIVATE IN DER VITAMIN D-REIHE, DIESE DERIVATE ENTHALTENDE PHARMAZEUTISCHE PRÄPARATE SOWIE DEREN VERWENDUNG ZUR HERSTELLUNG VON ARZNEIMITTELN**

25-CARBOXYLIC ACID DERIVATIVES IN THE VITAMIN D SERIES, PHARMACEUTICAL PREPARATIONS CONTAINING THESE DERIVATIVES AND THEIR USE IN THE MANUFACTURE OF MEDICINES

DERIVES D'ACIDE CARBOXYLIQUE 25 DE LA SERIE DE LA VITAMINE D, PREPARATIONS PHARMACEUTIQUES CONTENANT CES DERIVES ET LEUR UTILISATION POUR PREPARER DES MEDICAMENTS

(84) Benannte Vertragsstaaten:
**AT BE CH DE DK ES FR GB GR IE IT LI LU MC NL PT SE**

(30) Priorität: **06.10.1992 DE 4234382**
**18.05.1993 DE 4317415**

(43) Veröffentlichungstag der Anmeldung:
**26.07.1995 Patentblatt 1995/30**

(73) Patentinhaber: **SCHERING AKTIENGESELLSCHAFT**
**13342 Berlin (DE)**

(72) Erfinder:
- **STEINMEYER, Andreas**
  **D-14052 Berlin (DE)**
- **KIRSCH, Gerald**
  **D-14199 Berlin (DE)**
- **NEEF, Günter**
  **D-10711 Berlin (DE)**
- **SCHWARZ, Katica**
  **D-10585 Berlin (DE)**
- **THIEROFF-EKERDT, Ruth**
  **D-13469 Berlin (DE)**
- **WIESINGER, Herbert**
  **D-10781 Berlin (DE)**
- **HABEREY, Martin**
  **D-12247 Berlin (DE)**

(56) Entgegenhaltungen:
**EP-A- 0 421 561** **WO-A-91/00271**
**WO-A-93/09093**

- **PROCEEDINGS OF THE NATIONAL ACADEMY OF SCIENCES OF THE UNITED STATES OF AMERICA Bd. 84, Nr. 9 , Mai 1987 , WASHINGTON, US Seiten 2610 - 2614 V. K. OSTREM ET AL '24- and 26-homo-1,25-dihydroxyvitymin D3: Preferential activity in inducing differentiation of human leukemia cells HL-60 in vitro.'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 663 902 B1

## Beschreibung

Die vorliegende Erfindung betrifft 25-Carbonsäure-Derivate der allgemeinen Formel **I**,

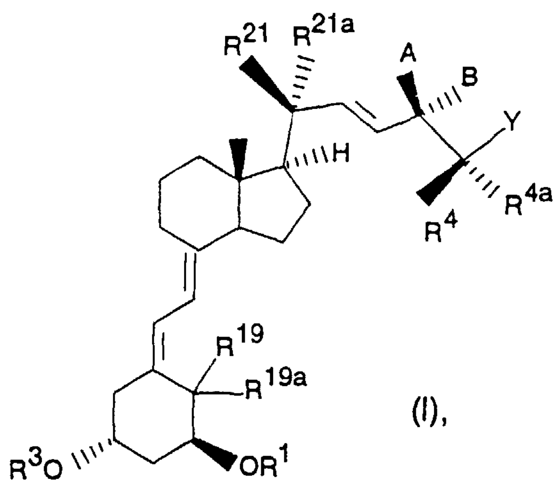

(I),

worin $R^1$ und $R^3$ unabhängig voneinander je ein Wasserstoffatom, eine gerad- oder verzweigtkettige gesättigte Alkanoylgruppe mit 1 bis 9 Kohlenstoffatomen oder eine Aroylgruppe,

$R^{19}$ und $R^{19a}$ je ein Wasserstoffatom oder gemeinsam eine exocyclische Methylengruppe,
A und B gemeinsam ein Ketosauerstoffatom oder A eine Gruppe $OR^{24}$ und B ein Wasserstoffatom oder A ein Wasserstoffatom und B eine Gruppe $OR^{24}$, wobei $R^{24}$ ein Wasserstoffatom oder eine gerad- oder verzweigtkettige gesättigte Alkanoylgruppe mit bis zu 9 Kohlenstoffatomen oder eine Aroylgruppe ist,
$R^{21}$ und $R^{21a}$ unabhängig voneinander ein Wasserstoffatom, ein Chlor- oder Fluoratom, eine Alkylgruppe mit 1 bis 4 Kohlenstoffatomen, gemeinsam eine Methylengruppe, gemeinsam mit dem Kohlenstoffatom 20 einen 3-7 gliedrigen, gesättigten oder ungesättigten carbocyclischen Ring,
$R^4$ und $R^{4a}$ gleichzeitig je ein Wasserstoffatom, ein Chlor- oder Fluoratom, eine Trifluormethylgruppe, einen gerad- oder verzweigtkettigen, gesättigten oder ungesättigten Kohlenwasserstoffrest mit bis zu 4 Kohlenstoffatomen oder $R^4$ und
$R^{4a}$ gemeinsam mit dem Kohlenstoffatom 25 einen 3- bis 7-gliedrigen, gesättigten oder ungesättigten carbocylischen Ring sowie Y einen der Reste $-C(O)NR^5R^{5'}$,$-C(O)OR^6$, -CN, wobei $R^5$ und $R^{5'}$ unabhängig voneinander und $R^6$ je für ein Wasserstoffatom oder eine lineare oder verzweigte Alkylgruppe mit bis zu 8 Kohlenstoffatomen sowie $R^6$ zusätzlich für einen ungesättigten, linearen oder verzweigten Kohlenwasserstoffrest mit 3 bis 8 Kohlenstoffatomen oder für die Gruppe $-(CH_2)_m-CH(CH_2)_n$ mit m = 0 oder 1 und n = 2, 3, 4, 5 oder 6 und wenn m = 1 zusätzlich mit n = 1 stehen,

bedeuten,
sowie Verfahren zu ihrer Herstellung, Zwischenprodukte für dieses Verfahren, pharmazeutische Präparate, die diese Verbindungen enthalten sowie deren Verwendung zur Herstellung von Arzneimitteln.

Vorzugsweise steht für die Reste $R^1$, $R^3$ und $R^{24}$ jeweils ein Wasserstoffatom. Die als Reste $R^1$, $R^3$ und $R^{24}$ möglichen Alkanoylgruppen sind von gesättigten Carbonsäuren, insbesondere der Essigsäure, Propionsäure, Buttersäure, iso-Buttersäure, Pivalinsäure oder Valeriansäure abgeleitet.
Als Aroylgruppe ist in erster Linie der Benzoylrest zu nennen.
Die Reste $R^4$ und $R^{4a}$ sind vorzugsweise Methyl-, Ethyl-,n-Propyl-, n-Butyl-, i-Propyl-, i-Butyl- und tert.-Butylreste. Auch die entsprechenden ungesättigten Reste die Doppel- und/oder Dreifachbindungen enthalten können, kommen infrage.
Von den Resten $R^{21}$ und $R^{21a}$ hat einer vorzugsweise ebenfalls eine dieser Bedeutungen und der andere Rest steht für eine Methylgruppe.
Für $R^{21}$ steht auch vorzugsweise ein Wasserstoffatom und für $R^{21a}$ eine Methylgruppe; in diesem Falle ist außerdem eine ß-ständige 24-Hydroxygruppe bevorzugt.
$R^{21}$ und $R^{21a}$ sind weiterhin bevorzugt zusammen eine Methylengruppe oder sie bilden gemeinsam mit dem Kohlenstoffatom 20 einen Cyclopropylring. Weiterhin gelten folgende bevorzugte Substitutionsmuster:

$R^{21}$=F und $R^{21a}$=Methyl sowie $R^{21}$=Methyl und $R^{21a}$=F.
$R^4$ und $R^{4a}$ stehen auch bevorzugt je für eine Methyl- oder Ethylgruppe oder sie bilden gemeinsam mit dem tertiären Kohlenstoffatom 25 einen Cyclopropyl-, -butyl-, -pentyl- oder hexylring.

In den Resten $R^5$, $R^{5'}$ und $R^6$ ist als Alkylgruppe beispielsweise die Methyl-, Ethyl-, n-Propyl, n-Butyl-, i-Propyl-, i-Butyl-, tert.-Butyl- oder auch eine höhere gerad- oder verzweigtkettige Alkylgruppe enthalten.

Außerdem sind folgende Substituentenkombinationen bevorzugt:

Wenn $R^{19}$ und $R^{19a}$ jeweils für ein Wasserstoffatom stehen,
bedeuten
$R^{21}$ ein Wasserstoffatom und $R^{21a}$ eine Methylgruppe,
$R^{21}$ und $R^{21a}$ gemeinsam eine Methylengruppe,
$R^{21}$ und $R^{21a}$ je eine Methylgruppe oder
$R^{21}$ ein Wasserstoffatom und $R^{21a}$ ein Fluoratom oder umgekehrt;

wenn $R^{19}$ und $R^{19a}$ gemeinsam für eine Methylengruppe stehen,
bedeuten

$R^{21}$ ein Wasserstoffatom und $R^{21a}$ eine Methylgruppe,
$R^{21}$ und $R^{21a}$ gemeinsam eine Methylengruppe oder
$R^{21}$ und $R^{21a}$ je eine Methylgruppe.

Besonders bevorzugt gemäß vorliegender Erfindung sind folgende Verbindungen:

*(5Z,7E,22E)-(1S,3R,24R)*-1,3,24-Trihydroxy-9,10-secocholesta-5,7,10(19),22-tetraen-25-carbonsäureethylester
*(5Z,7E,22E)-(1S,3R,24R)*-26,27-Dimethyl-1,3,24-trihydroxy-9,10-secocholesta-5,7,10(19),22-tetraen-25-carbonsäuremethylester
*(5Z,7E,22E)-(1S,3R,24R)*-1,3,24-Trihydroxy-9,10-secocholesta-5,7,10(19),22-tetraen-25-carbonsäure-1-methylethylester
*(5Z,7E,22E)-(1S,3R,24R)*-1,3,24-Trihydroxy-9,10-secocholesta-5,7,10(19),22-tetraen-25-carbonsäuremethylester
*(5Z,7E,22E)-(1S,3R,24S)*-1,3,24-Trihydroxy-9,10-secochola-5,7,10(19),22-tetraen-24-essigsäuremethylester
*(5Z,7E,22E)-(1S,3R,24R)*-1,3,24-Trihydroxy-9,10-secocholesta-5,7,10(19),22-tetraen-25-carbonsäurenitril
*(5Z,7E,22E)-(1S,3R,24R)*-1,3,24-Trihydroxy-9,10-secocholesta-5,7,10(19),22-tetraen-25-carbonsäurepropylester
*(5Z,7E,22E)-(1S,3R,24R)*-1,3,24-Trihydroxy-9, 10-secocholesta-5,7, 10(19),22-tetraen-25-carbonsäure
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäuremethylester
(5*Z*,7*E*,22*E*)-(1*S*,3*R*,24*R*)-1,3,24-Trihydroxy-26,27-cyclo-9,10-secocholesta-5 ,7,10(19),22-tetraen-25-carbonsäureethylester
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäurepropylester
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäurebutylester
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäure-2-methylpropylester.
(5*Z*,7*E*,22*E*)-(1*S*,3*R*,24*R*)-1,3,24-Trihydroxy-26,27-cydo-9,10-secocholesta-5,7,10(19),22-tetraen-25-carbonsäurepentylester
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäurehexylester
(5*Z*,7*E*,22*E*)-(1*S*,3*R*,24*R*)-1,3,24-Trihydroxy-9,10-secocholesta-5,7,10(19),22-tetraen-25-carbonsäuredimethylamid
(5*Z*,7*E*,22*E*)-(1*S*,3*R*,24*R*)-1,3,24-Trihydroxy-9,10-secocholesta-5,7,10(19),22-tetraen-25-carbonsäurediethylamid
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäurediethylamid
(5*Z*,7*E*,22*E*)-(1*S*,3*R*)-1,3-Dihydroxy-24-oxo-26,27-cyclo-9,10-secocholesta-5,7,10(19),22-tetraen-25-carbonsäure-n-butylamid
(5*Z*,7*E*,22*E*)-(1*S*,3*R*,24*R*)-20-Methyl-1,3,24-trihydroxy-9,10-secocholesta-5,7,10(19),22-tetraen-25-carbonsäuremethylester

(5*Z*,7*E*,22*E*)-(1*S*,3*R*,24*S*)-20-Methyl-1,3,24-trihydroxy-9,10-secocholesta-5,7,10(19),22-tetraen-25-carbonsäuremethylester

(5*Z*,7*E*,22*E*)-(1*S*,3*R*,24*R*)-20-Methyl-1,3,24-trihydroxy-9,10-secocholesta-5,7,10(19),22-tetraen-25-carbonsäureethylester

(5*Z*,7*E*,22*E*)-(1*S*,3*R*,24*S*)-20-Methyl-1,3,24-trihydroxy-9,10-secocholesta-5,7,10(19),22-tetraen-25-carbonsäureethylester

(5*Z*,7*E*,22*E*)-(1*S*,3*R*,24*R*)-20-Methyl-1,3,24-trihydroxy-9,10-secocholesta-5,7,10(19),22-tetraen-25-carbonsäurepropylester

(5*Z*,7*E*,22*E*)-(1*S*,3*R*,24*S*)-20-Methyl-1,3,24-trihydroxy-9,10-secocholesta-5,7,10(19),22-tetraen-25-carbonsäurepropylester

(5*Z*,7*E*,22*E*)-(1*S*,3*R*,24*R*)-20-Methyl-1,3,24-trihydroxy-9,10-secocholesta-5,7,10(19),22-tetraen-25-carbonsäure-1-methylethylester

(5*Z*,7*E*,22*E*)-(1*S*,3*R*,24*S*)-20-Methyl-1,3,24-trihydroxy-9,10-secocholesta-5,7,10(19),22-tetraen-25-carbonsäure-1-methylethylester

(5*Z*,7*E*,22*E*)-(1*S*,3*R*,24*R*)-20-Methyl-1,3,24-trihydroxy-9,10-secocholesta-5,7,10(19),22-tetraen-25-carbonsäurebutylester

(5*Z*,7*E*,22*E*)-(1*S*,3*R*,24*S*)-20-Methyl-1,3,24-trihydroxy-9,10-secocholesta-5,7,10(19),22-tetraen-25-carbonsäurebutylester

(5*Z*,7*E*,22*E*)-(1*S*,3*R*,24*R*)-1,3,24-Trihydroxy-9,10-secocholesta-5,7,10(19),20,22-pentaen-25-carbonsäuremethylester

(5*Z*,7*E*,22*E*)-(1*S*,3*R*,24*S*)-1,3,24-Trihydroxy-9,10-secocholesta-5,7,10(19),20,22-pentaen-25-carbonsäuremethylester

(5*Z*,7*E*,22*E*)-(1*S*,3*R*,24*R*)-1,3,24-Trihydroxy-9,10-secocholesta-5,7,10(19),20,22-pentaen-25-carbonsäureethylester

(5*Z*,7*E*,22*E*)-(1*S*,3*R*,24*S*)-1,3,24-Trihydroxy-9,10-secocholesta-5,7,10(19),20,22-pentaen-25-carbonsäureethylester

(5*Z*,7*E*,22*E*)-(1*S*,3*R*,24*R*)-1,3,24-Trihydroxy-9,10-secocholesta-5,7,10(19),20,22-pentaen-25-carbonsäurepropylester

(5*Z*,7*E*,22*E*)-(1*S*,3*R*,24*S*)-1,3,24-Trihydroxy-9,10-secocholesta-5,7,10(19),20,22-pentaen-25-carbonsäurepropylester

(5*Z*,7*E*,22*E*)-(1*S*,3*R*,24*R*)-1,3,24-Trihydroxy-9,10-secocholesta-5,7,10(19),20,22-pentaen-25-carbonsäure-1-methylethylester

(5*Z*,7*E*,22*E*)-(1*S*,3*R*,24*S*)-1,3,24-Trihydroxy-9,10-secocholesta-5,7,10(19),20,22-pentaen-25-carbonsäure-1-methylethylester

(5*Z*,7*E*,22*E*)-(1*S*,3*R*,24*R*)-1,3,24-Trihydroxy-9,10-secocholesta-5,7,10(19),20,22-pentaen-25-carbonsäurebutyl ester

(5*Z*,7*E*,22*E*)-(1*S*,3*R*,24*S*)-1,3,24-Trihydroxy-9,10-secocholesta-5,7,10(19),20,22-pentaen-25-carbonsäurebutylester

(5*Z*,7*E*,22*E*)-(1*S*,3*R*,24*R*)-20,2-Methylen-1,3,24-trihydroxy-9,10-secocholesta-5,7,10(19),22-tetraen-25-carbonsäuremethylester

(5*Z*,7*E*,22*E*)-(1*S*,3*R*,24*S*)-20,2-Methylen-1,3,24-trihydroxy-9,10-secocholesta-5,7,10(19),22-tetraen-25-carbonsäuremethylester

(5*Z*,7*E*,22*E*)-(1*S*,3*R*,24*R*)-20,21-Methylen-1,3,24-trihydroxy-9,10-secocholesta-5,7,10(19),22-tetraen-25-carbonsäureethylester

(5*Z*,7*E*,22*E*)-(1*S*,3*R*,24*S*)-20,21-Methylen-1,3,24-trihydroxy-9,10-secocholesta-5,7,10(19),22-tetraen-25-carbonsäureethylester

(5*Z*,7*E*,22*E*)-(1*S*,3*R*,24*R*)-20,21-Methylen-1,3,24-trihydroxy-9,10-secocholesta-5,7,10(19),22-tetraen-25-carbonsäurepropylester

(5*Z*,7*E*,22*E*)-(1*S*,3*R*,24*S*)-20,21-Methylen-1,3,24-trihydroxy-9,10-secocholesta-5,7,10(19),22-tetraen-25-carbonsäurepropylester

(5*Z*,7*E*,22*E*)-(1*S*,3*R*,24*R*)-20,21-Methylen-1,3,24-trihydroxy-9,10-secocholesta-5,7,10(19),22-tetraen-25-carbonsäure-1-methylethylester

(5*Z*,7*E*,22*E*)-(1*S*,3*R*,24*S*)-20,21-Methylen-1,3,24-trihydroxy-9,10-secocholesta-5,7,10(19),22-tetraen-25-carbonsäure-1-methylethylester

(5*Z*,7*E*,22*E*)-(1*S*,3*R*,24*R*)-20,21-Methylen-1,3,24-trihydroxy-9,10-secocholesta-5,7,10(19),22-tetraen-25-carbonsäurebutylester

(5*Z*,7*E*,22*E*)-(1*S*,3*R*,24*S*)-20,21-Methylen-1,3,24-trihydroxy-9,10-secocholesta-5,7,10(19),22-tetraen-25-carbonsäurebutylester
(7*E*,22*E*)-(1*R*,3*R*,24*R*)-1,3,24-Trihydroxy-19-nor-9,10-secocholesta-5,7,22-trien-25-carbonsäuremethylester
(7*E*,22*E*)-(1*R*,3*R*,24*S*)-1,3,24-Trihydroxy-19-nor-9,10-secocholesta-5,7,22-trien-25-carbonsäuremethylester
(7*E*,22*E*)-(1*R*,3*R*,24*R*)-1,3,24-Trihydroxy-19-nor-9,10-secocholesta-5,7,22-trien-25-carbonsäureethylester
(7*E*,22*E*)-(1*R*,3*R*,24*S*)-1,3,24-Trihydroxy-19-nor-9,10-secocholesta-5,7,22-trien-25-carbonsäureethylester
(7*E*,22*E*)-(1*R*,3*R*,24*R*)-1,3,24-Trihydroxy-19-nor-9,10-secocholesta-5,7,22-trien-25-carbonsäurepropylester
(7*E*,22*E*)-(1*R*,3*R*,24*S*)-1,3,24-Trihydroxy-19-nor-9,10-secocholesta-5,7,22-trien-25-carbonsäurepropylester
(7*E*,22*E*)-(1*R*,3*R*,24*R*)-1,3,24-Trihydroxy-19-nor-9,10-secocholesta-5,7,22-trien-25-carbonsäure-1-methylethylester
(7*E*,22*E*)-(1*R*,3*R*,24*S*)-1,3,24-Trihydroxy-19-nor-9,10-secocholesta-5,7,22-trien-25-carbonsäure-1-methylethylester
(7*E*,22*E*)-(1*R*,3*R*,24*R*)-1,3,24-Trihydroxy-19-nor-9,10-secocholesta-5,7,22-trien-25-carbonsäurebutylester
(7*E*,22*E*)-(1*R*,3*R*,24*S*)-1,3,24-Trihydroxy-19-nor-9,10-secocholesta-5,7,22-trien-25-carbonsäurebutylester
(7*E*,22*E*)-(1*R*,3*R*,24*R*)-1,3,24-Trihydroxy-19-nor-9,10-secocholesta-5,7,20,22-tetraen-25-carbonsäuremethylester
(7*E*,22*E*)-(1*R*,3*R*,24*S*)-1,3,24-Trihydroxy-19-nor-9,10-secocholesta-5,7,20,22-tetraen-25-carbonsäuremethylester
(7*E*,22*E*)-(1*R*,3*R*,24*R*)-1,3,24-Trihydroxy-19-nor-9,10-secocholesta-5,7,20,22-tetraen-25-carbonsäureethylester
(7*E*,22*E*)-(1*R*,3*R*,24*S*)-1,3,24-Trihydroxy-19-nor-9,10-secocholesta-5,7,20,22-tetraen-25-carbonsäureethylester
(7*E*,22*E*)-(1*R*,3*R*,24*R*)-1,3,24-Trihydroxy-19-nor-9,10-secocholesta-5,7,20,22-tetraen-25-carbonsäurepropylester
(7*E*,22*E*)-(1*R*,3*R*,24*S*)-1,3,24-Trihydroxy-19-nor-9,10-secocholesta-5,7,20,22-tetraen-25-carbonsäurepropylester
(7*E*,22*E*)-(1R,3R,24R)-1,3,24-Trihydroxy-19-nor-9,10-secocholesta-5,7,20,22-tetraen-25-carbonsäure-1-methylethylester
(7*E*,22*E*)-(1*R*,3*R*,24*S*)-1,3,24-Trihydroxy-19-nor-9,10-secocholesta-5,7,20,22-tetraen-25-carbonsäure-1-methylethylester
(7*E*,22*E*)-(1*R*,3*R*,24*R*)-1,3,24-Trihydroxy-19-nor-9,10-secocholesta-5,7,20,22-tetraen-25-carbonsäurebutylester
(7*E*,22*E*)-(1*R*,3*R*,24*S*)-1,3,24-Trihydroxy-19-nor-9,10-secocholesta-5,7,20,22-tetraen-25-carbonsäurebutylester
(7*E*,22*E*)-(1*R*,3*R*)-1,3-Dihydroxy-24-oxo-19-nor-9,10-secocholesta-5,7,22-trien-25-carbonsäuremethylester
(7*E*,22*E*)-(1*R*,3*R*)-1,3-Dihydroxy-24-oxo-19-nor-9,10-secocholesta-5,7,22-trien-25-carbonsäureethylester
(7*E*,22*E*)-(1*R*,3*R*)-1,3-Dihydroxy-24-oxo-19-nor-9,10-secocholesta-5,7,22-trien-25-carbonsäurepropylester
(7*E*,22*E*)-(1*R*,3*R*)-1,3-Dihydroxy-24-oxo-19-nor-9,10-secocholesta-5,7,22-trien-25-carbonsäure-1-methylethylester
(7*E*,22*E*)-(1*R*,3*R*)-1,3-Dihydroxy-24-oxo-19-nor-9,10-secocholesta-5,7,22-trien-25-carbonsäurebutylester
(7*E*,22*E*)-(1*R*,3*R*)-1,3-Dihydroxy-24-oxo-19-nor-9,10-secocholesta-5,7,20,22-tetraen-25-carbonsäuremethylester
(7*E*,22*E*)-(1*R*,3*R*)-1,3-Dihydroxy-24-oxo-19-nor-9,10-secocholesta-5,7,20,22-tetraen-25-carbonsäureethylester
(7*E*,22*E*)-(1*R*,3*R*)-1,3-Dihydroxy-24-oxo-19-nor-9,10-secocholesta-5,7,20,22-tetraen-25-carbonsäurepropylester
(7*E*,22*E*)-(1*R*,3*R*)-1,3-Dihydroxy-24-oxo-19-nor-9,10-secocholesta-5,7,20,22-tetraen-25-carbonsäure-1-methylethylester
(7*E*,22*E*)-(1*R*,3*R*)-1,3-Dihydroxy-24-oxo-19-nor-9,10-secocholesta-5,7,20,22-tetraen-25-carbonsäurebutylester
(7*E*,22*E*)-(1*R*,3*R*,20*S*,24*R*)-20-Fluor-1,3,24-trihydroxy-19-nor-9,10-secocholesta-5,7,22-trien-25-carbonsäureethylester
(7*E*,22*E*)-(1*R*,3*R*,20*S*,24*S*)-20-Fluor-1,3,24-trihydroxy-19-nor-9,10-secocholesta-5,7,22-trien-25-carbonsäureethylester
(7*E*,22*E*)-(1*R*,3*R*,20*R*,24*R*)-20-Fluor-1,3,24-trihydroxy-19-nor-9,10-secocholesta-5,7,22-trien-25-carbonsäureethylester
(7*E*,22*E*)-(1*R*,3*R*,20*R*,24*S*)-20-Fluor-1,3,24-trihydroxy-19-nor-9,10-secocholesta-5,7,22-trien-25-carbonsäureethylester
(7*E*,22*E*)-(1*R*,3*R*,20*S*,24*R*)-20-Fluor-1,3,24-trihydroxy-19-nor-9,10-secocholesta-5,7,22-trien-25-carbonsäure-1-methylethylester
(7*E*,22*E*)-(1*R*,3*R*,20*S*,24*S*)-20-Fluor-1,3,24-trihydroxy-19-nor-9,10-secocholesta-5,7,22-trien-25-carbonsäure-1-methylethylester
(7*E*,22*E*)-(1*R*,3*R*,20*R*,24*R*)-20-Fluor-1,3,24-trihydroxy-19-nor-9,10-secocholesta-5,7,22-trien-25-carbonsäure-1-methylethylester
(7*E*,22*E*)-(1*R*,3*R*,20*R*,24*S*)-20-Fluor-1,3,24-trihydroxy-19-nor-9,10-secocholesta-5,7,22-trien-25-carbonsäureethyl-1-methylester
(5*Z*,7*E*,22*E*)-(1*S*,3*R*,24*R*)-1,3,24-Trihydroxy-9,10-secocholesta-5,7,10(19),22-tetraen-25-carbonsäurebutylester
(5*Z*,7*E*,22*E*)-(1*S*,3*R*,24*S*)-1,3,24-Trihydroxy-9,10-secocholesta-5,7,10(19),22-tetraen-25-carbonsäurebutylester
(5*Z*,7*E*,22*E*)-(1*S*,3*R*,24*R*)-1,3,24-Trihydroxy-9,10-secocholesta-5,7,10(19),22-tetraen-25-carbonsäure-2-methylpropylester
(5*Z*,7*E*,22*E*)-(1*S*,3*R*,24*S*)-1,3,24-Trihydroxy-9,10-secocholesta-5,7,10(19),22-tetraen-25-carbonsäure-2-methylpropylester
(5*Z*,7*E*,22*E*)-(1*S*,3*R*,24*R*)-1,3,24-Trihydroxy-9,10-secocholesta-5,7,10(19),22-tetraen-25-carbonsäurepentylester
(5*Z*,7*E*,22*E*)-(1*S*,3*R*,24*S*)-1,3,24-Trihydroxy-9,10-secocholesta-5,7,10(19),22-tetraen-25-carbonsäurepentylester
(5*Z*,7*E*,22*E*)-(1*S*,3*R*)-1,3-Dihydroxy-24-oxo-9,10-secocholesta-5,7,10(19),22-tetraen-25-carbonsäuremethylester
(5*Z*,7*E*,22*E*)-(1*S*,3*R*)-1,3-Dihydroxy-24-oxo-9,10-secocholesta-5,7,10(19),22-tetraen-25-carbonsäureethylester
(5*Z*,7*E*,22*E*)-(1*S*,3*R*)-1,3-Dihydroxy-24-oxo-9,10-secocholesta-5 10(19),22-tetraen-25-carbonsäurepropylester
(5*Z*,7*E*,22*E*)-(1*S*,3*R*)-1,3-Dihydroxy-24-oxo-9,10-secocholesta-5,7,10(19),22-tetraen-25-carbonsäure-1-methylethylester
(5*Z*,7*E*,22*E*)-(1*S*,3*R*)-1,3-Dihydroxy-24-oxo-9,10-secocholesta-5,7,10(19),22-tetraen-25-carbonsäurebutylester
(5*Z*,7*E*,22*E*)-(1*S*,3*R*)-1,3-Dihydroxy-24-oxo-9,10-secocholesta-5,7,10(19),22-tetraen-25-carbonsäure-2-methylpropylester
(5*Z*,7*E*,22*E*)-(1*S*,3*R*)-1,3-Dihydroxy-24-oxo-9,10-secocholesta-5,7,10(19),22-tetraen-25-carbonsäurepentylester
(5*Z*,7*E*,22*E*)-(1*S*,3*R*)-1,3-Dihydroxy-20-methyl-24-oxo-9,10-secocholesta-5,7,10(19),22-tetraen-25-carbonsäureethylester
(5*Z*,7*E*,22*E*)-(1*S*,3*R*)-1,3-Dihydroxy-20-methyl-24-oxo-9,10-secocholesta-5,7,10(19),22-tetraen-25-carbonsäure-1-methylethylester
(5*Z*,7*E*,22*E*)-(1*S*,3*R*,24*R*)-1,3,24-Trihydroxy-26a,27-cyclo-26a-homo-9,10-secocholesta-5,7,10(19),22-tetraen-25-carbonsäureethylester
(5*Z*,7*E*,22*E*)-(1*S*,3*R*,24*S*)-1,3,24-Trihydroxy-26a,27-cyclo-26a-homo-9,10-secocholesta-5,7,10(19),22-tetraen-25-carbonsäureethylester
(5*Z*,7*E*,22*E*)-(1*S*,3*R*,24*R*)-1,3,24-Trihydroxy-26a,27-cyclo-26a-homo-9,10-secocholesta-5,7,10(19),22-tetraen-25-carbonsäure-1-methylethylester
(5*Z*,7*E*,22*E*)-(1*S*,3*R*,24*S*)-1,3,24-Trihydroxy-26a,27-cyclo-26a-homo-9,10-secocholesta-5,7,10(19),22-tetraen-25-carbonsäure-1-methylethylester

Die natürlichen Vitamine $D_2$ und $D_3$ (vergl. allgemeine Formel Vit. D) sind an sich biologisch inaktiv und werden erst nach Hydroxylierung in 25-Position in der Leber bzw. in 1-Position in der Niere in deren biologisch aktive Metaboliten umgewandelt. Die Wirkung der Vitamine $D_2$ und $D_3$ besteht in der Stabilisierung des Plasma-$Ca^{++}$- und Plasma-Phosphat-Spiegels; sie wirken einem Absinken des Plasma-$Ca^{++}$-Spiegels entgegen.

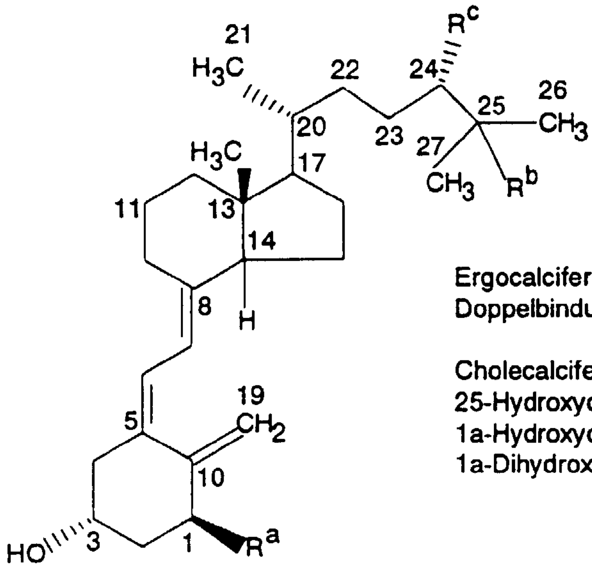

Ergocalciferol: Ra=Rb=H, Rc=CH3 Doppelbindung C-22/23 — Vitamin D2

Cholecalciferol: Ra=Rb=Rc=H — Vitamin D3
25-Hydroxycholecalciferol: Ra=Rc=H,Rb=OH
1a-Hydroxycholecalciferol: Ra=OH,Rb=Rc=H
1a-Dihydroxycholecalciferol: Ra=Rb=OH,Rc=H — Calcitriol

Neben ihrer ausgeprägten Wirkung auf den Calcium- und Phosphatstoffwechsel besitzen Vitamin $D_2$ und $D_3$ und seine synthetischen Abkömmlinge auch proiiferationshemmende und zelldifferenzierende Wirkungen (H.F. De Luca, The Metabolism and Function of Vitamin D in Biochemistry of Steroid Hormones, Hrsg. H.LJ. Makin, 2nd Edition, Blackwell Scientific Publications 1984, S. 71-116).
Bei Vitamin D-Anwendung kann es aber zu Überdosierungserscheinungen kommen (Hypercalcämie).

In 24-Stellung hydroxylierte 1α-Cholecalciferole gehen bereits aus der DE-A-25 26 981 hervor; sie besitzen eine geringere Toxizität als das entsprechende nicht-hydroxylierte 1α-Cholecalciferol. Die hydroxylierten Verbindungen zeigen eine selektive Aktivierung der intestinalen Calciuniabsorption und eine schwächere Knochenabsorptionswirkung als 1α-Cholecalciferol.
Die in der internationalen Patentanmeldung WO 87/00834 beschriebenen 24-Hydroxy-Vitamin D-Analoga können für die Behandlung von durch abnormer Zellproliferation und/oder Zelldifferentiation hervorgerufenen Störungen beim Menschen und Tier dienen.

Für verschiedene 1,25-Dihydroxy-Homo-Vitamin D-Derivate ist eine Dissoziation bezüglich der Eigenschaften Knochenabsorptionswirkung und HL-60 Zelldifferentiation schon kürzlich von De Luca erwähnt worden. Die Knochenabsorptionswirkung in vitro ist dabei ein direktes Maß für die Calciummobilisierung in vivo.

Die Intemationale Anmeldung WO-A 93 09093 beschreibt Vitamin D-Derivate mit terminalen Carbonsäureamid-Funktionen. Diese Analoga leiten sich strukturell aus dem inaktiven Endprodukt des Calcitriol-Metabolismus - der Calcitroic Acid - durch Homologisierungen der Seitenkette ab. Neben Doppelbindungen besitzen die Seitenketten keine weiteren Substituenten.
Den Derivaten werden potente zellmodulierende Effekte neben minimalen calcitropen Aktivitäten zugeschrieben. Da die Derivate jedoch nicht gut an den intestinalen Vitamin D-Rezeptor binden, ist naheliegend, daß die Potenzen der Analoga bzgl. der zellmodulierenden Effekte sehr viel geringer als die des Calcitriols sind.

Schließlich werden in der EP-A 0 421 561 24-Cycloalkylmethyl- substituierte Vitamin D-Derivate beschrieben, die ein günstigeres Wirkungsspektrum als Calcitriol aufweisen. Während deren Effekte auf den Calcium- und Phosphatstoffwechsel im Vergleich zu Calcitriol deutlich abgeschwächt sind, bleiben die proliferationshemmenden und zelldifferenzierenden Wirkungen annähernd erhalten.
Gegenüber diesen strukturell verwandten Verbindungen zeichnen sich die erfindungsgemäßen 25-Carbonsäure-Derivate in der Vitamin D-Reihe dadurch aus, daß sie im Hinblick auf die Zelldifferenzierung gegenüber der hypercalcämischen Wirkung noch stärker dissoziiert sind.

Die Vitamin D-Aktivität der erfindungsgemäßen Verbindungen wird mittels des Calcitriol-Rezeptortests bestimmt. Er wird unter Verwendung eines spezifischen Rezeptorproteins aus dem Darm von jungen Schweinen durchgeführt.

Rezeptorhaltiges Bindungsprotein wird mit $^3H$-Calcitriol ($5x10^{-10}$mol/l) in einem Reaktionsvolumen von 0,270 ml in Abwesenheit und in Anwesenheit der Prüfsubstanzen für zwei Stunden bei 4° C in einem Teströhrchen inkubiert. Zur Trennung von freiem und rezeptorgebundenem Calcitriol wird eine Charcoal-Dextran-Absorption durchgeführt. Dazu werden 250 µl einer Charcoal-Dextran-Suspension jedem Teströhrchen zugeführt und bei 4° C für 20 Minuten inkubiert. Anschließend werden die Proben bei 10 000 x g 5 Minuten bei 4° C zentrifugiert. Der Überstand wird dekantiert und nach lstündiger Äquilibrierung in Picofluor 15 TM in einem β-Zähler gemessen.

Die mit verschiedenen Konzentrationen der Prüfsubstanz sowie der Referenzsubstanz (unmarkiertes Calcitriol) bei konstanter Konzentration der Bezugssubstanz ($^3$H-Calcitriol) erhaltenen Kompetitionskurven werden in Beziehung zueinander gesetzt und ein Kompetitionsfaktor (KF) ermittelt.
Er ist definiert als Quotient aus den Konzentrationen der jeweiligen Prüfsubstanz und der Referenzsubstanz, die für 50%ige Kompetition erforderlich sind:

$$KF = \frac{\text{Konzentration Prüfsubstanz bei 50\% Kompetition}}{\text{Konzentration Referenzsubstanz bei 50\% Kompetition}}$$

Den erfindungsgemäßen Verbindungen ist gemein, daß sie alle über eine beträchtliche Affinität zum Calcitriol-Rezeptor verfügen.

Zur Bestimmung der akuten hypercalcämischen Wirkung verschiedener Calcitriolderivate wird nachfolgend beschriebener Test durchgeführt:
Die Wirkung von Kontrolle (Lösungsgrundlage), Referenzsubstanz (1,25$(OH)_2$-$D_3$= Calcitriol) und Testsubstanz wird jeweils nach einmaliger subcutaner Applikation in Gruppen von 10 nativen männlichen Ratten (140-170 g) getestet. Die Ratten werden während der Versuchszeit in speziellen Käfigen zur Bestimmung der Exkretion von Wasser und Mineralstoffen gehalten. Der Harn wird in 2 Fraktionen (0-16 h und 16-22h) gesammelt. Eine orale Calciumlast (0.1 mM Calcium in 6.5% Alphahydroxypropylcellulose, 5 ml/ Tier) ersetzt zum Zeitpunkt 16 h die durch Futterentzug fehlende Calciumaufnahme. Zu Versuchsende werden die Tiere durch Dekapitieren getötet und für die Bestimmung der Serum-Calciumwerte entblutet. Für die primäre Screen-Untersuchung in vivo wird eine einzelne Standarddosis (200 µg/kg) getestet. Für ausgewählte Substanzen wird das Ergebnis durch Erstellung einer Dosis-Wirkungs-Beziehung abgesichert.

Eine hypercalcämische Wirkung zeigt sich in im Vergleich zur Kontrolle erhöhten Serum-Calciumspiegel-Werten.

Die Signifikanz auftretender Unterschiede zwischen Substanzgruppen und Kontrollen sowie zwischen Testsubstanz und Referenzsubstanz werden mit geeigneten statistischen Verfahren abgesichert. Das Ergebnis wird als Dosisrelation DR (DR = Faktor Testsubstanzdosis/Referenzsubstanzdosis für vergleichbare Wirkungen) angegeben.

Die differenzierungsstimulierende Wirkung von Calcitriolanaloga wird ebenfalls quantitativ erfaßt.
Es ist literaturbekannt (Mangelsdorf, D. J. et al., J. Cell. Biol. 98: 391-398 (1984)), daß die Behandlung humaner Leukämiezellen (Promyelozytenzellinie HL 60) in vitro mit Calcitriol die Differenzierung der Zellen zu Makrophagen induziert.

HL 60-Zellen werden in Gewebekulturmedium (RPMI -10% fetales Kälberserum) bei 37° C in einer Atmosphäre 5% $CO_2$ in Luft kultiviert.

Zur Substanztestung werden die Zellen abzentrifugiert und 2,0 x $10^5$ Zellen/ml in phenolrotfreiem Gewebekulturmedium aufgenommen. Die Testsubstanzen werden in Ethanol gelöst und mit Gewebekulturmedium ohne Phenolrot auf die gewünschte Konzentration verdünnt. Die Verdünnungsstufen werden mit der Zellsuspension in einem Verhältnis von 1:10 gemischt und je 100 µl dieser mit Substanz versetzten Zellsuspension in eine Vertiefung einer 96-Loch-Platte pipettiert. Zur Kontrolle wird eine Zellsuspension analog mit dem Lösungsmittel versetzt.

Nach Inkubation über 96 Stunden bei 37° C in 5 % $CO_2$ in Luft wird in jede Vertiefung der 96-Loch-Platte zu der Zellsuspension 100 µl einer NBT-TPA-Lösung (Nitroblautetrazolium (NBT), Endkonzentration im Ansatz 1 mg/ml, Tetradecanoylphorbolmyristat-13-acetat (TPA), Endkonzentration im Ansatz 2 x $10^7$ mol/l) pipettiert.

Durch Inkubation über 2 Stunden bei 37° C und 5% $CO_2$ in Luft wird infolge der intrazellulären Sauerstoffradikalfreisetzung, stimuliert durch TPA, in den zu Makrophagen differenzierten Zellen NBT zu unlöslichem Formazan reduziert.

Zur Beendigung der Reaktion werden die Vertiefungen der 96-Loch-Platte abgesaugt und die anhaftenden Zellen durch Zugabe von Methanol fixiert und nach Fixation getrocknet. Zur Lösung der gebildeten intrazellulären Formazankristalle werden in jede Vertiefung 100 µl Kaliumhydroxid (2 val/l) und 100 µl Dimethylsulfoxid pipettiert und 1 Minute ultrabeschallt. Die Konzentration von Formazan wird spektralphotometrisch bei 650 nm gemessen.
Als Maß für die Differenzierungsinduktion der HL 60-Zellen zu Makrophagen gilt die Konzentration an gebildetem Formazan. Das Ergebnis wird ebenfalls als Dosisrelation (DR = Faktor Testsubstanzdosis/Referenzsubstanzdosis für vergleichbare Wirkungen) angegeben.

Die Ergebnisse des Calcitriol-Rezeptortests sowie der Bestimmung der Dosisrelation der Differenzierungsinduktion von HL 60-Zellen und der Dosisrelation für Hypercalcämie sind nachfolgend zusammengefaßt:

Testverbindungen:

(5*Z*,7*E*,22*E*)-(1*S*,3*R*,24*R*)-1,3,24-Trihydroxy-9,10-secocholesta-5,7,10(19),22tetraen-25-carbonsäureethylester **10a**
(5*Z*,7*E*,22*E*)-(1*S*,3*R*,24*R*)-1,3,24-Trihydroxy-9,10-secocholesta-5,7,10(19),22-tetraen-25-carbonsäure-1-methylethylester **13a**
(5*Z*,7*E*,22*E*)-(1*S*,3*R*,24*R*)-1,3,24-Trihydroxy-9,10-secocholesta-5,7,10(19),22-tetraen-25-carbonsäurepropylester **19a**
(5*Z*,7*E*,22*E*)-(1*S*,3*R*,24*R*)-1,3,24-Trihydroxy-9,10-secocholesta-5,7,10(19),22-tetraen-25-carbonsäuredimethylamid **31a**
(5*Z*,7*E*,22*E*)-(1*S*,3*R*,24*R*)-20-Methyl-1,3,24-trihydroxy-9,10-secocholesta-5,7,10(19),22-tetraen-25-carbonsäureethylester **46a**
(5*Z*,7*E*,22*E*)-(1*S*,3*R*,24*R*)-20-Methyl-1,3,24-trihydroxy-9,10-secocholesta-5,7,10(19),22-tetraen-25-carbonsäurepropylester 48a
(5*Z*,7*E*,22*E*)-(1*S*,3*R*,24*S*)-20-Methyl-1,3,24-trihydroxy-9,10-secocholesta-5,7,10(19),22-tetraen-25-carbonsäurepropylester 48b
(5*Z*,7*E*,22*E*)-(1*S*,3*R*,24*R*)-20-Methyl-1,3,24-trihydroxy-9,10-secocholesta-5,7,10(19),22-tetraen-25-carbonsäure-1-methylethylester **50a**
(5*Z*,7*E*,22*E*)-(1*S*,3*R*,24*R*)-1,3,24-Trihydroxy-9,10-secocholesta-5,7,10(19),20,22-pentaen-25-carbonsäure-1-methylethylester **64a**
(7*E*,22*E*)-1*R*,3*R*,20*R*,24*S*)-20-Fluor-1,3,24-trihydroxy-19-nor-9,10-secocholesta-5,7,22-trien-25-carbonsäureethylester **111**
(5*Z*,7*E*,22*E*)-(1*S*,3*R*)-1,3-Dihydroxy-24-oxo-9,10-secocholesta-5,7,10(19),22-tetraen-25-carbonsäuremethylester **125**
(5*Z*,7*E*,22*E*)-(1*S*,3*R*,24*R*)-1,3,24-Trihydroxy-9,10-secocholesta-5,7,10(19),22-tetraen-25-carbonsäure **21a**
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäuremethylester **22a**
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäureethylester **23a**
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäurepropylester **24a**
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäurebutylester **25a**
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäure-2-methylpropylester **26a**

Vergleichsverbindung: Calcitriol

| Verbindung | Kompetitionsfaktor KF für Rezeptorbindung | Dosisrelation für HL 60-Zellen | Dosisrelation für Hypercalcämie |
|---|---|---|---|
| **6a** | 4,1 | 1,3 | 100 |
| **10a** | 2,4 | 0,6 | 100 |
| **13a** | 2,1 | 0,3 | 1500 |
| **19a** | 1,8 | 0,2 | 100 |
| **31a** | 2,6 | 0,8 | 100 |
| **46a** | 4,1 | 1,3 | 100 |
| **48a** | 2,8 | 0,2 | >100 |
| **48b** | 16 | 1 | 100 |
| **50a** | 2,4 | 0,6 | >100 |
| **64a** | 2,7 | 2 | >>100 |
| **111** | 11 | 4 | >100 |
| **125** | 3,6 | 2 | 100 |
| **21a** | 32 | >1000 | 1000 |
| Calcitriol | 1 | 1 | 1 |

Die aufgeführten Verbindungen zeigen neben einer Affinität zum Calcitriolrezeptor wie Calcitriol teilweise stärkere agonistische Aktivitäten *in vitro* (HL 60-Funktionstest). Die Induktion einer Hypercacämie *in vivo* erfolgt dagegen erst bei deutlich höheren Dosen als bei Calcitriol.
Die freie Carbonsäure **21a**, die ein potentieller Metabolit der Testverbindungen sein könnte, bindet dagegen deutlich schlechter an den Rezeptor und ist *in vitro* und *in vivo* praktisch inaktiv.
Durch das verminderte Hypercalciämie-Risiko eignen sich die erfindungsgemäßen Substanzen in besonderer Weise zur Herstellung von Arzneimitteln für die Behandlung von Erkrankungen, die durch eine Hyperproliferation gekennzeichnet sind, z.B. hyperproliferative Erkrankungen der Haut (Psoriasis) und maligne Tumoren (Leukämie, Coloncarcinom, Mammacarcinom) und Akne (J. Invest. Dermatol., Vol. 92 No. 3,1989). Auch zur Behandlung und Prophylaxe von Störungen, die durch eine Störung des Gleichgewichts des Immunsystems gekennzeichnet sind, beispielsweise Autoimmunkrankheiten, einschließlich Diabetes mellitus und der Abstoßungsreaktionen bei Transplantationen (WO-A-91/00855), können die erfindungsgemäßen Verbindungen verwendet werden. In einer besonders bevorzugten Ausführungsform der Erfindung werden vor der Behandlung im Zielorgan Calcitriolrezeptoren nachgewiesen.

Weiterhin wurde überraschenderweise gefunden, daß durch topische Applikation der erfindungsgemäßen Verbindungen auf die Haut von Mäusen, Ratten und Meerschweinchen eine vermehrte Hautrötung und Zunahme der Epidermisdicke induziert werden kann. Die Zunahme der Hautrötung wird anhand der Erhöhung des mit einem Farbmeßgerät quantifizierbaren Rotwertes der Hautoberfläche ermittelt. Der Rotwert ist nach dreimaliger Substanzapplikation (Dosis 0,003%) im Abstand von 24 Stunden typischerweise um das 1,5-fache erhöht. Die Zunahme der Epidermisdicke wird im histologischen Präparat quantifiziert. Sie ist typischerweise um das 2,5-fache erhöht. Die Anzahl der proliferierenden Epidermiszellen (Zellen in der S-Phase des Zellcyclus) wird durchflußcytometrisch ermittelt und ist typischerweise erhöht.

Diese Eigenschaften der erfindungsgemäßen 25-Carbonsäure-Derivate in der Vitamin D-Reihe läßt sie zum therapeutischen Einsatz bei atrophischer Haut, wie sie bei natürlicher Hautalterung, vorzeitiger Hautalterung infolge erhöhter Lichtexposition oder medikamentös induzierter Hautatrophie durch Behandlung mit Glucocorticoiden auftritt, geeignet erscheinen.

Weiterhin ist anzunehmen, daß die Wundheilung durch topische Applikation mit den neuen Verbindungen beschleunigt werden kann.

Die erfindungsgemäßen Verbindungen der allgemeinen Formel I sind auch potente Hemmstoffe der Proliferation und Interleukin (IL 2)-Synthese von humanen Lymphocyten. Infolge der Hemmung der Lymphocytenproliferation und IL 2-Synthese in niedriger Konzentration sind die erfindungsgemäßen Verbindungen der allgemeinen Formel I zur Therapie von Erkrankungen des Immunsystems geeignet, z. B. Erkrankungen des atopischen Formenkreises (atopische Dermatitis, Asthma), Autoimmunerkrankungen einschließlich Diabetes mellitus, Transplantatabstoßungsreaktionen und AIDS.

Für Calcitriol wurde gefunden, daß es aufgrund eines rezeptorvermittelten Mechanismus nicht nur die IL 2-Sekretion, sondern auch die Produktion anderer entzündungsfördender Cytokoine hemmt. Da die Verbindungen der allgemeinen Formel I etwa ebenso gut an den Rezeptor binden wie Calcitriol sind sie geeignet zur Behandlung von entzündlichen Erkrankungen wie Arthritis, Colitis ulcerosa und Morbus Crohn.

Bei der Behandlung von Autoimmunerkrankungen, Transplantatabstoßungsreaktionen und AIDS können die neuen Verbindungen der allgemeinen Formel I vorteilhaft mit anderen immunsuppressiv wirksamen Stoffen wie Cyclosporin A und FK 506 kombiniert werden.

Es wurde außerdem gefunden, daß bestimmte Verbindungen der allgemeinen Formel I in HL 60-Zellen überraschenderweise die Wirkung von Calcitriol antagonisieren.. In der 26,27-Cyclo-Reihe ($R^4$ + $R^{4a}$ + C20 = Cyclopropyl) zeigen die Carbonsäureester mit zunehmender Kettenlänge von $R^6$ im Rest Y bei gleichbleibend guter Rezeptor-Affinität deutlich schwächere Vitamin D-Aktivitäten *in vitro* und *in vivo.* Der Übergang vom Agonismus zum Antagonismus liegt zwischen dem Propyl- und Butylester **24a** und **25a**. So bindet 25a mit der gleichen Affinität wie Calcitriol an dessen Rezeptor, hat aber in HL 60-Zellen keine differenzierungsstimulierende Wirkung. Diese Eigenschaft, Calcitriol in HL 60-Zellen zu antagonisieren, setzt sich mit zunehmender Kettenlänge im Rest $R^6$ fort.

Bei gleichzeitiger Inkubation von Calcitriol mit steigenden Konzentrationen der auf Antagonismus in HL 60-Zellen zu testenden Verbindung wird eine Hemmung der durch Calcitriol induzierten NBT-Reduktion als Maß für die differenzierungsstimulierende Wirkung von Calcitriol gefunden, die beispielsweise bei 100-fachem Überschuß der Verbindung 25a gegenüber Calcitriol vollständig ist. Gleiches gilt auch für die Verbindung 26a sowie die höheren Carbonsäureester.

Einige Testergebnisse, die die abnehmende agonistische Wirkung mit zunehmender Kettenlänge von $R^6$ zeigen, sind nachstehend aufgelistet:

| Verbindung | Kompetitionsfaktor KF für Rezeptorbindung | Dosisrelation für HL 60-Zellen | Dosisrelation für Hypercalcämie |
|---|---|---|---|
| Calcitriol | 1 | 1 | 1 |
| **22a** | 4,0 | 2,2 | >100 |
| **23a** | 2,8 | 2,9 | >>100 |
| **24a** | 4,6 | 22 | >>100 |
| **25a** | 1,0 | >1000 | >1000 |
| **26a** | 2,5 | >1000 | 1000 |

Solche Verbindungen, die die Wirkung von Calcitriol antagonisieren, können bei der Therapie von Hypercalcämien eingesetzt werden, wie z.B. bei Hypervitaminose D oder Intoxikation mit Calcitriol und calcitriolartig wirksamen Substanzen, oder bei erhöhter extrarenaler Calcitriolsynthese bei granulomatösen Erkrankungen (z.B. Sarkoidose) vorkommen. Auch paraneoplastische Hypercalcämien (z.B. bei osteolytischen Knochenmetastasen) und Hypercalcämien bei Hyperparathyreodismus können mit einem Calcitriolantagonisten behandelt werden.

Weiterhin sind Calcitriolantagonisten zur Fertilitätskontrolle einzusetzen. Im Reproduktionstrakt weiblicher und männlicher Tiere wird der Vitamin D-Rezeptor exprimiert. Es ist bekannt, daß die weibiiche und männliche Fertilität Vitamin D-defizienter Tiere herabgesetzt ist. Durch kurzfristige Substitution von Calcitriol kann die Reproduktionsleistung erhöht werden. Daher sind Calcitriolantagonisten in der Lage, die weibliche und männliche Fertilität zu beeinflussen.

Da Calcitriol unter bestimmten Bedingungen eine immunsuppressive Wirkung zeigt, sind Calcitriolrezeptorantagonisten auch als Immunstimulantien, z.B. bei Infektabwehrschwäche, einzusetzen.

Von Calcitriol ist bekannt, daß es das Haarwachstum modulieren kann. Calcitriolantagonisten können daher bei unerwünschtem Haarwuchstum, z.B. beim Hirsutismus, therapeutische Verwendung finden.

Die vorliegende Erfindung bezieht sich somit auch auf pharmazeutische Präparate, die mindestens eine Verbindung gemäß der allgemeinen Formel I zusammen mit einem pharmazeutisch verträglichen Träger enthalten.

Die Verbindungen können formuliert werden als Lösungen in pharmazeutisch verträglichen Solventien oder als Emulsionen, Suspensionen oder Dispersionen in geeigneten pharmazeutischen Solventien oder Trägern oder als Pillen, Tabletten oder Kapseln, die in an sich bekannter Weise feste Trägerstoffe enthalten. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsstoffe enthalten, wie z.B. Stabilisatoren, Antioxidantien, Bindemittel, Farbstoffe, Emulgatoren oder Geschmackskorrigentien. Die Verbindungen werden vorteilhafterweise durch Injektion oder intravenöse Infusion geeigneter steriler Lösungen oder als orale Dosierung über den Ernährungstrakt oder topisch in der Form von Cremes, Salben, Lotions oder geeigneter transdermaler Pflaster appliziert, wie in der EP-A 0 387 077 beschrieben ist. Die tägliche Dosis liegt bei

0,1 µg/Patient/Tag - 1000 µg (1 mg)/Patient/Tag,

vorzugsweise

1,0 µg/Patient/Tag - 500 µg/Patient/Tag.

Die erfindungsgemäßen Verbindungen werden im allgemeinen verabreicht analog zur Verabreichung des bekannten Mittels "Calcipotriol" zur Behandlung der Psoriasis.

Außerdem betrifft die Erfindung die Verwendung der Verbindungen gemäß Formel I zur Herstellung von Arzneimitteln.

Die Herstellung der 25-Carbonsäure-Derivate der allgemeinen Formel **I** erfolgt erfindungsgemäß dadurch, daß eine Verbindung der allgemeinen Formel **II**

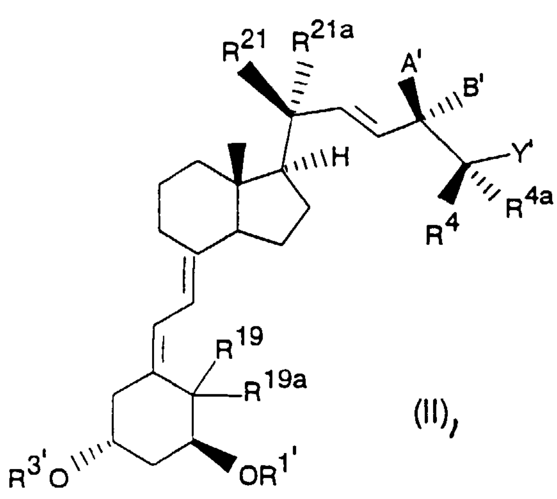

(II),

worin

$R^{1'}$ und $R^{3'}$ alkyl-, aryl- oder gemischte alkyl- und arylsubstituierte Silylgruppen, vorzugsweise die tert.-Butyldimethylsilyl-, tert.-Butyldiphenylsilyl- oder Triisopropylsilylgruppe bedeuten,
A' und B' gemeinsam eine Ketogruppe oder einer der beiden Substituenten eine gegebenenfalls geschützte Hydroxygruppe und der andere ein Wasserstoffatom darstellt (Silylschutzgruppe obiger Definition, Tetrahydrofuranyl-, Tetrahydropyranyl-, Methoxymethyl-, Methoxyethoxymethyl- oder Trimethylsilylethoxymethylgruppe), $R^{19}$, $R^{19a}$, $R^{21}$, $R^{21a}$, $R^4$, und $R^{4a}$ die schon in der allgemeinen Formel **I** beschriebenen Bedeutungen haben, und Y' dieselben Reste wie Y in der Verbindung der allgemeinen Formel I oder, wenn Y in der Verbindung der Formel I -C(O)$OR^6$ und $R^6$ Wasserstoff sein soll, gegebenenfalls eine 2-(Trimethylsilyl)ethyl-carbonsäureestergruppe bedeutet,

durch gleichzeitige oder sukzessive Abspaltung der Hydroxy- und gegebenenfalls Carbonsäureschutzgruppen und gegebenenfalls durch partielle, sukzessive oder vollständige Veresterung freier Hydroxygruppen und/oder, wenn dann Y' für einen Carboxylrest -COOH steht, gewünschtenfalls durch dessen Veresterung oder Umwandlung in einen Amidrest -C(O)$NR^5R^{5a}$ in eine Verbindung der allgemeinen Formel **I** überführt wird.

Im Falle der Silylschutzgruppen oder der Trimethylsilylethoxymethylgruppe verwendet man zu deren Abspaltung Tetrabutylammoniumfluorid, Fluorwasserstoffsäure oder Fluorwasserstoffsäure/Pyridin; im Falle der übrigen Ethergruppen werden diese unter der katalytischen Einwirkung von Säure, beispielsweise p-Toluolsulfonsäure, Pyridinium-p-toluolsulfonat, Essigsäure, Salzsäure oder einem sauren Ionenaustauscher abgetrennt.

Wenn $R^6$ in der allgemeinen Formel **I** Wasserstoff bedeuten soll, steht Y' in der allgemeinen Formel **II** vorzugsweise für eine 2-(Trimethylsilyl)ethylcarbonsäureestergruppe sein, deren Spaltung mit einem der genannten Fluorreagenzien erfolgt. Die entstehende freie Carbonsäure kann gegebenenfalls nach gängigen Verfahren durch Veresterung oder Umwandlung in einen Amidrest -C(O)$NR^5R^{5'}$ oder einen Thioester -C(O)$SR^6$ weiter umgesetzt werden.

Die Herstellung der Ausgangsverbindungen für die allgemeine Formel **II** geht je nach letzendlich gewünschtem Substitutionsmuster in 10- und 20-Position von verschiedenen Startverbindungen aus.

Für die Gewinnung der Verbindungen der allgemeinen Formel II, worin $R^{19}$ und $R^{19a}$ gemeinsam für eine exoständige Methylengruppe stehen, wird von den in Tetrahedron **43**, 4609 (1987) bzw. in der internationalen Patentanmeldung WO 87/00834 von M. Calverley et al. beschriebenen (20*S*)-Formylsecopregnatrienen der allgemeinen Formel **III,**

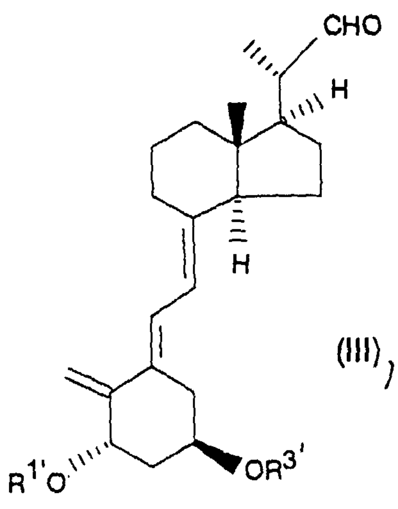

(III),

worin $R^{1'}$ und $R^{3'}$ die schon beschriebenen Bedeutungen haben.,
ausgegangen.

Andere Schutzgruppen in III als in den angegebenen Fundstellen beschrieben, lassen sich durch analoge Vorgehensweise unter Verwendung entsprechend modifizierter Silylchloride (z.B. tert.-Butyldiphenylsilylchlorid anstelle von tert.-Butyldimethylsilylchlorid) erhalten.

Die Verbindungen der allgemeinen Formel **III** werden, wenn die zur Herstellung der letzendlich gewünschten Verbindungen der allgemeinen Formel I benötigten Verbindungen der allgemeinen Formel II in der 20-Position ein vom Calcitriol abweichendes Substitutionsmuster aufweisen sollen, nach neuen Verfahren in die 20-modifizierten Analoga der allgemeinen Formel **IV**

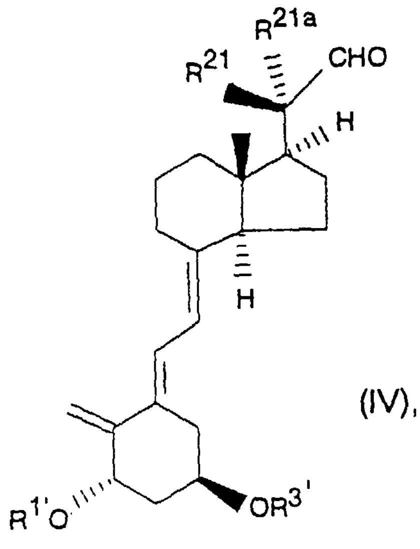

(IV),

worin $R^{1'}$, $R^{3'}$, $R^{21}$ und $R^{21a}$ die schon beschriebenen Bedeutungen haben, überführt.

Zur Synthese der Verbindungen der allgemeinen Formel **V**,

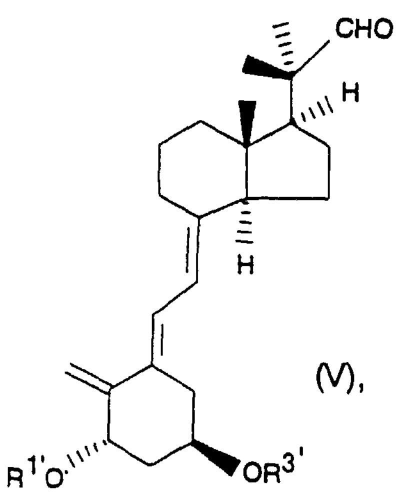

(V),

die sich aus der allgemeinen Formel **IV** dadurch ableitet, daß $R^{21}$ und $R^{21a}$ je eine Methylgruppe darstellen, wird die Verbindung der allgemeinen Formel **III** mit einer Base wie Natriumhydrid, Kaliumhydrid, Lithiumdiisopropylamid oder Kalium-tert.-butanolat deprotoniert und mit einem elektrophilen, die Methylgruppe liefernden Reagenz wie etwa $CH_3X$ (X=Cl, Br, I, Tosylat, Mesylat) umgesetzt.

Die homologen Alkylgruppen $R^{21}$ bzw. $R^{21a}$ werden analog durch Alkylierung mit einem die homologe Alkylgruppe liefernden Reagenz eingeführt.

Die Einführung eines Chlor- oder Fluoratoms in 20-Position gelingt durch α-Halogenierung nach Standardverfahren.

Zum Aufbau der Seitenkette wird nun entweder, wenn letztendlich $R^{21}$ für ein Wasserstoffatom und $R^{21a}$ für eine Methylgruppe stehen soll, eine Verbindung der allgemeinen Formel III oder eine Verbindung der allgemeinen Formel **IV** (bzw. V) in einer Wittig-Reaktion mit N-Methoxy-N-methyl-2-(triphenylphosphoranyliden)-acetamid (D.A. Evans et al. J. Am. Chem. Soc. **112**, 7001 (1990)) oder einem anderen, analog reagierenden Phosphoranyliden bei erhöhter Temperatur zu einer Verbindung der allgemeinen Formel **VI**

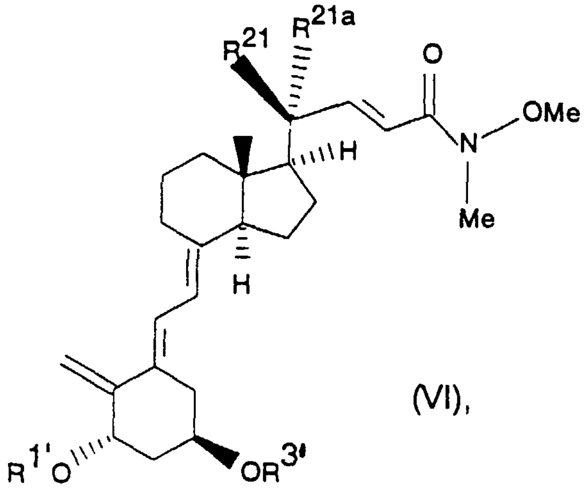

(VI),

worin $R^{1'}$, $R^{3'}$, $R^{21}$ sowie $R^{21a}$ die angegebenen Bedeutungen haben, umgesetzt.

Die Umsetzung läuft vorzugsweise bei einer Temperatur im Bereich zwischen 90 und 120°C in einem Lösungsmittel wie z.B. Dimethylsulfoxid (DMSO) oder Toluol ab.

Im nächsten Reaktionsschritt wird durch Behandlung der Verbindung der allgemeinen Formel **VI** mit einem Reduktionsmittel wie Diisobutylaluminiumhydrid (DIBAH) oder Lithiumaluminiumhydrid in einem Lösungsmittel wie Tetrahydrofuran oder einem anderen Ether bei tiefer Temperatur (-60 bis -100°C) aus dieser der Aminrest $-N(CH_3)(OCH_3)$ vertrieben und der homologe Aldehyd der allgemeinen Formel **VII**

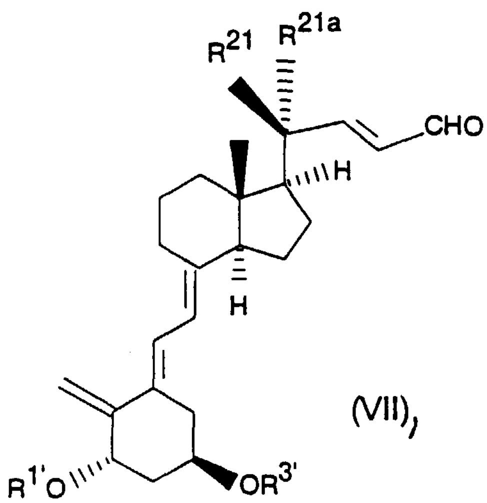

(VII),

erhalten.

Ist darin $R^{21}$ = H und $R^{21a}$ = $CH_3$, handelt es sich hierbei um bereits aus der WO-A-91/00855 bekannte Verbindungen.

Durch Addition einer geeigneten nukleophilen Komponente an die Carbonylfunktion des Aldehydes **VII** läßt sich nun die Seitenkette, wie sie im letztendlich gewünschten 25-Carbonsäure-Derivates der allgemeinen Formel **I** vorliegen soll, aufbauen. Wenn allerdings $R^4$ und $R^{4a}$ gemeinsam mit dem Kohlenstoffatom 25 einen Cyclopropylring bilden sollen, muß die ab Seite 26 beschriebene Syntheseroute beschritten werden.

Unter Einwirkung einer starken Base wie z.B. Lithiumdiisopropylamid, Lithiumdiethylamid, Lithium-, Natrium- oder Kaliumhexamethyldisilazid wird zunächst eine Verbindung der allgemeinen Formel **VIII,**

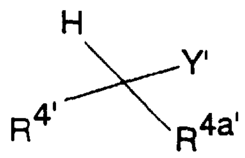

(VIII),

worin $R^{4'}$ und $R^{4a'}$ die schon in der allgemeinen Formel II für $R^4$ und $R^{4a}$ beschriebenen Bedeutungen und Y' die schon in der allgemeinen Formel II beschriebene Bedeutung haben, mit der Ausnahme, daß $R^{4'}$ und $R^{4a'}$ gemeinsam mit dem zentralen Kohlenstoffatom nicht für einen Cyclopropylring stehen können, in einem Lösungsmittel wie Tetrahydrofuran oder einem anderen Ether bei einer Temperatur zwischen -60 und -90°C deprotoniert und anschließend an eine Verbindung der allgemeinen Formel **VII** addiert, wobei eine Verbindung der allgemeinen Formel **IX**

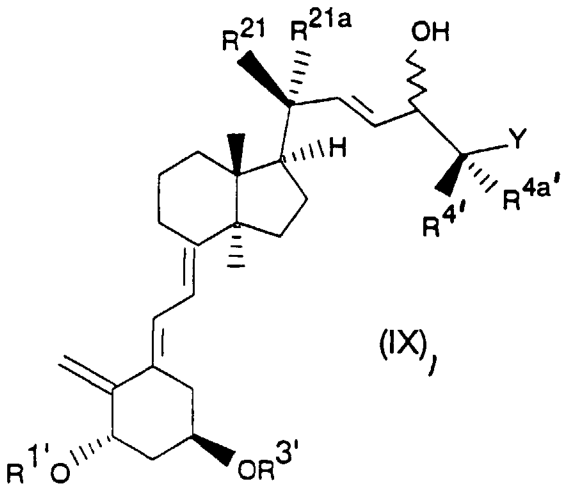

(IX),

worin $R^{1'}$, $R^{3'}$, $R^{4'}$, $R^{4a'}$ sowie Y' die bereits angegebenen Bedeutungen haben,
erhalten wird.

Es entstehen dabei sowohl das 24α- als auch das 24β-Hydroxyisomere, die sich auf dieser oder einer späteren Stufe chromatographisch trennen lassen. In die nachfolgenden Reaktionen können daher je nach Wunsch die getrennten Diastereomeren oder das Gemisch eingesetzt werden.

Im Falle, daß $R^{21}$ = H und $R^{21a}$ = $CH_3$ bedeuten und $R^{19}$ und $R^{19a}$ gemeinsam für eine Methylengruppe stehen (sogenannte "Normalreihe"), zeichnen sich die 24β-Hydroxyverbindungen der allgemeinen Formel I gegenüber den 24α-Hydroxyverbindungen in der Regel durch eine höhere Affinität zum Calcitriol-Rezeptor (niedrigere Rezeptor-Werte KF) aus.

Sollen A und B in der letztendlich gewünschten Verbindung der allgemeinen Formel I gemeinsam ein Ketosauerstoffatom bedeuten, wird die 24-Hydroxyverbindung der allgemeinen Formel IX auf dieser oder einer späteren Stufe mit Braunstein, Pyridiniumchlorochromat, Pyridiniumdichromat, Bariummanganat oder Oxalylchlorid/Dimethylsulfoxid zur 24-Ketoverbindung oxidiert.

Die Umwandlung einer Verbindung der allgemeinen Formel **IX** in die entsprechende Verbindung der allgemeinen Formel **II** erfolgt z.B. durch Bestrahlung mit ultraviolettem Licht in Gegenwart eines sogenannten "Triplettsensibilisators". Im Rahmen der vorliegenden Erfindung wird hierfür Anthracen verwendet. Durch Spaltung der π-Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt. Anschließend wird, falls vorhanden, die 24-Hydroxygruppe gegebenenfalls mit einer Schutzgruppe (Tetrahydrofuranyl-, Tetrahydropyranyl-, Methoxymethyl-, Methoxyethoxymethyl- oder Trimethylsilylethoxymethylgruppe) unter den üblichen, dem Fachmann geläufigen Bedingungen versehen. Diese Vorgehensweise ermöglicht eine saubere Spaltung der Silylschutzgruppen $R^{1'}$ und $R^{3'}$, speziell wenn $R^{1'}$ und $R^{3'}$ je für eine tert.-Butyldiphenylsilylschutzgruppe stehen. Schließlich wird, falls vorhanden, die 24-Hydroxyschutzgruppe in der letzten Stufe unter der katalytischen Einwirkung eines sauren Agens (Pyridinium-p-toluolsulfonat [PPTS], p-Toluolsulfonsäure, Essigsäure, Salzsäure, saurer Ionentauscher) wieder entfernt und die freien Hydroxygruppen gegebenenfalls wie schon beschrieben weiter umgesetzt.
Wenn für $R^{1'}$ und $R^{3'}$ je eine tert.-Butyldimethylsilyl- oder Triisopropylsilylgruppe steht, kann die Schutzgruppenabspaltung direkt, d.h. ohne temporären Schutz der 24-Hydroxygruppe, mit sauren Ionenaustauschern (p-Toluolsulfonsäure, Essigsäure, Salzsäure oder Pyridinium-p-toluolsulfonat) oder durch Einwirkung von Tetrabutylammoniumfluorid (Trihydrat) oder Fluorwasserstoff oder Fluorwasserstoff/Pyridin-Komplex bei Temperaturen unter 30°C durchgeführt werden.

Soll in den letztendlich gewünschten aktiven Verbindungen der allgemeinen Formel I $R^4$ und $R^{4a}$ gemeinsam mit dem Kohlenstoffatom 25 einen 3- oder 4-gliedrigen Cycloalkylrest bilden, muß (im Falle von Cyclopropyl) oder kann (im Falle von Cyclobutyl) ein anderer Weg zur Herstellung der benötigten Verbindungen der allgemeinen Formel II beschritten werden:
Als Startverbindung dient wiederum eine Verbindung der allgemeinen Formel III, an deren Carbonylgruppe unter Einwirkung einer starken Base wie beispielsweise Lithiumdiisopropylamid eine C-H-acide Verbindung der allgemeinen Formel XXVIII (D.F. Taber et al., J. Org. Chem. (1992) 57, 436)

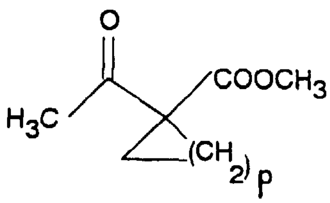

(VIII),

(XXVIII),

worin
p für den Index 1 oder 2 steht,
zur Seitenkettenverlängerung addiert wird. Dabei wird unter Spaltung des Esters die freie 25-Carbonsäure der allgemeinen Formel XXIX

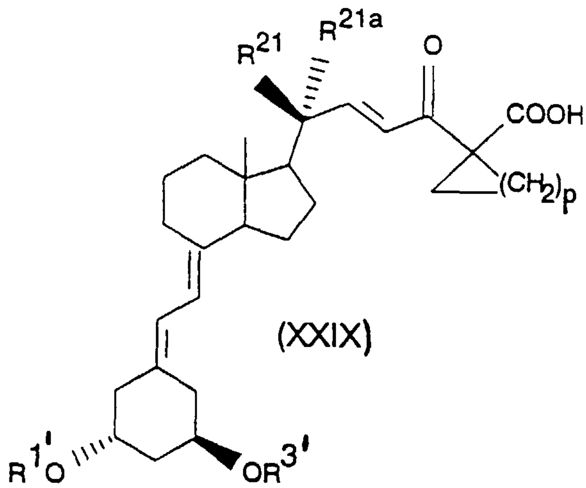

(XXIX)

gebildet, worin $R^{1'}$, $R^{3'}$ sowie p die vorstehend angegebenen Bedeutungen haben.

Soll Y letztendlich nicht eine unsubstituierte Carboxylgruppe sein, gilt der nächste Reaktionsschritt deren Derivatisierung.

Die Carboxylgruppe läßt sich unter milden Bedingungen bei -20 bis -30°C (Synth. Commun. 12, 727-731 (1982)) durch Reaktion einer Verbindung der allgemeinen Formel IX mit Methansulfonylchlorid und Triethylamin aktivieren und durch Behandlung des intermediär gebildeten gemischten Anhydrids mit einem Alkohol der allgemeinen Formel XXX

$R^6OH$ (XXX),

worin
$R^6$ dieselbe Bedeutung wie $R^6$ in Formel I hat, in den entsprechenden Ester der allgemeinen Formel XXXI

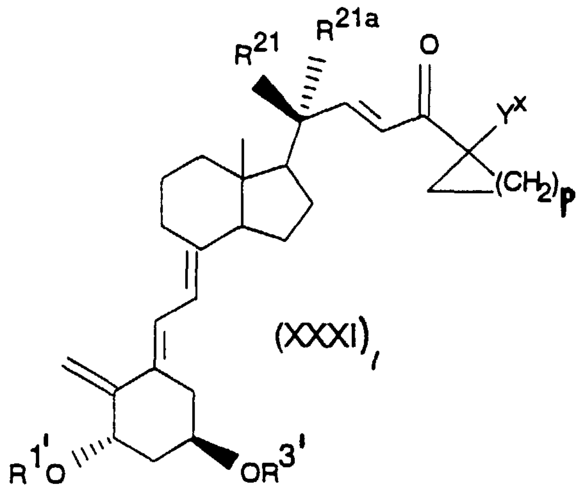

(XXXI),

worin $R^{1'}$, $R^{3'}$ und p die bereits angegebene Bedeutung haben und
$Y^x$ für eine Carbonestergruppe $-C(O)OR^6$ mit den bereits für $R^6$ angegebenen Bedeutungen
stehen.
Die Carbonsäure der allgemeinen Formel XXIX kann auch nach bekannten Verfahren in eine zur Verbindung der allgemeinen Formel XXXI analoge Verbindung überführt werden, in der dann $Y^x$ für eine Amidgruppe $-C(O)NR^5R^{5a}$ oder einen Cyanorest steht.

Anschließend wird die 24-Ketogruppe mit Natriumborhydrid zur 24-Hydroxygruppe reduziert. Dabei werden wie bei der nucleophilen Addition einer Verbindung der allgemeinen Formel VI an einen Aldehyd der allgemeinen Formel IV ebenfalls beide möglichen 24-Hydroxyisomeren der allgemeinen Formel XXXII

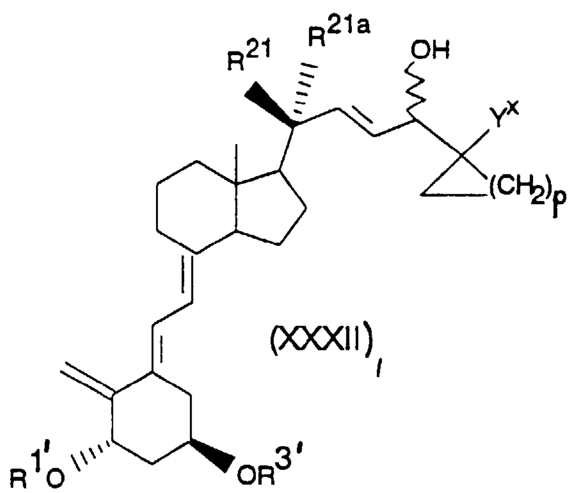

(XXXII),

worin ∿∿ OH eine 24α- oder 24β-Hydroxygruppe bedeutet und die anderen Substituenten sowie p die bereits angegebenen Bedeutungen haben,
erhalten.

Hinsichtlich Trennung der Isomeren und deren Weiterumsetzung gilt das bei den Verbindungen der allgemeinen Formel IX Gesagte.
Ganz analog der Bestrahlung einer Verbindung der allgemeinen Formel IX zur Umwandlung in eine Verbindung der allgemeinen Formel II, wird nun eine Verbindung der allgemeinen Formel XXXII (oder ein Gemisch der entsprechenden 24-Hydroxyisomeren) in die entsprechende Verbindung der allgemeinen Formel II überführt.

Zur Herstellung der gewünschten Endverbindungen der allgemeinen Formel I werden anschließend vorhandene Hydroxyschutzgruppen abgespalten, sowie gewünschtenfalls die freien Hydroxygruppen nach gängigen Verfahren partiell, sukzessive oder vollständig mit dem entsprechenden Carbonsäurehalogenid (Halogenid = Chlorid, Bromid) oder Carbonsäureanhydrid verestert.
Stellt Y in der Verbindung der allgemeinen Formel I eine freie Carboxylgruppe dar, kann diese auch auf der Endstufe nach gängigen Verfahren mit einem den Rest $-OR^6$ liefernden Reagenz verestert werden.
Die Abspaltung der Schutzgruppen der freien Hydroxygruppen gelingt generell durch Behandlung der entsprechenden Verbindung der allgemeinen Formel II mit Tetrabutylammoniumfluorid (Trihydrat) in einem polaren Lösungsmittel wie etwa Tetrahydrofuran, gegebenenfalls unter Zusatz einer geringen Menge Eisessig. Ein eventuell vorhandener 2-(Trimethylsilyl)ethylrest zum Schutz der 25-Carbonsäuregruppe wird hierbei mitabgespalten.

Die Hydroxyschutzgruppen können, außer wenn $R^4$, $R^{4a}$ und das Kohlenstoffatom 25 gemeinsam einen Cyclopropylring bilden, auch durch Behandlung der entsprechenden Verbindung der allgemeinen Formel II mit einem sauren Ionenaustauscher wie etwa "Dowex 50WX8" in einem Lösungsmittel wie Methanol/Methylenchlorid abgespalten werden.
Eine partielle, sukzessive oder vollständige aber unterschiedliche Substitution der freien Hydroxygruppen läßt sich durch Beachtung deren unterschiedlichen Reaktivitäten und durch Verwendung entsprechender molarer Mengen an Veresterungsreagenz erreichen.

Zur Etablierung einer weiteren C-20-Modifikation ($R^{21}$ + $R^{21a}$ = Methylen) wird eine Verbindung der allgemeinen Formel **X**

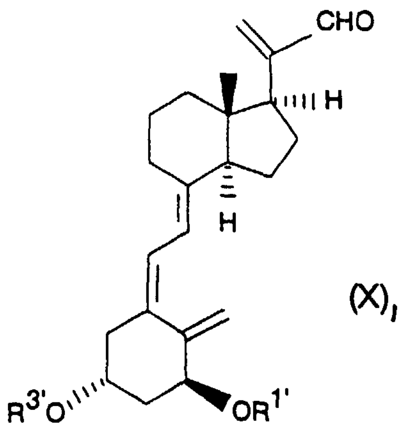

(X),

worin $R^{1'}$ und $R^{3'}$ die bereits angegebenen Bedeutungen haben,
erzeugt,
die sich aus der allgemeinen Formel **IV** dadurch ableitet, daß $R^{21}$ und $R^{21a}$ zusammen eine Methylengruppe bilden und die Isomerisierung des Triensystems bereits erfolgt ist.
Hierzu wird eine Verbindung der allgemeinen Formel **III** analog zu dem in der WO-90/09991 beschriebenen Verfahren (im vorliegenden Fall sind $R^{1'}$ und $R^{3'}$ vorzugsweise tert.-Butyldiphenylsilylgruppen) in eine Verbindung der allgemeinen Formel **XI**

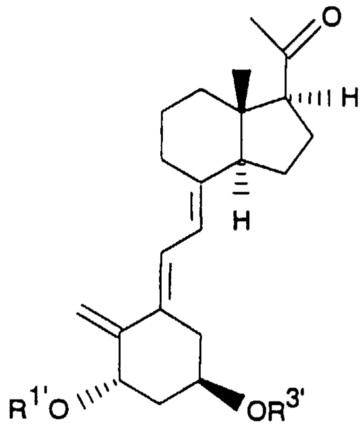

(XI),

worin $R^{1'}$ und $R^{3'}$ die bereits angegebenen Bedeutungen haben, überführt.

Durch Umsetzung einer derartigen 17β-Acetylverbindung der allgemeinen Formel XI mit Schwefelyliden, die aus Reagenzien des Typs $Me_3S^+I^-$ oder $Me_3S^+(O)I^-$ durch Deprotonierung mit einer Base wie Kalium-tert.-butanolat, Natriumhydrid oder Kaliumhydrid erzeugt werden, erhält man eine Verbindung der allgemeinen Formel **XII**

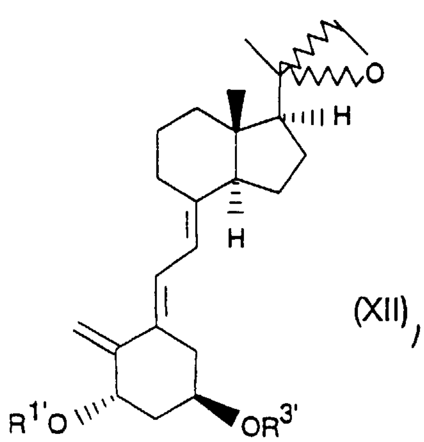

(XII),

worin $R^{1'}$ und $R^{3'}$ die bekannte Bedeutung haben und wobei die Stereochemie an C-20 nicht einheitlich sein muß.

Durch Umlagerung der Epoxide der allgemeinen Formel **XII** mit Basen wie z.B. Lithiumdiisopropylamid, Lithiumdiethylamid oder Aluminiumisopropylat erhält man die Allylalkohole der allgemeinen Formel **XIII,**

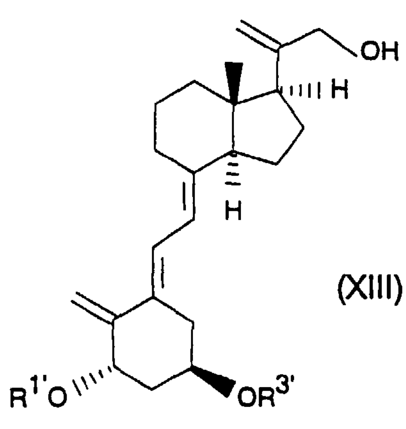

(XIII),

worin $R^{1'}$ und $R^{3'}$ die bekannte Bedeutung haben,
die analog wie schon beschrieben durch photochemische Isomerisierung des Triensystems in eine Verbindung der allgemeinen Formel **XIV**

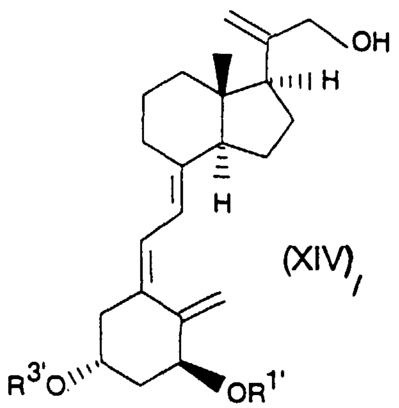

(XIV),

worin $R^{1'}$ und $R^{3'}$ die bekannte Bedeutung haben,
überführt werden.
Deren Umwandlung in eine Verbindung der allgemeinen Formel **X** erfolgt nun durch Oxidation mit einem Oxidationsmittel wie z.B. Mangandioxid, Pyridiniumchlorochromat, Pyridiniumdichromat, Bariummanganat oder Oxalylchlorid/DMSO nach bekannten Methoden.

Eine weitere C-20-Modifikation ($R^{21}$ + $R^{21a}$ + C20 = Cyclopropyl) wird durch Umsetzung einer Verbindung der allgemeinen Formel **XIV** mit einem metallorganischen Reagenz vom Typ $I-CH_2-Zn-I$, das aus Zn/Cu, Zn/Ag oder $Et_2Zn$ mit $CH_2I_2$ gebildet wird (Simmons-Smith-Reaktion), eingeführt, wobei eine Verbindung der allgemeinen Formel **XV**

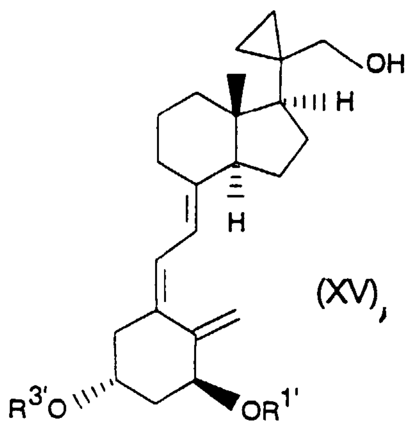

(XV),

worin $R^{1'}$ und $R^{3'}$ die bekannten Bedeutungen haben,
entsteht.

Die primäre Alkoholfunktion in einer Verbindung der allgemeinen Formel **XV** wird nun mit einem Oxidationsmittel wie z.B. Pyridiniumchlorochromat, Pyridiniumdichromat oder Oxalylchlorid/DMSO unter Bildung einer Verbindung der allgemeinen Formel **XVI**

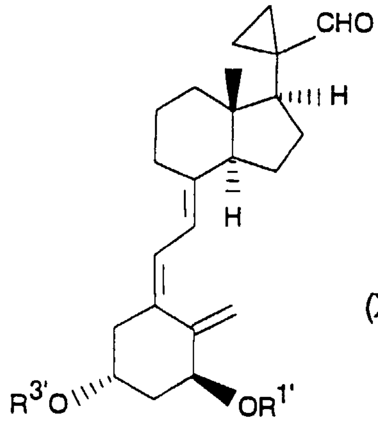

(XVI),

worin $R^{1'}$ und $R^{3'}$ die bekannten Bedeutungen haben,
umgesetzt,
die sich aus den Verbindungen der allgemeinen Formel **IV** dadurch ableitet, daß $R^{21}$ und $R^{21a}$ gemeinsam mit dem quartären Kohlenstoffatom C-20 einen Cyclopropylring bilden und das Triensystem bereits isomerisiert ist.

Die Einführung der Seitenketten erfolgt nun an einer Verbindung der allgemeinen Formel X oder XVI ganz analog wie von einer Verbindung der allgemeinen Formel III ausgehend beschrieben, über die Zwischenstufen der allgemeinen Formeln XVII, XVIII und XIX, wobei letzendlich eine Verbindung der allgemeinen Formel II erhalten wird, worin $R^{19}$ und $R^{19a}$ in beiden Fällen gemeinsam eine Methylengruppe bilden und $R^{21}$ und $R^{21a}$ gemeinsam ebenfalls eine Methylengruppe oder gemeinsam mit C20 einen Cyclopropylring bilden. $R^{1'}$, $R^{3'}$, $R^4$, $R^{4a}$ und Y' haben die bereits angegebene Bedeutung.

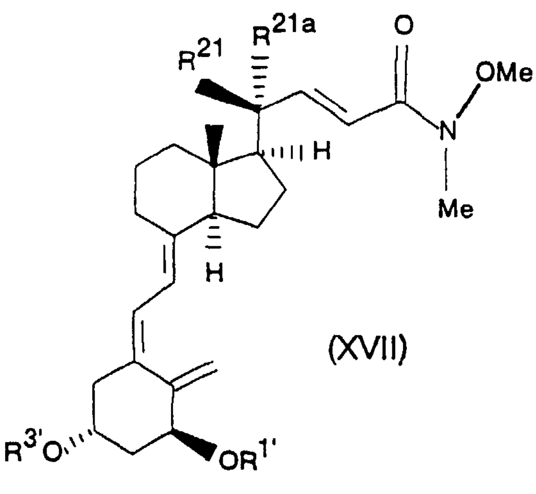

(XVII)

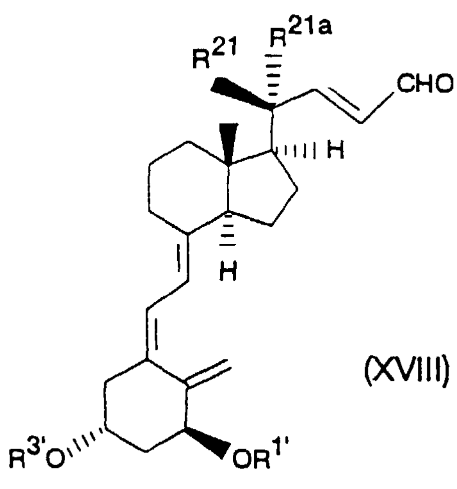

(XVIII)

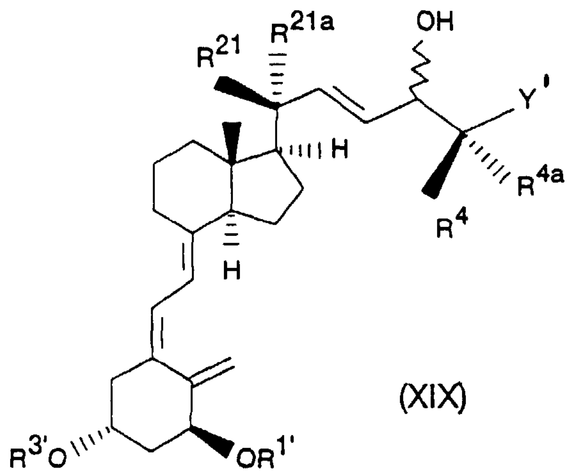

(XIX)

Die Diastereomerentrennungen bzw. Freisetzungen und Derivatisierungen der Hydroxygruppen erfolgen wie schon beschrieben. Gewünschtenfalls wird vor der Schutzgruppenabspaltung wiederum die 24-Hydroxygruppe analog wie schon beschrieben zur Ketofunktion oxidiert.

Zur Synthese der Verbindungen der allgemeinen Formel II, worin $R^{19}$ und $R^{19a}$ jeweils ein Wasserstoffatom bedeuten ("19-Nor-Reihe") verwendet man den literaturbekannten Aldehyd XX

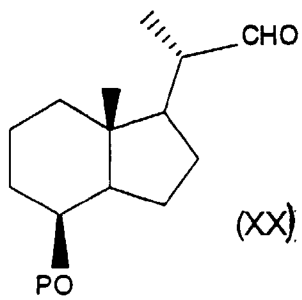

(XX),

(H.H. Inhoffen et al., Chem. Ber. 91, 780 (1958), Chem Ber. 92, 1772 (1959); W,G. Dauben et al., Tetrahedron Lett. 30, 677 (1989)), worin P eine acyl-, alkyl- oder arylsubstituierte Silyl- oder Tetrahydropyranyl-, Tetrahydrofuranyl- oder Methoxymethyl-oder eine andere Alkoholschutzgruppe bedeutet.

Die strukturellen Modifikationen am Kohlenstoffatom 20 können analog zu den schon für die Vitamin D-Derivate vorstehend beschriebenen Verfahren erfolgen, wobei Verbindungen der allgemeinen Formel XXI

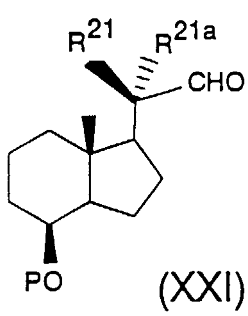

(XXI)

entstehen,

worin P die bereits angegebene Bedeutung hat und $R^{21}$ und $R^{21a}$ je eine Methylgruppe, zusammen eine Methylengruppe oder gemeinsam mit dem Kohlenstoffatom 20 einen Cyclo-propylring bedeuten. Weiterhin können $R^{21}$ ein

Fluoratom und $R^{21a}$ eine Methy!gruppe bzw. $R^{21}$ eine Methylgruppe und $R^{21a}$ ein Fluoratom bedeuten.

Diese Substitutionsmuster können wie folgt aufgebaut werden:

Eine Verbindung der allgemeinen Formel XXI wird mit einer Base wie Diisopropylethylamin, Triethylamin oder 2,5-Di-tert.-Butylpyridin und einem Silylierungsreagenz wie Tri-methylsilylchlorid oder Trimethylsilyltrifluorsulfonat zum E,Z-Gemisch der Silylenolether der allgemeinen Formel XXII umgesetzt

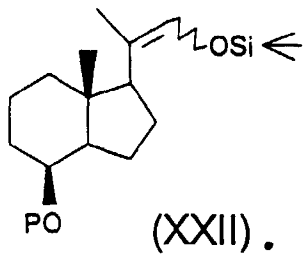

(XXII).

Reaktion mit einem elektrophilen Fluorierungsreagenz (z.B. N-Fluordiphenylsulfonimid o.ä.) (E. Differding, H. Ofner, Synlett 187 (1991)) liefert die α-Fluoraldehyde der allgemeinen Formel XXI vorstehender Definition. Eventuell auftretende Diastereomere können chromatographisch getrennt und separat weiter umgesetzt werden.

Die wie beschrieben in Position 20 modifizierten Verbindungen aber auch Verbindungen der allgemeinen Formel XXI, für die gilt $R^{21}$ = Wasserstoff und $R^{21a}$ = Methyl (Normalkonfiguration) oder $R^{21}$ = Methyl und $R^{21a}$ = Wasserstoff ("20-Epi-Reihe") werden wie bereits für die Vitamin D-Derivate vorstehend beschrieben (analog III→VI) in Verbindungen der allgemeinen Formel XXIII

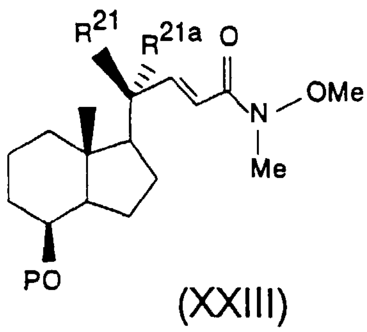

(XXIII)

umgewandelt.

Die Schutzgruppe P in der Verbindung der allgemeinen Formel XXIV wird nun unter Standardbedingungen gespalten:

Für Silyletherschutzgruppen wird Tetrabutylammoniumfluorid (TBAF), Fluorwasserstoff oder Fluorwasserstoff-Pyridin-Komplex verwendet;

für Tetrahydropyranyl-, Methoxymethyl- oder Methoxyethoxymethylether werden saure Reaktionsbedingungen (Pyridinium-p-toluolsulfonat, p-Toluolsulfonsäure, Essigsäure, Salzsäure, saure Ionentauscher) benutzt, wobei eine Verbindung der allgemeinen Formel XXIV

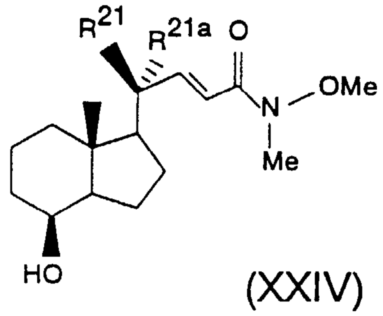

(XXIV)

entsteht.

Die freie Hydroxylgruppe wird mit einem Oxidationsmittel (Pyridiniumchlorochromat, Pyridiniumdichromat, Bariummanganat, Oxalylchlorid oder Dimethylsulfoxid) zum Keton oxidiert (Verbindung der allgemeinen Formel XXV)

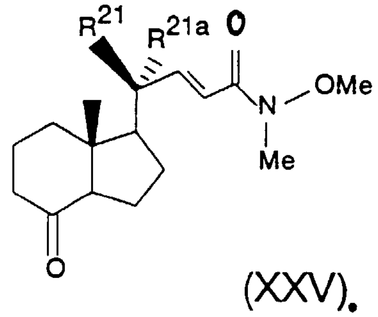

(XXV).

Die Verbindungen der allgemeinen Formel XXV werden nun durch Reaktion mit dem durch eine Base wie n-Butyllithium oder Lithiumdiisopropylamin erzeugten Anion des literaturbekannten Phosphinoxides der allgemeinen Formel XXVI (H.F. DeLuca et al., Tetrahedron, Lett. 32, 7663 (1991))

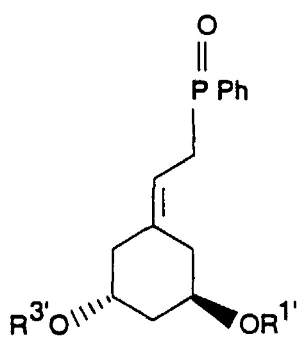

(XXVI),

worin $R^{1'}$ und $R^{3'}$ wie bereits vorstehend in Formel II angegeben, alkyl- oder aryl- oder alkyl-arylsubstituierte Silylgruppen bedeuten,

in die entsprechenden Verbindungen der allgemeinen Formel XXVII

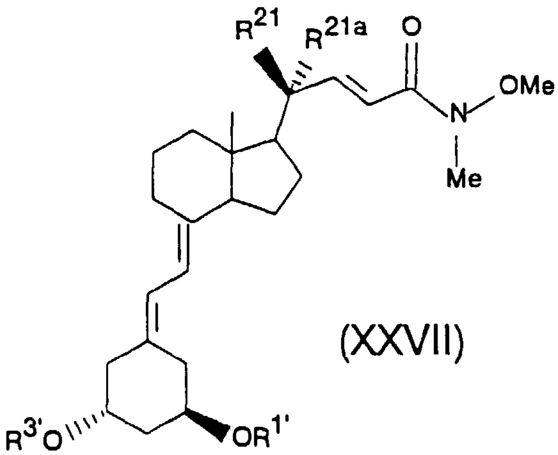

(XXVII)

überführt (Horner-Wittig-Reaktion).

Analog zur "Normalreihe" wird über eine Zwischenstufe der allgemeinen Formel XXIII

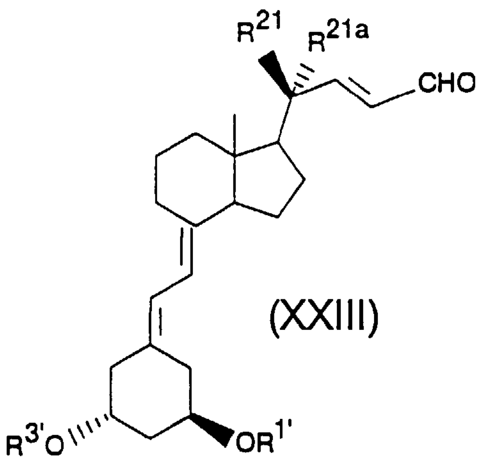

(XXIII)

die entsprechende Verbindung der allgemeinen Formel II, worin $R^{19}$ und $R^{19a}$ je für ein Wasserstoffatom stehen, erzeugt und diese dann wie vorstehend beschrieben in eine Verbindung der allgemeinen Formel I überführt.

Die nachfolgenden Beispiele dienen der näheren Erläuterung der Erfindung.

**Herstellung der Ausgangsverbindungen in der Normalreihe**

**1.) (5*E*,7*E*,22*E*)-(1*S*,3*R*)-1,3-Bis[[dimethyl(1,1-dimethylethyl)silyl]oxy]-N-methoxy-N-methyl-9,10-secochola-5,7,10(19),22-tetraen-24-amid 2**

17,65 g Aldehyd 1 (Calverley, Tetrahedron 43 4609 (1987)) und 26,97 g N-Methoxy-N-methyl-2-(triphenylphosphoranyliden)acetamid (D.A. Evans et al., J. Am. Chem. Soc. 112 7001 (1990)) werden 6 Stunden bei 105°C in 101 ml DMSO gerührt. Die abgekühlte Reaktionsmischung wird in NaCl-Lösung eingerührt, mit Ethylacetat extrahiert, über Natriumsulfat getrocknet und eingeengt. Der Rückstand wird an Kieselgel mit Ethylacetat gereinigt. Es werden 14,9 g von 2 als farbloses kristallisiertes Öl erhalten.

$^{1}$H-NMR(300 MHz, $CDCl_3$): δ = 0,05 ppm (s,12H); 0,57 (s,3H); 0,84 (s,9H); 0,87 (s,9H); 1,10 (d,7Hz,3H); 3,24 (s, 3H); 3,72 (s,3H); 4,23 (m,1H); 4,53 (m,1H); 4,94 (br.s,1H); 4,99 (br.s,1H); 5,83 (d, J=11Hz,1H); 6,33 (d,J=15Hz,1H); 6,45 (d,J=11Hz,1H); 6,85 (dd,J=15Hz, J=9Hz,1H).

**2.) (5*E*,7*E*,22*E*)-(1*S*,3*R*)-1,3-Bis [[dimethyl(1,1-dimethylethyl)silyl]oxy]-9,10-secochola-5,7,10(19),22-tetraen-24-al 3**

10,7 g 2 in 54 ml Tetrahydrofuran werden bei -78°C unter Stickstoff mit 68,2 ml Diisobutylaluminiumhydrid (1,2 molar in Toluol) tropfenweise versetzt und noch 70 Minuten bei -78°C gerührt. Nach tropfenweiser Zugabe von 3,66 ml Methanol bei -78°C wird die Reaktionsmischung in 1 l Eis/Kalium-Natriumtartrat-Lösung eingerührt. Es werden 700 ml Ether zugesetzt, 1,5 Stunden gerührt, mit Ether extrahiert, über Natriumsulfat getrocknet und eingeengt. Durch Reinigung des öligen Rückstandes an Kieselgel mit Ethylacetat/Hexan werden 8,86 g von 3 als hellgelbe Masse erhalten.

**3.) (5*E*,7*E*,22*E*)-(1*S*,3*R*,24*R*)-1,3-Bis[[dimethyl(1,1-dimethylethyl)silyl]oxy]-24-hydroxy-9,10-secocholesta-5,7,10(19),22-tetraen-25-carbonsäureethylester 4a**

4,6 ml Butyllithium (1.6 molar in Hexan) werden bei Eiskühlung unter Stickstoff zu 1,13 ml Diisopropylamin in 51,6 ml Tetrahydrofuran getropft und noch 15 Minuten gerührt. Danach werden bei -78°C 0,99 ml Isobuttersäureethylester zugetropft und 75 Minuten nachgerührt. Anschließend werden bei gleicher Temperatur 2,0 g 3 in 6,0 ml Tetrahydrofuran zugetropft
und weitere 75 Minuten bei -78°C gerührt. Die Reaktionsmischung wird bei -78°C mit gesättigter $NH_4Cl$-Lösung versetzt, bei -10°C mit eisgekühlter NaCl-Losung verdünnt, mit Ether extrahiert, über Natriumsulfat getrocknet und eingeengt. Durch Chromatographie des öligen Rückstandes an Kieselgel mit Ethylacetat/Hexan erhält man in der Elutionsreihenfolge 730 mg (5*E*,7*E*,22*E*)-(1*S*,3*R*,24*S*)-1,3-Bis[[dimethyl(1,1-dimethylethyl)silyl]oxy]-24-hydroxy-9,10-secocholesta-5,7,10(19),22-tetraen-25-carbonsäureethylester (4b) als kristallisiertes Öl und 520 mg der Titelverbindung 4a als farbloses Öl.
In den nachfolgenden Reaktionen wird - bis auf eine Ausnahme - nur die weitere Umsetzung von 4a beschrieben.

**4.) (5*Z*,7*E*,22*E*)-(1*S*,3*R*,24*R*)-1,3-Bis[[dimethyl(1,1-dimethylethyl)silyl]oxy]-24-hydroxy-9,10-secocholesta-5,7,10(19),22-tetraen-25-carbonsäureethylester 5a**

520 mg 4a werden in 68 ml Toluol gelöst und nach Zugabe von 80 mg Anthracen und 1 Tropfen Triethylamin 13 Minuten unter Stickstoff mit einer Quecksilberhochdrucklampe (Heraeus TQ 150) durch Pyrex-Glas bestrahlt. Die Reaktionsmischung wird eingeengt und der Rückstand (600 mg) - ein Gemisch aus 5a und Anthracen - direkt der nachfolgenden Silyletherspaltung unterworfen.

**5.) (5Z,7*E*,22*E*)-(1*S*,3*R*,24*S*)-1,3-Bis[[dimethyl(1,1-dimethylethyl)silyl]oxy]-24-hydroxy-9,10-secocholesta-5,7,10(19),22-tetraen-25-carbonsäureethylester 5b**

730 mg 4b (vergl. Vorschrift 3) werden in 95 ml Toluol gelöst und nach Zugabe von 80 mg Anthracen und 2 Tropfen Triethylamin 15 Minuten unter Stickstoff mit einer Quecksilberhochdrucklampe (Heraeus TQ 150) durch Pyrex-Glas bestrahlt. Die Reaktionsmischung wird eingeengt und der Rückstand (845 mg) - ein Gemisch aus 5b und Anthracen - direkt der nachfolgenden Silyletherspaltung unterworfen.

**6.) (5*E*,7*E*,22*E*)-(1*S*,3*R*)-1,3-Bis[[dimethyl(1,1-dimethylethyl)silyl]oxy]-24-oxo-26'27-cyclo-9,10-secocholesta-5,7,10(19),22-tetraen-25-carbonsäure 6**

38,8 ml Butyllithium (1,6 molar in Hexan) werden bei Eiskühlung unter Stickstoff zu 9,5 ml Diisopropylamin in 607 ml Tetrahydrofuran getropft und noch 15 Minuten gerührt. Danach werden bei - 78°C 8,83 g 1-Acetylcyclopropancarbonsäure-methylester (D.F. Taber et al., J. Org. Chem. (1992) 57, 436) zugetropft und 1 Stunde gerührt. Anschließend werden bei -78°C 5,96 g Aldehyd 1 (Calverley, Tetrahedron 43 4609 (1987)) in 18,4 ml Tetrahydrofuran zugetropft und 1,5 Stunden gerührt. Danach läßt man die Reaktionsmischung innerhalb 1,5 Stunden auf 0°C kommen und rührt noch 15 Minuten bei dieser Temperatur. Nach Zugabe von gesättigter Ammoniumchlorid-Lösung bei -20°C wird mit gesättigter Natriumchlorid-Lösung bei Raumtemperatur verdünnt, mit Ethylacetat unter Zusatz von 5%iger Oxalsäure extrahiert, über Natriumsulfat getrocknet und eingeengt. Das so erhaltene Rohprodukt (11,90 g gelbes Öl) wird ohne weitere Reinigung der nachfolgenden Veresterung unterworfen.
Durch dünnschichtchromatographische (Kieselgel, Ethylacetat/Hexan) Aufreinigung wird eine NMR-Probe erhalten.

NMR (300 MHz,$CDCl_3$): δ= 0,05 ppm (s,12H); 0,57 (s,3H); 0,84 (s,9H); 0,87 (s,9H); 1,10 (d,J=7Hz,3H); 1,73 (m, 2H); 2,03(m,2H); 4,23 (m,1H); 4,53 (m,1H); 4,94 (br.s,1H); 4,97 (br.s,1H); 5,82 (d,J=11Hz,1H); 5,85 (d,J=15Hz,1H); 6,43 (d,J=11Hz,1H); 7,13 (dd,J=15Hz, J=9Hz,1H).

**7.) (5*E*,7*E*,22*E*)-(1*S*,3*R*)-1,3-Bis [[dimethyl(1,1-dimethylethyl)silyl]oxy]-24-oxo-26,27-cyclo-9,10-secocholesta-5,7,10(19),22-tetraen-25-carbonsäuremethylester 7**

11,90 g 6 in 119 ml Methylenchlorid werden bei -30°C nacheinander mit 7,28 ml Triethylamin und 2,06 ml Mesylchlorid tropfenweise versetzt und 1 Stunde gerührt. Anschließend werden bei -30°C nacheinander 3,53 ml Methanol und 0,43 g 4-Dimethylaminopyridin hinzugegeben. Nach 1,25 Stunden bei -10°C wird die Reaktionsmischung auf Eis/Natriumhydrogencarbonat-Lösung gegossen. Anschließend wird mit Natriumchlorid-Lösung verdünnt, mit Ethylacetat extrahiert, über Natriumsulfat getrocknet und eingeengt. Chromatographie des öligen Rückstandes an Kieselgel mit Ethylacetat/Hexan liefert 4,10 g 7 als farblose Masse.

$^1$H-NMR (300 MHz,$CDCl_3$): δ= 0,05 ppm (s,12H); 0,57 (s,3H); 0,85 (s,9H); 0,89 (s,9H); 1,10 (d,J=7Hz,3H); 1,45 (m,4H); 3,73 (s,3H); 4,21 (m,1H); 4,53 (m,1H); 4,93 (br.s,1H); 4,95 (br.s,1H); 5;82 (d,J=11Hz,1H); 6,40 (d,J=15Hz,1H); 6,44 (d,J=11Hz,1H); 6,75 (dd,J=15Hz, J=9Hz,1H).

**8.) (5*E*,7*E*,22*E*)-(1*S*,3*R*,24*R*)-1,3-Bis[[dimethyl(1,1-dimethylethyl)silyl]oxy]-24-hydroxy-26,27-cyclo-9,10-secocholesta-5,7,10(19),22-tetraen-25-carbonsäure-methylester 8a**

2,0g 7 in 4,8 ml Tetrahydrofuran und 5,8 ml Methanol werden mit 11,2 ml einer 0,4 molaren methanolischen Cer (III)-chlorid-Heptahydrat-Lösung versetzt.

Unter Stickstoff werden bei Eiskühlung 310 mg Natriumborhydrid portionsweise hinzugegeben. Die Reaktionsmischung wird noch 45 Minuten bei Eiskühlung gerührt und danach mit Eis/Wasser versetzt. Anschließend wird mit Ethylacetat extrahiert, über Natriumsulfat getrocknet und eingeengt. Durch Chromatographie des Rückstandes an Kieselgel mit Ethylacetat/Hexan erhält man in der Elutionsreihenfolge 610 mg (5*E*,7*E*,22*E*)-(1*S*,3*R*,24*S*)-1,3-Bis[[dimethyl(1,1-dimethylethyl)silyl]oxy]-24-hydroxy-26,27-cyclo-9,10-secocholesta-5,7,10(19),22-tetraen-25-carbonsäuremethylester 8b und 370 mg der Titelverbindung 8a als kristallisiertes Öl.

In den nachfolgenden Reaktionen wird nur die weitere Umsetzung von 8a beschrieben.

**9.) (5*Z*,7*E*,22*E*)-(1*S*,3*R*,24*R*)-1,3-Bis[[dimethyl(1,1-dimethylethyl)silyl] oxy]-24-hydroxy-26,27-cyclo-9,10-secocholesta-5,7,10(19),22-tetraen-25-carbonsäuremethylester 9a**

370 mg 8a werden in 53 ml Toluol gelöst und nach Zugabe von 61 mg Anthracen und 1 Tropfen Triethylamin 5 Minuten unter Stickstoff mit einer Quecksilberhochdrucklampe (Heraeus TQ 150) durch Pyrex-Glas bestrahlt. Die Reaktionsmischung wird eingeengt und der Rückstand (435 mg) - ein Gemisch aus 9a und Anthracen - direkt der nachfolgenden Silyletherspaltung unterworfen.

**Beispiel 1**

**10.) (5*Z*,7*E*,22*E*)-(1*S*,3*R*,24*R*)-1,3,24-Trihydroxy-9,10-secocholesta-5,7,10(19),22-tetraen-25-carbonsäureethylester 10a**

600 mg des Rückstandes von 5a werden mit 9,23 g Dowex 50WX8 in 22,5 ml Methanol/ Methylenchlorid (9:1) 25 Stunden bei Raumtemperatur gerührt. Die Suspension wird filtriert, das Filtrat eingeengt und der Rückstand an Kieselgel mit Ethylacetat/Hexan chromatographiert. Es werden 201 mg 10a als Schaum erhalten.

$^1$H-NMR(300 MHz,$CDCl_3$): δ = 0,58 ppm (s,3H); 1,05 (d,J=7Hz,3H); 1,17 (s,6H); 1,28 (t,J=7Hz,3H); 2,60 (m,2H); 4,08 (br.t,1H); 4,15 (q,J=7Hz,2H); 5,00 (br.s,1H); 5,32 (br.s,1H); 5,36 (dd,J=15Hz, J=7,5Hz,1H); 5,55 (dd,J=15Hz, J=9Hz,1H); 6,02 (d,J=11Hz,1H); 6,48 (d,J=11Hz,1H).

**Beispiel 2**

**11.) (5*Z*,7*E*,22*E*)-(1*S*,3*R*,24*S*)-1,3,24-Trihydroxy-9,10-secocholesta-5,7,10(19),22-tetraen-25-carbonsäureethylester 10b**

845 mg des Rückstandes von 5b werden mit 12,95 g Dowex 50WX8 in 31,6 ml Methanol/Methylenchlorid (9:1) 25 Stunden bei Raumtemperatur gerührt. Die Suspension wird filtriert, das Filtrat eingeengt und der Rückstand an Kieselgel mit Ethylacetat/Hexan chromatographiert. Es werden 251 mg der Titelverbindung vom Schmelzpunkt 133-134°C isoliert.

**Beispiel 3**

**12.) (5*Z*,7*E*,22*E*)-(1*S*,3*R*,24*R*)-26,27-Dimethyl-1,3,24-trihydroxy-9,10-secocholesta-5,7,10(19),22-tetraen-25-carbonsäuremethylester 11a**

Ausgehend von Aldehyd 3 und 2-Ethylbuttersäuremethylester wird analog der Sequenz der Synthese von 10a aus 4a die Titelverbindung 11a als Schaum erhalten.

$^1$H-NMR(300 MHz,$CDCl_3$): δ = 0,57 ppm (s,3H); 0,82 (t,J=7,5Hz,3H); 0,88 (t,J=7,5Hz,3H); 1,05 (d,J=7Hz,3H); 1,72 (q,J=7,5Hz); 2,95 (d,J=6,5Hz,1H); 3,72 (s,3H); 4,15 (br.t,1H); 4,23 (m,1H); 4,42 (m,1H); 5,00 (br.s,1H); 5,32 (br.s,1H); 5,37 (dd,J=15Hz, J=7,5Hz,1H); 5,55 (dd, J=15Hz, J=9Hz, 1H); 6,02 (d,J=11Hz,1H); 6,39 (d,,J=11Hz,1H).

**Beispiel 4**

**13.) (5*Z*,7*E*,22*E*)-(1*S*,3*R*,24*R*)-1,3,24-Trihydroxy-9,10-secocholesta-5,7,10(19),22-tetraen-25-carbonsäure-1-methylethylester 13a**

Ausgehend von 1,82 g Aldehyd 3 und Isobuttersäureisopropylester werden analog zu 4a und 5a 580 mg (5*Z*,7*E*,22*E*)-(1*S*,3*R*,24*R*)-1,3-Bis[[dimethyl(1,1-dimethylethyl)silyl]oxy]-24-hydroxy-9,10-secocholesta-5,7,10(19),22-tetraen-25-carbonsäure-1-methylethyl 12a - verunreinigt mit Anthracen - erhalten.
Zur Silyletherspaltung werden 20 ml Tetrahydrofuran und 1,06 g Tetrabutylammoniumfluorid Trihydrat hinzugegeben. Die Reaktionsmischung wird 50 Minuten bei 60°C gerührt und nach Abkühlung auf Eis/Natriumhydrogencarbonatlösung gegossen. Danach wird mit Ethylacetat extrahiert, über Natriumsulfat getrocknet und eingeengt. Chromatographie an Kieselgel mit Ethylacetat/Hexan ergeben 68 mg 13a als Schaum.

$^1$H-NMR (300 MHz,$CDCl_3$); δ = 0,57 ppm (s,3H); 1,05(d,J=7Hz,3H); 1,16 (s,3H); 1,17 (s,3H); 1,27 (d,J=7Hz,6H); 2,70 (br.d,1H); 4,05 (m,1H); 4,24 (m,1H); 4,44 (m,1H); 5,05 (m,2H); 5,31(br.s,1H); 5,36 (dd,J=15Hz, J=7,5Hz,1H); 5,51 (dd,J=15Hz, J=9Hz,1H); 6,02 (d,J=11Hz,1H); 6,38 (d,J=11Hz,1H).

**Beispiel 5**

**14.) (5*Z*,7*E*,22*E*)-(1*S*,3*R*,24*R*)-1,3,24-Trihydroxy-9,10-secocholesta-5,7,10(19),22-tetraen-25-carbonsäuremethylester 15a**

Ausgehend von 1,82 g Aldehyd 3 und 0,77 ml Isobuttersäuremethylester werden analog zu 4a und 5a 540 mg (5*Z*,7*E*,22*E*)-(1*S*,3*R*,24*R*)-1,3-Bis[[dimethyl(1,1-dimethylethyl)-silyl]oxy]-24-hydroxy-9,10-secocholesta-5,7,10(19), 22-tetraen-25-carbonsäuremethylester 14a - verunreinigt mit Anthracen - erhalten.
Zur Silyletherspaltung wird in 5 ml Tetrahydrofuran und 0,098 ml Eisessig gelöst. Nach dreimaliger Zugabe von 865 mg Tetrabutylammoniumfluorid.Trihydrat und jeweiliger Behandlung (2 Stunden 40°C; 1,5 Stunden 40°C und 12 Stunden Raumtemperatur; 5 Stunden 60°C) werden bei üblicher Aufarbeitung (vergl. 13a) 110 mg der Titelverbindung vom Schmelzpunkt 145°C isoliert.

**Beispiel 6**

**15.) (5*Z*,7*E*,22*E*)-(1*S*,3*R*,24*S*)-1,3,24-Trihydroxy-9,10-secochola-5,7,10(19),22-tetraen-24-essigsäuremethylester 16a**

Ausgehend von Aldehyd 3 und Essigsäuremethylester wird analog der Reaktionssequenz zu 15a die Titelverbindung 16a als Schaum erhalten.

$^1$H-NMR(300 MHz,$CDCl_3$): δ= 0,57 ppm (s,3H); 1,05 (d,J=7Hz,3H); 2,54 (d,J=5Hz,2H); 2,70 (br.d,1H); 3,71 (s, 3H); 4,23 (m,1H); 4,47 (m,2H); 5,00 (br.s,1H); 5,32 (br.s,1H); 5,40 (dd,J=15Hz, J=7,5Hz,1H); 5,56 (dd,J=15Hz, J=9Hz, 1H); 6,01 (d,J=11Hz,1H); 6,38 (d,J=11Hz,1H).

**Beispiel 7**

**16.) (5*Z*,7*E*,22*E*)-(1*S*,3*R*,24*R*)-1,3,24-Trihydroxy-9,10-secocholesta-5,7,10(19),22-tetraen-25-carbonsäurenitril 17a**

Ausgehend von Aldehyd 3 und Isobuttersäurenitril wird analog der Sequenz der Synthese von 6a aus 4a die Titelverbindung 17a als kristallisiertes Öl vom Schmelzpunkt 138°C erhalten.

**Beispiel 8**

**17.) (5*Z*,7*E*,22*E*)-(1*S*,3*R*,24*R*)-1,3,24-Trihydroxy-9,10-secocholesta-5,7,10(19),22-tetraen-25-carbonsäurepropylester 19a**

Ausgehend von Aldehyd 3 und Isobuttersäurepropylester wird analog der Sequenz der Synthese von 6a aus 4a über das Zwischenprodukt (5*Z*,7*E*,22*E*)-(1*S*,3*R*,24*R*)-1,3-Bis-[[dimethyl(1,1-dimethylethyl)silyl]oxy]-24-hydroxy-9,10-secocholesta-5,7,10(19),22-tetraen-25-carbonsäurepropylester 18a die Titelverbindung 19a als Feststoff vom Schmelzpunkt 123°C erhalten.

**Beispiel 9**

**18.) (5*Z*,7*E*,22*E*)-(1*S*,3*R*,24*R*)-1,3,24-Trihydroxy-9,10-secocholesta-5,7,10(19),22-tetraen-25-carbonsäure 21a**

Ausgehend von 3,10 g Aldehyd 3 und 2,92 g Isobuttersäure-2-(trimethylsilyl)ethylester - hergestellt aus Isobuttersäure und 2-(Trimethylsilyl)-ethanol in Gegenwart von N,N'-Di-cyclohexylcarbodiimid und 4-Dimethylaminopyridin (vergl. Tetrahedron Lett. (1978) 4475 - werden analog der Synthese von 4a und 5a 1,04 g (5*Z*,7*E*,22*E*)-(1*S*,3*R*,24*R*)-1,3-Bis-[[dimethyl(1,1-dimethylethyl)silyl]oxy]-24-hydroxy-9,10-secocholesta-5,7,10(19),22-tetraen-25-carbonsäure-2-(trimethylsilyl)ethylester 20a - verunreinigt mit Anthracen - erhalten.
Zur Silylether- und Esterspaltung werden 100 mg 20a in 3,7 ml Tetrahydrofuran mit 196 mg Tetrabutylammoniumfluorid Trihydrat 5 Tage bei Raumtemperatur stehengelassen. Die Reaktionsmischung wird auf Eis/Ammoniumchloridlösung gegossen und mit Ethylacetat extrahiert. Nach Trocknung über Natriumsulfat wird eingeengt und der farblose, feste Rückstand in Ethylacetat gerührt. Durch Filtration der Suspension werden 23 mg der Titelverbindung 21a vom Schmelzpunkt 208°C (Zers.) erhalten.

**Beispiel 10**

**19.) (5*Z*,7*E*,22*E*)-(1*S*,3*R*,24*R*)-1,3,24-Trihydroxy-26,27-cyclo-9,10-secocholesta-5,7,10(19),22-tetraen-25-carbonsäuremethylester 22a**

435 mg des Rückstandes von 9a in 15,4 ml Tetrahydrofuran werden mit 815 mg Tetrabutylammoniumfluorid Trihydrat über Nacht bei Raumtemperatur stehengelassen. Die Reaktionsmischung wird auf Eis/Natriumhydrogencarbonat-Lösung gegossen und Natriumchlorid-Lösung hinzugesetzt. Danach wird mit Ethylacetat extrahiert, über Natriumsulfat getrocknet und eingeengt. Chromatographie des Rückstandes an Kieselgel mit Ethylacetat/Hexan ergeben 130 mg einer hellbraunen Masse. Nach Umkristallisation in Ethylacetat/Cyclohexan werden 45 mg 22a vom Schmelzpunkt 100-101°C erhalten.

**Beispiel 11**

**20.) (5*Z*,7*E*,22*E*)-(1*S*,3*R*,24*R*)-1,3,24-Trihydroxy-26,27-cyclo-9,10-secocholesta-5,7,10(19),22-tetraen-25-carbonsäureethylester 23a**

Ausgehend von Carbonsäure 6 und Ethanol wird analog der Sequenz der Synthese von 22a aus 7 die Titelverbindung 23a als Schaum erhalten.
$^1$H-NMR (300 MHz,$CDCl_3$): $\delta$ = 0,57 ppm (s,3H); 0,86 (m,1H); 0,95 (m,1H); 1,05 (d,J=7Hz,3H); 1,25 (t,J=7Hz,5H); 3,17 (d,J=6,5Hz,1H); 3,97 (br.t,1H); 4,15 (q,J=7Hz,2H); 4,22 (m,1H); 4,43 (m,1H); 4,99 (br.s,1H); 5,33(br.s,1H); 5,41 (dd,J=15Hz, J=7,5Hz,1H); 5,52 (dd,J=15Hz, J=9Hz,1H); 6,01 (d,J=11Hz,1H); 6,38 (d,J=11Hz,1H).

**Beispiel 12**

**21.) (5*Z*,7*E*,22*E*)-(1*S*,3*R*,24*R*)-1,3,24-Trihydroxy-26,27-cyclo-9,10-secocholesta-5,7,10(19),22-tetraen-25-carbonsaurepropylester 24a**

Ausgehend von Carbonsäure 6 und Propanol wird analog der Sequenz der Synthese von 22a aus 7 die Titelverbindung 24a als Schaum erhalten.
$^1$H-NMR (300 MHz,$CDCl_3$): $\delta$ = 0,57 ppm (s,3H); 0,86 (m,1H); 0,95 (t,J=7Hz,4H); 1,05 (d,J=7Hz,3H); 1,23 (m,2H)); 3,19 (d,J=6,5Hz,1H); 3,96 (br.t,1H); 4,04 (t,J=7Hz,2H); 4,22 (m,1H); 4,42 (m,1H); 4,99 (br.s,1H); 5,32(br.s,1H); 5,41

(dd,J=15Hz, J=7,5Hz,1H); 5,52 (dd,J=15Hz, J=9Hz,1H); 6,01 (d,J=11Hz,1H); 6,38 (d,J=11Hz,1H).

**Beispiel 13**

**22.) (5*Z*,7*E*,22*E*)-(1*S*,3*R*,24*R*)-1,3,24-Trihydroxy-26,27-cyclo-9,10-secocholesta-5,7,10(19),22-tetraen-25-carbonsäurebutylester 25a**

Ausgehend von Carbonsäure 6 und Butanol wird analog der Sequenz der Synthese von 22a aus 7 die Titelverbindung 25a als Schaum erhalten.

$^1$H-NMR (300 MHz,$CDCl_3$): δ = 0,57 ppm (s,3H); 0,86 (m,1H); 0,93 (t,J=7Hz,4H); 1,05 (d,J=7Hz,3H); 1,25 (m,2H)); 3,19 (d,J=6,5Hz,1H); 3,97 (br.t,1H); 4,10 (t,J=7Hz,2H); 4,24 (m,1H); 4,43 (m,1H); 5,00 (br.s,1H); 5,32(br.s,1H); 5,41 (dd,J=15Hz, J=7,5Hz,1H); 5,52 (dd,J=15Hz, J=9Hz,1H); 6,01 (d,J=11Hz,1H); 6,38 (d,J=11Hz,1H).

**Beispiel 14**

**23.) (5*Z*,7*E*,22*E*)-(1*S*,3*R*,24*R*)-1,3,24-Trihydroxy-26,27-cyclo-9,10-secocholesta-5,7,10(19),22-tetraen-25-carbonsäure-2-methylpropylester 26a**

Ausgehend von Carbonsäure 6 und 2-Methyl-1-propanol wird analog der Sequenz der Synthese von 22a aus 7 die Titelverbindung 26a als Schaum erhalten.

$^1$H-NMR (300 MHz,$CDCl_3$): δ = 0,57 ppm (s,3H); 0,86 (m,1H); 0,92 (d,J=7Hz,7H); 1,05 (d,J=7Hz,3H); 1,25 (m, 2H)); 3,20 (d,J=6,5Hz,1H); 3,87 (d,J=7Hz,2H); 3,98 (br.t,1H); 4,25 (m,1H); 4,45 (m,1H); 4,99 (br.s,1H); 5,32(br.s,1H); 5,41 (dd,J=15Hz, J=7,5Hz,1H); 5,52 (dd,J=15Hz, J=9Hz,1H); 6,01 (d,J=11Hz,1H); 6,38 (d,J=11Hz,1H).

**Beispiel 15**

**24.) (5*Z*,7*E*,22*E*)-(1*S*,3*R*,24*R*)-1,3,24-Trihydroxy-26,27-cyclo-9,10-secocholesta-5,7,10(19),22-tetraen-25-carbonsäure-n-pentylester 27a**

Ausgehend von Carbonsäure 6 und Pentanol wird analog der Sequenz der Synthese von 22a aus 7 die Titelverbindung 27a als Schaum erhalten.

$^1$H-NMR (300 MHz,$CDCl_3$): δ = 0,57 ppm (s,3H); 0,90 (m,5H); 1,05 (d,J=7Hz,3H); 3,20 (br, s,1H); 3,98 (br,d,1H); 4,08 (t,J=7Hz,2H); 4,23 (m,1H); 4,42 (m,1H); 5,00 (br,s,1H); 5,32(br,s,1H); 5,41 (dd,J=15Hz,J=7,5Hz,1H); 5,52 (dd, J=15Hz,J=9Hz,1H); 6,00 (d,J=11Hz,1H); 6,38 (d,J=11Hz,1H).

**Beispiel 16**

**25.) (5*Z*,7*E*,22*E*)-(1*S*,3*R*,24*R*)-1,3,24-Trihydroxy-26,27-cyclo-9,10-secocholesta-5,7,10(19),22-tetraen-25-carbonsäure-n-hexylester 28a**

Ausgehend von Carbonsäure 6 und Hexanol wird analog der Sequenz der Synthese von 22a aus 7 die Titelverbindung 28a als Schaum erhalten.

$^1$H-NMR (300 MHz,$CDCl_3$): δ = 0,57 ppm (s,3H); 0,90 (m,5H); 1,05 (d,J=7Hz,3H); 3,2 (br,s,1H); 3,98 (br,d,1H); 4,08 (t,J=7Hz,2H); 4,23 (m,1H); 4,42 (m,1H); 5,00 (br,s,1H); 5,32 (br,s,1H); 5,41 (dd,J=15Hz,J=7,5Hz,1H); 5,52 (dd, J=15Hz,J=9Hz,1H); 6,00 (d,J=11Hz,1H); 6,38 (d,J=11Hz,1H).

**Ausgangsverbindungen für die 25-Carbonsäureamide (Normalreihe) und Beispiele**

**26.) (5*E*,7*E*,22*E*)-(1*S*,3*R*,24*R*)-1,3-Bis [[dimethyl(1,1-dimethylethyl)silyl]oxy]-24-hydroxy-9,10-secocholesta-5,7,10(19),22-tetraen-25-carbonsäuredimethylamid 29a**

3,0 g Aldehyd 3 und 1,32 g aktiviertes Zink [E.A. Hallinau et al. Tetrahedron Lett. 25 2301 (1984)] werden in 40 ml Tetrahydrofuran 5 Minuten in einer Stickstoff-Atmosphäre unter Rückfluß gehalten. Danach werden 3,92 g α-Bromisobuttersäure-dimethylamid [Lauceill et al., C.R. 248 3311 (1959)] hinzugegeben und die Reaktionsmischung noch 1 Stunde unter Rückfluß gekocht. Die abgekühlte Reaktionsmischung wird in Eis/$KHSO_4$-Lösung eingerührt, mit Ethylacetat extrahiert, über Natriumsulfat getrocknet und eingeengt.Durch Chromatographie des öligen Rückstandes an Kieselgel mit Ethylacetat/Hexan erhält man in der Eluationsreihenfolge 1,01 g (5*E*,7*E*,22*E*)-(1*S*,3*R*,24*R*)-1,3-Bis[[dimethyl (1,1-dimethyl-ethyl)silyl]oxy]-24-hydroxy-9,10-secocholesta-5,7,10(19),22-tetraen-25-carbonsäure-dimethylamid 29b

und 0,49 g der Titeiverbindung 29a als kristallisierte Masse.
In den nachfolgenden Reaktionen wird nun die weitere Umsetzung von 29a beschrieben.

**27.) (5*Z*,7*E*,22*E*)-(1*S*,3*R*,24*R*)-1,3-Bis[[dimethylethyl)silyl]oxy]-24-hydroxy-9,10-secocholesta-5,7,10(19),22-tetraen-25-carbonsäuredimethylamid 30a**

0,48 g 29a werden in 68 ml Toluol gelöst und nach Zugabe von 74 mg Anthracen und 1 Tropfen Triethylamin 30 Minuten unter Stickstoff mit einer Quecksilberhochdrucklampe Heraeus TQ 150) durch Pyrex-Glas bestrahlt. Die Reaktionsmischung wird eingeengt und der Rückstand (0,56 g) - ein Gemisch aus 30a und Anthracen - direkt der nachfolgenden Silyletherspaltung unterworfen.

**Beispiel 17**

**28.) (5*Z*,7*E*,22*E*)-(1*S*,3*R*,24*R*)-1,3,24-Trihydroxy-9,10-secocholesta-5,7,10(19),22-tetraen-25-carbonsäuredimethylamid 31a**

0,56 g des Rückstandes von 30a werden mit 8,5 g Dowex 50WX8 in 20,8 ml Methanol/Methylenchlorid (9:1) 30 Stunden bei Raumtemperatur gerührt. Die Suspension wird filtirert, das Filtrat eingeengt und der Rückstand an Kieselgel mit Ethylacetat/Hexan chromatographiert. Es werden 242 mg 31a als kristallisiertes Öl erhalten.

$^1$H-NMR(300 MHz, $CDCl_3$): δ = 0,57 ppm (S,3H); 1,06 (d, J = 7Hz, 3H); 1,28 (S,6H); 3,05 (S, 6H); 4,05 (br, t, 1H); 4,23 (m, 1H); 4,43 (br, d, 2H); 4,99 (br, s, 1H); 5,30 (br, S, 1H); 5,50 (br, t, 1H); 6,00 (d, J = 11Hz, 1H); 6,38 (d, J =11Hz, 1H).

**Beispiel 18**

**29.) (5*Z*,7*E*,22*E*)-(1*S*,3*R*,24*R*)-1,3,24-Trihydroxy-9,10-secocholesta-5,7,10(19),22-tetraen-25-carbonsäurediethylamid 32a**

Ausgehend von Aldehyd 3 und α-Bromisobuttersäure-diethylamid [Neemau, J. Chem. Soc. 2525 (1955)] wird analog der Sequenz der Beispiele 29a - 31a die Titelverbindung 32a als Schaum erhalten.

$^1$H-NMR(300 MHz, $CDCl_3$): δ = 0,57 ppm (S,3H); 1,05 (d, J = 7Hz, 3H); 1,15 (br, t, 6H); 1,22 (S, 3H); 1,23 (S, 3H); 3,38 (m,1H); 4,00 (br,t,1H); 4,20 (m,1H); 4,40 (m,1H); 4,58(br,d,1H); 4,98(br,S,1H); 5,30(br,S,1H); 5,48 (m,2H); 6,00 (d, J = 11Hz,1H); 6,38 (d,J = 11Hz,1H)

**30.) (5*E*,7*E*,22*E*)-(1*S*,3*R*)-1,3-Bis[[dimethyl(1,1-dimethylethyl)silyl]oxy]-24-oxo-26,27-cyclo-9,10-secocholesta-5,7,10(19),22-tetraen-25-carbonsäurediethylamid 33**

8,31 g Rohprodukt der Carbonsäure 6 und 2,51 g N,N'-Dicyclohexylcarbodiimid in 25,1 ml Methylenchlorid werden bei Eiskühlung mit 1,40 g N-Hydroxysuccinimid versetzt, und nach 30 Minuten werden 976 mg Diethylamin hinzugefügt. Die Reaktionsmischung wird noch 40 Minuten bei Eiskühlung gerührt und über Nacht bei Raumtemperatur stehengelassen. Danach wird mit gesättigter Natriumchloridlösung/Ethylacetat extrahiert, die organische Phase mit Natriumsulfat getrocknet und eingeengt. Chromatographie des Rückstandes an Kieselgel mit Ethylacetat/Hexan ergeben 2,94 g 33 als Öl.

**31.) (5*E*,7*E*,22*E*)-(1*S*,3*R*,24*R*)-1,3-Bis[[dimethyl(1,1-dimethylethyl)silyl]oxy]-24-hydroxy-26,27-cyclo-9,10-secocholesta-5,7,10(19),22-tetraen-25-carbonsäurediethylamid 34a**

3,60 g 33 in 8,2 ml Tetrahydrofuran und 18,9 ml Methanol werden mit 18,9 ml einer 0,4 molaren methanolischen Cer(III)-chlorid Heptahydrat-Lösung versetzt. Unter Stickstoff werden bei Eiskühlung 520 mg Natriumborhydrid portionsweise hinzugegeben. Die Reaktionsmischung wird noch 90 Minuten bei Eiskühlung gerührt und dann mit Eis/Wasser versetzt. Anschließend wird mit Ethylacetat extrahiert, die organische Phase über Natriumsulfat getrocknet und eingeengt. Durch Chromatographie des Rückstandes an Kieselgel mit Ethylacetat/Hexan erhält man in der Elutionsreihenfolge 760 mg (5*E*,7*E*,22*E*)-(1*S*,3*R*,24*S*)-1,3-Bis[[dimethyl(1,1-dimethylethyl)silyl]oxy]-24-hydroxy-26,27-cydo-9,10-secocholesta-5,7,10(19),22-tetraen-25-carbonsäurediethylamid 34b und 300 mg der Titelverbindung 34a als kristallisiertes Öl.
In den nachfolgenden Reaktionen wird nur die weitere Umsetzung von 34a beschrieben.

**32.) (5*Z*,7*E*,22*E*)-(1*S*,3*R*,24*R*)-1,3-Bis[[dimethyl(1,1-dimethylethyl)silyl]oxy]-24-hydroxy-26,27-cyclo-9,10-secocholesta-5,7,10(19),22-tetraen-25-carbonsäurediethylamid 35a**

290 mg 34a und 42 mg Anthracen in 50 ml Toluol werden in Gegenwart von 1 Tropfen Triethylamin 5 Minuten unter Stickstoff mit einer Quecksilberhochdrucklampe bestrahlt. Die Reaktionsmischung wird eingeengt und der Rückstand (330 mg) - ein Gemisch aus 35a und Anthracen - der nachfolgenden Silyletherspaltung unterworfen.

**Beispiel 19**

**33.) (5*Z*,7*E*,22*E*)-(1*S*,3*R*,24*R*)-1,3,24-Trihydroxy-26,27-cyclo-9,10-secocholesta-5,7,10(19),22-tetraen-25-carbonsäurediethylamid 36a**

330 mg des Rückstandes von 35a in 11,4 ml Tetrahydrofuran werden mit 605 mg Tetrabutylammoniumfluorid Trihydrat über Nacht bei Raumtemperatur stehengelassen. Die Reaktionsmischung wird auf Eis/Natriumhydrogencarbonatlösung gegossen und Natriumchlorid-lösung hinzugesetzt. Danach wird mit Ethylacetat extrahiert, über Natriumsulfat getrocknet und eingeengt. Chromatographie des Rückstandes an Kieselgel mit Ethylacetat/Hexan ergeben 100 mg der Titelverbindung 36a als Schaum.

$^1$H-NMR(300 MHz, $CDCl_3$): $\delta$ = 0,55 ppm (S,3H); 0,85 (m,4H); 1,02 (d, J=7Hz,3H); 1,14 (br,t,6H); 2,76(br,d,1H); 3,4 (m,4H); 3,95 (br,t,1H); 4,23(m,1H); 4,43(m,1H); 5,00 (br,s,1H); 5,30 (dd, J=1sHz, J=7,5Hz,1H); 5,32(br,s,1H); 5,56 (dd,J=15Hz, J=9Hz,1H); 6,02 (d, J=11Hz,1H); 6,48 (d, J=11Hz,1H).

**34.) (5*Z*,7*E*)-(1*S*,3*R*,20*S*)-1,3-Bis[[dimethyl(1,1-dimethylethyl)silyl]oxy]-formyl-9,10-secopregna-5,7,10(19)-trien 37**

1,0 g Aldehyd 1 und 192 mg Anthracen werden in Gegenwart von 3 Tropfen Triethylamin 15 Minuten unter Stickstoff mit einer Quecksilberhochdrucklampe bestrahlt. Dieser Vorgang wird noch zweimal wiederholt. Die vereinigten Reaktionsmischungen werden eingeengt und der Rückstand von 3,6 g direkt der nachfolgenden Reaktion unterworfen.

**35.) (5*Z*,7*E*,22*E*)-(1*S*,3*R*)-1,3-Bis[[dimethyl(1,1-dimethylethyl)silyl]oxy]-24-oxo-26,27-cyclo-9,10-secocholesta-5,7,10(19),22-tetraen-25-carbonsäure 38**

19,4 ml Butyllithium (1,6 molar in Hexan) werden bei Eiskühlung unter Stickstoff zu 4,79 ml Diisopropylamin in 304 ml Tetrahydrofuran getropft und noch 15 Minuten gerührt. Danach werden bei -78°C 4,42 g 1-Acetylcydopropancarbonsäuremethylester [D.F. Taber et al., J. Org. Chem. 57 436 (1992)] zugetropft und 1 Stunde gerührt. Anschließend wird bei -78°C der Aldehyd 37 in 9,2 ml Tetrahydrofuran zugetropft und 1,5 Stunden bei -78°C gerührt. Man läßt die Reaktionsmischung in 100 Minuten auf -10°C kommen und gibt dann gesättigte Ammoniumchloridlösung hinzu. Die Reaktionsmischung wird mit Natriumchloridlösung verdünnt und mit Ethylacetat in Gegenwart von 5 %iger Oxalsäure extrahiert. Die organische Phase wird mit Natriumchloridlösung gewaschen, über Natriumsulfat getrocknet und eingeengt. Es werden 7,7 g Rohprodukt der Titelverbindung als Öl erhalten.

**36.) (5*Z*,7*E*,22*E*)-(1*S*,3*R*)-1,3-Bis[[dimethyl(1,1-dimethylethyl)silyl]oxy]-24-oxo-26,27-cyclo-9,10-secocholesta-5,7,10(19),22-tetraen-25-carbonsäure-n-butylamid 39**

7,70 g 38 und 1,30 g N-Hydroxysuccinimid werden in 15 ml Methylenchlorid gelöst und 2,33 g N,N'-Dicyclohexylcarbodiimid bei 0°C hinzugegeben. Nach 40 Minuten werden 1,12 ml n-Butylamin hinzugefügt und 5 Stunden bei Raumtemperatur gerührt. Nach Stehen über Nacht wird die Reaktionsmischung mit Eis/Natriumchloridlösung verdünnt. Danach wird mit Ethylacetat extrahiert, die organische Phase über Natriumsulfat getrocknet und eingeengt. Es werden 1,84 g Rohprodukt der Titelverbindung 39 als Öls erhalten.

**Beispiel 20**

**37.) (5*Z*,7*E*,22*E*)-(1*S*,3*R*)-1,3-Dihydroxy-24-oxo-26,27-cyclo-9,10-secocholesta-5,7,10(19),22-tetraen-25-carbonsäure-n-butylamid 40**

970 mg 39 werden in 38 ml Methylenchlorid/Methanol (1:9) gelöst und mit 16,28 g Dowex 50WX8 26 Stunden bei Raumtemperatur gerührt. Nach Filtration und Einengung des Filtrats wird der Rückstand an Kieselgel mit Ethylacetat/Hexan chromatographiert. Es werden 340 mg 40 als Schaum erhalten.

$^1$H-NMR(300 MHz, $CDCl_3$): $\delta$ = 0,58 ppm (s,3H); 0,93 (t,J=7,5Hz,3H); 1,08 (d, J=7Hz,3H); 1,50(m,4H); 3,30

(J=5Hz,2H); 4,24 (m,1H); 4,45 (m,1H); 4,98 (br,s,1H); 5,22 (br,s,1H); 5,85 (d,J=15Hz,1H), 6,00 (d,J=11Hz,1H); 6,38 (d,J=11Hz,1H); 6,91 (dd,J=15Hz, J=9Hz,1H); 8,95 (br,t,1H).

**Ausgangsmaterialien in der 20-Methylreihe (tert.-Butyldiphenylsilyl-Schutzgruppen)**

**38) (5*E*,7*E*)-(1*S*,3*R*)-1,3-Bis[[1,1-dimethylethyl(diphenyl)silyl]oxyl-20-formyl-20-methyl 9,10-secopregna-5,7,10 (19)-trien 42**

Man legt 140 mg (4,5 mmol) Natriumhydrid (80%) in 20 ml Tetrahydrofuran vor und entfernt den Sauerstoff durch mehrmaliges Entgasen im Vakuum und Spülen mit Argon. Nun werden bei 0°C 2,5 g (3,0 mmol) (5*E*,7*E*)-(1*S*,3*R*,20*S*)-1,3-Bis[[1,1-dimethylethyl(diphenyl)silyl]-oxy]-20-formyl-9,10-secopregna-5,7,10(19)-trien **41**, (M. Calverley Tetrahedron **43**, 4609 (1987), WO 87/00834, tert.-Butyldimethylsilyl-Schutzgruppen) in 20 ml Tetrahydrofuran und anschließend 1,87 g (13,5 mmol) Methyliodid zugetropft. Man rührt über Nacht bei Raumtemperatur nach, gießt dann das Reaktionsgemisch auf Natriumchlorid-Lösung, extrahiert mit Essigester, wäscht die organische Phase mit Natriumchlorid-Lösung, trocknet über Natriumsulfat, entfernt das Solvens und reinigt das Rohprodukt durch Chromatographie an Kieselgel mit Hexan/Essigester als Laufmittel, wobei man 2,0 g der Titelverbindung als farblosen Schaum erhält.

$^1$H-NMR ($CDCl_3$): δ= 0,52 ppm (s, 3H, H-18); 0,96 u. 0,98 (2x s, je 9H, Si-t-butyl); 1,13 u. 1,15 (2x s, je 3H, H-21 u. C-20-Me); 4,29 (m, 1H, H-3); 4,64 (m, 1H, H-1); 4,73 u. 4,90 (2x s, je 1H, H-19); 5,62 u. 6,38 (2x d, J=11 Hz, je 1H, H-6 u. H-7); 7,22-7,68 (m, 20H, Si-phenyl); 9,68 (s, 1H, H-22)

(Es wird durchgängig die Steroid-Nummerierung zur Zuordnung verwendet)

**39) (5*E*,7*E*,22*E*)-(1*S*,3*R*)-Bis[[1,1-dimethylethyl(diphenyl)silyl]oxy]-20,N-dimethyl-N-metboxy-9,10-secochola-5,7,10(19)-22-tetraen-24-amid 43**

Man rührt 2,0 g (2,44 mmol) **42** und 5,2 g (14,6 mmol) N-Methoxy-N-methyl-2-(triphenylphosphoranyliden)acetamid [D.A. Evans et al. J. Am. Chem. Soc. **112,** 7001(1990)] in 100 ml Toluol unter Argon für 48 Std. bei 80°C. Nach dem Abkühlen engt man die Lösung ein und chromatographiert den Rückstand an Kieselgel mit Hexan/Essigester, wobei neben 905 mg des Ausgangsmaterials 880 mg der Titelverbindung als farbloser Schaum verbleiben.

$^1$H-NMR ($CDCl_3$): δ= 0,53 ppm (s, 3H, H-18); 0,99 (s, 18H, Si-t-butyl); 1,16 u. 1,18 (2x s, je 3H, H-21 u. C-20-Me); 3,27 (s, 3H, N-Me); 3,70 (s, 3H, N-OMe); 4,29 (m, 1H, H-3); 4,60 (m, 1H, H-1); 4,68 u. 4,88 (2x s, je 1H, H-19); 5,60 u. 6,38 (d, J=11 Hz, je 1H, H-6 u. H-7); 6,30 (d, J=16 Hz, 1H, H-23); 7,19 (d, J=16 Hz, 1H, H-22); 7,23-7,70 (m, 20H, Si-phenyl)

**40) (5*E*,7*E*,22*E*)-(1*S*,3*R*)-1,3-Bis[[1,1-dimethylethyl(diphenyl)silyl]oxy]-20-methyl9,10-secochola-5,7,10(19), 22-tetraen-24-al 44**

Man löst 880 mg (0,96 mmol) **43** unter Argon in 15 ml Tetrahydrofuran, kühlt auf -78°C und tropft 4,2 ml (5 mmol) DIBAH-Lösung (1,2 M in Toluol) zu. Nach 2 Std. bei -78°C gibt man 0,3 ml Methanol zu und rührt 1 Std. bei Raumtemperatur nach. Anschließend filtriert man den Niederschlag ab, engt das Filtrat ein und reinigt den Rückstand chromatographisch, wobei man 690 mg der Titelverbindung als farblosen Schaum erhält.

$^1$H-NMR ($CDCl_3$): δ= 0,51 ppm (s, 3H, H-18); 0,98 (s, 18H, Si-t-butyl); 1,16 u. 1,20 (2x s, je 3H, H-21 u. C-20-Me); 4,30 (m, 1H, H-3); 4,61 (m, 1H, H-1); 4,70 u. 4,88 (2x s, je 1H, H-19); 5,61 u. 6,37 (2x d, J=11 Hz, je 1H, H-6 u. H-7); 6,07 (dd, J=16, 8 Hz, 1H, H-23); 7,04 (d, J=16 Hz, 1H, H-22); 7,22-7,68 (m, 20H, Si-phenyl); 9,57 (d, J=8 Hz, 1H, H-24)

**Beispiel 21**

**41) (5*E*,7*E*,22*E*)-(1*S*,3*R*)-1,3-Bis[[1,1-dimethylethyl(diphenyl)silyl]oxy]-24-hydroxy-20-methyl-9,10-secocholesta-5,7,10(19),22-tetraen-25-carbonsäureethylester 45**

Aus 180 mg (1,8 mmol) Düsopropylamin und 1,1 ml (1,7 mmol) n-Butyllithium (1,6 M in Hexan) in 30 ml Tetrahydrofuran wird unter Argon LDA bereitet und auf -78°C gekühlt. Man gibt nun 200 mg (1,7 mmol) Isobuttersäureethylester zu, rührt 30 Min. bei -78°C und addiert dann 590 mg (0,68 mmol) **44** in 3 ml Tetrahydrofuran. Es wird 2 Std. bei -78°C nachgerührt und anschließend auf Natriumchlorid-Lösung gegossen. Nach Extraktion mit Essigester, Waschen der organischen Phase mit Natriumchlorid-Lösung, Trocknen über Natriumsulfat und Entfernen des Lösungsmittels wird der Rückstand chromatographisch gereinigt, wobei 480 mg der Titelverbindung als farbloser Schaum anfallen (1:1-Diastereomere bzgl. C-24).

$^1$H-NMR ($CDCl_3$): δ= 0,53 ppm (s, 3H, H-18); 0,95 (s, 18H, Si-t-butyl); 1,05/1,07, 1,10/1,12, 1,17, 1,18 (4x s, je 3H, H-21, C-20-Me, H-26 u. H-27); 1,28 (t, J=7 Hz,3H, COOEt); 2,57 (d, J=5 Hz, 1H, OH); 4,15 (q, J=7 Hz, 2H, COOEt);

4,18 (m, 1H, H-24); 4,28 (m, 1H, H-3); 4,63 (m, 1H, H-1); 4,72 u. 4,90 (2x s, je 1H, H-19); 5,32 (dd, J=15,5, 7,5 Hz, H-23); 5,89/5,90 (d, J=15,5 Hz, 1H, H-22); 5,60 u. 6,38 (2x d, J=11 Hz, je 1H, H-6 u. H-7); 7,20-7,68 (m, 20H, Si-phenyl)

**42) (5*Z*,7*E*,22*E*)-(1*S*,3*R*,24*R*)-20-Methyl-1,3,24-trihydroxy-9,10-secocholesta-5,7,10(19),22-tetraen-25-carbonsäureethylester <u>46a</u> und (5*Z*,7*E*,22*E*)-(1*S*,3*R*,24*S*)-20-Methyl-1,3,24-trihydroxy-9,10-secocholesta-5,7,10(19),22-tetraen-25-carbonsäureethylester <u>46b</u>**

Man rührt 480 mg (0,5 mmol) **<u>45</u>** mit 65 mg (0,77 mmol) Dihydropyran und einer Spatelspitze Pyridinium-p-toluolsulfonat in 5 ml Methylenchlorid unter Argon für 24 Std. bei Raumtemperatur. Die organische Phase wird mit Natriumchlorid-Lösung gewaschen, über Natriumsulfat getrocknet, das Solvens entfernt und der Rückstand chromatographisch an Kieselgel mit Hexan/Essigester gereinigt, wobei 480 mg des Diastereomerengemisches als farbloser Schaum verbleiben. Dieses Produkt wird in 80 ml Toluol gelöst und in Gegenwart von 100 mg (0,55 mmol) Anthracen und 0,1 ml Triethylamin in einem Pyrex-Tauchreaktor mit einer Quecksilberhochdrucklampe (Philips HPK 125) unter Stickstoff für 15 Min. bestrahlt. Das Lösungsmittel wird entfernt und das Rohprodukt wird mit 708 mg (2,25 mmol) Tetrabutylammoniumfluorid in 10 ml Tetrahydrofuran unter Argon bei 60°C für 1 Std. gerührt. Man gießt dann in Natriumchlorid-Lösung, extrahiert mit Essigester, wäscht mit Natriumchlorid-Lösung, trocknet über Natriumsulfat und entfernt das Solvens. Der Rückstand wird in 5 ml Methanol/Methylenchlorid (9:1) gelöst und unter Argon mit 500 mg Amberlite (aktiviert) für 24 Std, gerührt. Man filtriert nun, wäscht das Filtrat mit Natriumhydrogencarbonat-Lösung und Natriumchlorid-Lösung, trocknet über Natriumsulfat, entfernt das Solvens und reinigt den Rückstand chromatographisch. Die Trennung der Diastereomeren erfolgt nun via HPLC (Reversed Phase Bedingungen, Acetonitril/Wasser als Laufmittel, wobei man nacheinander 5 mg **<u>46b</u>** und 6 mg **<u>46a</u>** als farblose Schäume erhält.

$^1$H-NMR ($CD_2Cl_2$): **<u>46a:</u>** δ= 0,58 ppm (s, 3H, H-18); 1,03, 1,07, 1,18 (3x s, 3H, 3H, 6H, H-21, C-20-Me, H-26 u. H-27); 1,27 (t, J=7 Hz, 3H, COOEt); 4,10 (m, 1H, H-24); 4,12 (q, J=7 Hz, 2H, COOEt); 4,18 (m, 1H, H-3); 4,38 (m, 1H, H-1); 4,97 u. 5,30 (2x s, je 1H, H-19); 5,31 (dd, J=15,5, 7 Hz, 1H, H-23); 5,86 (d, J=15,5 Hz, 1H, H-22); 5,99 u. 6,35 (2x d, J=11 Hz, je 1H, H-6 u. H-7)

**<u>46b:</u>** δ= 0,58 ppm (s, 3H, H-18); 1,01, 1,09, 1,12 (3x s, 3H, 3H, 6H, H-21, C-20-Me, H-26 u. H-27); 1,27 (t, J=7 Hz, 3H, COOEt); 4,10 (m, 1H, H-24); 4,11 (q, J=7 Hz, 2H, COOEt); 4,18 (m, 1H, H-3); 4,38 (m, 1H, H-1); 4,96 u. 5,30 (2x s, je 1H, H-19); 5,30 (dd, J=15,5,7 Hz, 1H, H-23); 5,88 (d, J=15,5 Hz, 1H, H-22); 5,99 u. 6,35 (2x d, J=11 Hz, je 1H, H-6 u. H-7)

**Beispiel 22**

**43) (5*E*,7*E*,22*E*)-(1*S*,3*R*)-1,3-Bis[[1,1-dimethylethyl(diphenyl)silyl]oxy]-24-hydroxy-20-methyl-9,10-secocholesta-5,7,10(19),22-tetraen-25-carbonsäurepropylester <u>47</u>**

Analog **41)** werden 690 mg (0,8 mmol) **<u>44</u>** mit 2 mmol LDA und 256 mg (2 mmol) Isobuttersäurepropylester umgesetzt, wobei 550 mg der Titelverbindung als farbloser Schaum anfallen (1:1 Diastereomere bzgl. C-24)

$^1$H-NMR ($CDCl_3$): δ= 0,53 ppm (s, 3H, H-18); 0,95 (t, J=7 Hz, 3H, COOPr); 0,97 u. 0,98 (2x s, je 9H, Si-t-butyl); 1,07/1,08, 1,10/1,12, 1,18 u. 1,19 (4x s, je 3H, H-21, C-20-Me, H-26 u. H-27); 1,68 (hex, J=7 Hz, 2H, COOPr); 2,53 (d, J=7 Hz, 1H, OH); 4,08 (q, J=7 Hz, 2H, COOPr); 4,15 (m, 1H, H-24); 4,28 (m, 1H, H-3); 4,62 (m, 1H, H-1); 4,72 u. 4,89 (2x s, je 1H, H-19); 5,33 (dd, J=15,5, 7 Hz, H-23); 5,90/5,91 (d, J=15,5 Hz, 1H, H-22); 5,60 u. 6,39 (2x d, J=11 Hz, je 1H, H-6 u. H-7); 7,22-7,65 (m, 20H, Si-phenyl)

**44) (5*Z*,7*E*,22*E*)-(1*S*,3*R*,24*R*)-20-Methyl-1,3,24-trihydroxy-9,10-secocholesta-5,7,10(19),22-tetraen-25-carbonsäurepropylester <u>48a</u> und (5*Z*,7*E*,22*E*)-(1*S*,3*R*,24*S*)-20-Methyl-1,3,24-trihydroxy-9,10-secocholesta-5,7,10(19),22-tetraen-25-carbonsäurepropylester <u>48b</u>**

Man setzt 540 mg (0,55 mmol) **<u>47</u>** analog den unter **42)** beschriebenen Bedingungen um und erhält 34 mg **<u>48b</u>** sowie 24 mg **<u>48a</u>** als farblose Schäume.

$^1$H-NMR ($CD_2Cl_2$):**<u>48a</u>**: δ= 0,58 ppm (s, 3H, H-18); 0,95 (t, J=7 Hz, 3H, COOPr); 1,04, 1,08, 1,22 (3x s, 3H, 3H, 6H, H-21, C-20-Me, H-26 u. H-27); 1,67 (hex, J=7 Hz, 3H, COOPr); 4,02 (t, J=7 Hz, 2H, COOPr); 4,10 (m, 1H, H-24); 4,18 (m, 1H, H-3); 4,38 (m, 1H, H-1); 4,96 u. 5,29 (2x s, je 1H, H-19); 5,31 (dd, J=15,5, 7 Hz, 1H, H-23); 5,86 (d, J=15,5 Hz, 1H, H-22); 6,00 u. 6,36 (2x d, J=11 Hz, je 1H, H-6 u. H-7)

**<u>48b:</u>** δ= 0,58 ppm (s, 3H, H-18); 0,95 (t, J=7 Hz, 3H, COOPr); 1,00, 1,09, 1,22 (3x s, 3H, 3H, 6H, H-21, C-20-Me, H-26 u. H-27); 1,68 (hex, J=7 Hz, 3H, COOPr); 4,02 (t, J=7 Hz, 2H, COOPr); 4,10 (m, 1H, H-24); 4,18 (m, 1H, H-3); 4,38 (m, 1H, H-1); 4,96 u. 5,29 (2x s, je 1H, H-19); 5,32 (dd, J=15,5, 7 Hz, 1H, H-23); 5,89 (d, J=15,5 Hz, 1H, H-22); 6,00

u. 6,36 (2x d, J=11 Hz, je 1H, H-6 u. H-7)

**Beispiel 23**

**45) (5*E*,7*E*,22*E*)-(1*S*,3*R*)-1,3-Bis[[1,1-dimethylethyl(diphenyl)silyl]oxy]-24-hydroxy-20-methyl-9,10-secocholesta-5,7,10(19),22-tetraen-25-carbonsäure-1-methylethylester 49**

Analog **41)** werden 440 mg (0,51 mmol) **44** mit 1,7 mmol LDA und 221 mg (1,7 mmol) Isobuttersäureisopropylester umgesetzt, wobei 390 mg der Titelverbindung als farbloser Schaum anfallen (1:1 Diastereomere bzgl. C-24).

$^1$H-NMR ($CDCl_3$): δ= 0,53 ppm (s, 3H, H-18); 0,95 u. 0,97 (2x s, je 9H, Si-t-butyl); 1,05/1,07, 1,09/1,11, 1,17 u. 1,18 (4x s, je 3H, H-21, C-20-Me, H-26 u. H-27); 1,28 (d, J=7 Hz, 6H, COOiPr); 4,12 (m, 1H, H-24); 4,28 (m, 1H, H-3); 4,64 (m, 1H, H-1); 4,72 u. 4,90 (2x s, je 1H, H-19); 5,24 (hept, J=7 Hz, 1H, COOiPr); 5,33 (dd, J=15,5, 7 Hz, H-23); 5,90/5,91 (d, J=15,5 Hz, 1H, H-22); 5,61 u. 6,38 (2x d, J=11 Hz, je 1H, H-6 u. H-7); 7,22-7,65 (m, 20H, Si-phenyl)

**46) (5*Z*,7*E*,22*E*)-(1*S*,3*R*,24*R*)-20-Methyl-1,3,24-trihydroxy-9,10-secocholesta-5,7,10(19),22-tetraen-25-carbonsäure-1-methylethylester 50a und (5*Z*,7*E*,22*E*)-(1*S*,3*R*,24*S*)-20-Methyl-1,3,24-trihydroxy-9,10-secocholesta-5,7,10(19),22-tetraen-25-carbonsäure-1-methylethylester 50b**

Man setzt 385 mg (0,39 mmol) **49** analog den unter **42)** beschriebenen Bedingungen um und erhält 12 mg **50b** sowie 11 mg **50a** als farblose Schäume.

$^1$H-NMR ($CD_2Cl_2$): **50a:** δ= 0,58 ppm (s, 3H, H-18); 1,03, 1,06, 1,18 (3x s, 3H, 3H, 6H, H-21, C-20-Me, H-26 u. H-27); 1,28 (d, J=7 Hz, 6H, COOiPr); 4,09 (m, 1H, H-24); 4,18 (m, 1H, H-3); 4,38 (m, 1H, H-1); 4,96 u. 5,30 (2x s, je 1H, H-19); 4,98 (hept, J=7 Hz, 1H, COOiPr); 5,31 (dd, J=15,5, 7 Hz, H-23); 5,85 (d, J=15,5 Hz, 1H, H-22); 5,99 u. 6,36 (2x d, J=11 Hz, je 1H, H-6 u. H-7)

$^1$H-NMR ($CD_2Cl_2$): **50b:** δ= 0,59 ppm (s, 3H, H-18); 1,02, 1,11, 1,18 (3x s, 3H, 3H, 6H, H-21, C-20-Me, H-26 u. H-27); 1,27 (d, J=7 Hz, 6H, COOiPr); 4,09 (m, 1H, H-24); 4,18 (m, 1H, H-3); 4,38 (m, 1H, H-1); 4,96 u. 5,30 (2x s, je 1H, H-19); 4,99 (hept, J=7 Hz, 1H, COOiPr); 5,31 (dd, J=15,5, 7 Hz, H-23); 5,88 (d, J=15,5 Hz, 1H, H-22); 6,00 u. 6,36 (2x d, J=11 Hz, je 1H, H-6 u. H-7)

**Ausgangsmaterialien in der 20-Methylreihe (tert.-Butyldimethylsilyl-Schutzgruppen)**

**47) (5*E*,7*E*)-(1*S*,3*R*)-1,3-Bis[[dimethyl(1,1-dimethylethyl)silyl]oxy]-20-formyl-20-methyl-9,10-secopregna-5,7,10(19)-trien 51**

Man setzt 4,0 g (7 mmol) (5*E*,7*Z*)-(1*S*,3*R*,20*S*)-1,3-Bis[[dimethyl(1,1-dimethylethyl)silyl]-oxy]-20-formyl-9,10-secopregna-5,7,10(19)-trien **1** (M. Calverley Tetrahedron **43,** 4609 (1987), WO 87/00834) mit 314 mg (10,5 mmol) Natriumhydrid und 3,87 g (28 mmol) Methyliodid analog **38)** um und erhält 3,1 g der Titelverbindung als farblosen Schaum.

$^1$H-NMR ($CDCl_3$): δ= 0,00 ppm (s, 12H, SiMe); 0,50 (s, 3H, H-18); 0,80 u. 0,85 (2x s, je 9H, Si-t-butyl); 1,06 u. 1,08 (2x s, je 3H, H-21 u. C-20-Me); 4,18 (m, 1H, H-3); 4,48 (m, 1H, H-1); 4,89 u. 4,92 (2x s, je 1H, H-19); 5,76 u. 6,38 (2x d, J=11 Hz, H-6 u. H-7); 9,59 (s, 1H, H-22)

**48) (5*E*,7*E*,22*E*)-(1*S*,3*R*)-Bis[[dimethyl(1,1-dimethylethyl)silyl]oxy]-20,N-dimethyl-N-methoxy-9,10-secochola-5,7,10(19),22-tetraen-24-amid 52**

Man setzt 3,1 g (5,3 mmol) **51** mit 15,4 g (42,4 mmol) N-Methoxy-N-methyl-2-(triphenylphosphoranyliden)acetamid analog **39)** um, wobei man 1,3 g der Titelverbindung als farblosen Schaum neben 1,1 g des Ausgangsmaterials erhält.

$^1$H-NMR ($CDCl_3$): δ= 0,01 ppm (s, 12H, SiMe); 0,50 (s, 3H, H-18); 0,81 u. 0,84 (2x s, je 9H, Si-t-butyl); 1,07 u. 1,10 (2x s, je 3H, H-21 u. C-20-Me); 3,19 (s, 3H, N-Me); 3,64 (s, 3H, N-OMe); 4,17 (m, 1H, H-3); 4,48 (m, 1H, H-1); 4,88 u. 4,92 (2x s, je 1H, H-19); 5,73 u. 6,39 (2x d, J=11 Hz, je 1H, H-6 u. H-7); 6,23 (d, J=16 Hz, 1H, H-23); 7,11 (d, J=16 Hz, 1H, H-22)

**49) (5*E*,7*E*,22*E*)-(1*S*,3*R*)-1,3-Bis[[dimethyl(1,1-dimethylethyl)silyl]oxy]-20-methyl-9,10-secochola-5,7,10(19),22-tetraen-24-al 53**

Man setzt 1,3 g (1,9 mmol) **52** mit 9,5 mmol DIBAH-Lösung in Toluol analog **40)** um und erhält 840 mg der Titelverbindung als farblosen Schaum.

$^1$H-NMR ($CDCl_3$): δ= 0,02 ppm (s, 12H, SiMe); 0,50 (s, 3H, H-18); 0,83 u. 0,87 (2x s, je 9H, Si-t-butyl); 1,09 u.

1,14 (2x s, je 3H, H-21 u. C-20-Me); 4,18 (m, 1H, H-3); 4,48 (m, 1H, H-1); 4,88 u. 4,92 (2x s, je 1H, H-19); 5,74 u. 6,38 (2x d, J=11 Hz, je 1H, H-6 u. H-7); 5,99 (dd, J=15, 8 Hz, 1H, H-23); 6,98 (d, J=15 Hz, 1H, H-22); 9,48 (d, J=8 Hz, H-24)

**Beispiel 24**

**50) (5*E*,7*E*,22*E*)-(1*S*,3*R*)-1,3-Bis[[dimethyl(1,1-dimethylethyl)silyl]oxy]-24-hydroxy-20-methyl-9,10-secocholesta-5,7,10(19),22-tetraen-25-carbonsäureethylester 54**

Analog 41) werden 210 mg (0,34 mmol) **53** mit 1,4 mmol LDA und 165 mg (1,4 mmol) Isobuttersäureethylestcr umgesetzt, wobei 180 mg der Titelverbindung als farbloser Schaum anfallen (1:1 Diastereomere bzgl. C-24).

$^1$H-NMR ($CDCl_3$): δ= 0,02 ppm (s, 12H, SiMe); 0,51 (s, 3H, H-18); 0,80 u. 0,83 (2x s, je 9H, Si-t-butyl); 0,98/1,00, 1,02/1,06, 1,09, 1,10 (4x s, je 3H, H-21, C-20-Me, H-26 u. H-27); 1,23 (t, J=7 Hz, 3H, COOEt); 4,07 (m, 1H, H-24); 4,10(q, J=7 Hz, 2H, COOEt); 4,15 (m, 1H, H-3); 4,48 (m, 1H, H-1); 4,88 u. 4,92 (2x s, je 1H, H-19); 5,27 (dd, J=15, 7 Hz, 1H, H-23); 5,82/5,83 (2x d, J=15 Hz, 1H, H-22); 5,73 u. 6,39 (2x d, J=11 Hz, je 1H, H-6 u. H-7)

**51) (5*Z*,7*E*,22*E*)-(1*S*,3*R*)-1,3-Dihydroxy-20-methyl-24-oxo-9,10-secocholesta-5,7,10(19),22-tetraen-25-carbonsäureethylester 55**

Man löst 210 mg (0,29 mmol) **54** in 80 ml Toluol, setzt 80 mg (0,44 mmol) Anthracen und 3 Tropfen Triethylamin zu und bestrahlt in einem Pyrex-Tauchreaktor mit einer Quecksilberhochdrucklampe (Philips HPK 125) unter Stickstoff für 15 Min. Das Lösungsmittel wird entfernt und der Rückstand chromatographisch an Kieselgel mit Hexan/Essigester gereinigt, wobei 200 mg des isomerisierten Produktes als farbloser Schaum anfallen. Dieses Material wird in 1 ml Methylenchlorid gelöst und bei -50°C zu einer Mischung aus 0,1 ml (1 mmol) Oxalylchlorid und 0,15 ml (2 mmol) DMSO in 10 ml Methylenchlorid getropft. Man rührt 15 Min. nach und addiert dann 0,65 ml (5 mmol) Triethylamin. Nach Erwärmung auf Raumtemperatur wird Natriumchlorid-Lösung zugegeben, mit Essigester extrahiert, mit Natriumchlorid-Lösung gewaschen, über Natriumsulfat getrocknet und das Solvens entfernt. Das Rohprodukt wird nun an Kieselgel chromatographiert, wobei 103 mg des Oxidationsproduktes verbleiben, die man in 5 ml Methylenchlorid/Methanol (1: 9) mit 500 mg Dowex (aktiviert) für 3 Tage bei Raumtemperatur rührt. Anschließend wird der Ionentauscher abfiltriert, das Filtrat eingeengt und chromatographisch aufgereinigt, wobei 38 mg der Titelverbindung als farbloser Schaum anfallen.

$^1$H-NMR ($CD_2Cl_2$): δ= 0,50 ppm (s, 3H, H-18); 1,04 u. 1,10 (2x s, je 3H, H-21 u. C-20-Me); 1,20 (t, J=7 Hz, 3H, COOEt); 1,33 (s, 6H, H-26 u. H-27); 4,12 (q, J=7 Hz, 2H, COOEt); 4,15 (m, 1H, H-3); 4,38 (m, 1H, H-1); 4,93 u. 5,29 (2x s, je 1H, H-19); 5,98 u. 6,32 (2x d, J=11 Hz, je 1H, H-6 u. H-7); 6,10 (d, J=15 Hz, 1H, H-23); 7,13 (d, J=15 Hz, 1H, H-22)

**Ausgangsmaterialien in der 20-Methylenreihe**

**52) (5*E*,7*E*)-(1*S*,3*R*,20*R*)-1,3-Bis[[1,1-dimethylethyl(diphenyl)silyl]oxy]-20,21-epoxy-20-methyl-9,10-secopregna-5,7,10(19)-trien 57**

3,1 g (3,84 mmol) (5*E*,7*E*)-(1*S*,3*R*)-Bis[[1,1-dimethylethyl(diphenyl)silyl]oxyl-9,10-seco-pregna-5,7,10(19)-trien-20-on **56** (Bis-TBDMS-Ether siehe WO 90/09991, Leo Pharmaceutical Products) werden in 70 ml Dimethylformamid unter Argon gelöst und mit 1,06 g (5,2 mmol) Trimethylsulfoniumiodid versetzt. Man kühlt auf 0°C und gibt portionsweise 0,51 g (5,2 mmol) Kalium-tert.-Butanolat zu. Nach 15 Min. bei 0°C wird gesättigte Natriumchlorid-Lösung zugegeben, mit Essigester extrahiert und die organische Phase mehrmals mit Natriumchlorid-Lösung gewaschen. Nach Trocknen über Natriumsulfat entfernt man das Solvens und reinigt den Rückstand an Kieselgel mit Hexan/Essigester, wobei 2,2 g der Titelverbindung als farbloser Schaum anfallen.

$^1$H-NMR ($CDCl_3$): δ= 0,58 ppm (s, 3H, H-18); 0,89 u. 0,94 (2x s, je 9H, Si-t-butyl); 1,32 (s, 3H, H-21); 2,31 u. 2,50 (2x d, J=5 Hz, je 1H, H-22); 4,19 (m, 1H, H-3); 4,59 (t, J=5,5 Hz, 1H, H-1); 4,70 u. 4,82 (2x s, je 1H, H-19); 5,57 u. 6,31 (2x d, J=11 Hz, je 1H, H-6 u. H-7); 7,12-7,68 (m, 20H, Si-phenyl)

**53) (5*E*,7*E*)-(1*S*,3*R*)-1,3-Bis[[1,1-dimethylethyl(diphenyl)silyl]oxy]-20-methylen-9,10-secopregna-5,7,10(19)-trien-21-ol 58**

Man löst 0,28 g (3,8 mmol) Diethylamin unter Argon in 35 ml Diethylether und gibt bei 0°C 2,4 ml (3,8 mmol) n-Butyllithium-Lösung (1,6 M in Hexan) zu. Nach 30 Min. bei dieser Temperatur tropft man 0,72 g (0,88 mmol) **57** in 5 ml Diethylether zu und rührt 1 Std. bei 0°C und 1 Std. bei Raumtemperatur nach. Anschließend wird mit Natriumchlorid-Lösung versetzt, mit Essigester extrahiert und die organische Phase mit Natriumchlorid-Lösung gewaschen. Nach

dem Trocknen über Natriumsulfat engt man ein und chromatographiert den Rückstand an Kieselgel mit Hexan/Essigester, wobei man 360 mg der Titelverbindung als farblosen Schaum neben 280 mg des Ausgangsproduktes erhält.

$^1$H-NMR ($CDCl_3$): δ= 0,45 ppm (s, 3H, H-18); 0,99 u. 1,00 (2x s, je 9H, Si-t-butyl); 4,08 u. 4,17 (2x d, J=14,5 Hz, je 1H, H-22); 4,29 (m, 1H, H-3); 4,65 (m, 1H, H-1); 4,75 u. 4,90 (2x s, je 1H, H-19); 5,03 u. 5,23 (2x s, je 1H, H-21); 5,67 u. 6,39 (2x d, J=11 Hz, je 1H, H-6 u. H-7); 7,20-7,62 (m, 2CH, Si-phenyl)

**54) (5*Z*,7*E*)-(1*S*,3*R*)-1,3-Bis[[1,1-dimethylethyl(diphenyl)silyl]oxy]-20-methylen-9,10-secopregna-5,7,10(19)-trien-21-ol 59**

Man löst 500 mg (0,61 mmol) **58** in 80 ml Toluol, versetzt mit 80 mg (0,44 mmol) Anthracen und 15 µl Triethylamin und bestrahlt 18 Min. in der unter **42)** beschriebenen Apparatur. Nach Aufarbeitung und Reinigung erhält man 450 mg der Titelverbindung als farblosen Schaum.

$^1$H-NMR ($CDCl_3$): δ= 0,43 ppm (s, 3H, H-18); 0,95 u. 1,00 (2x s, je 9H, Si-t-butyl); 4,05 u. 4,15 (2x d, J=14,5 Hz, je 1H, H-22); 4,25 (m, 1H, H-3); 4,55 (m, 1H, H-1); 4,83 u. 5,08 (2x s, je 1H, H-19); 5,00 u. 5,21 (2x s, je 1H, H-21); 6,02 u. 6,10 (2x d, J=11 Hz, je 1H, H-6 u. H-7); 7,15-7,68 (m, 20H, Si-phenyl)

**55) (5*Z*,7*E*)-(1*S*,3*R*)-1,3-Bis[[1,1-dimethylethyl(diphenyl)silyl]oxy]-20-formyl-9,10-secopregna-5,7,10(19),20-tetraen 60**

Man löst 2,8 g (3,36 mmol) **59** in 100 ml Methylenchlorid und gibt 11,6 g (133 mmol) Mangandioxid zu. Es wird 1 Std. bei Raumtemperatur nachgerührt und anschließend über Celite abgesaugt. Nach Entfernen des Lösungsmittels erhält man 2,5 g der Titelverbindung als farblosen Schaum.

$^1$H-NMR ($CDCl_3$): δ= 0,35 ppm (s, 3H, H-18); 0,92 u. 0,99 (2x s, je 9H, Si-t-butyl); 4,23 (m, 1H, H-3); 4,55 (m, 1H, H-1); 4,83 u. 5,10 (2x s, je 1H, H-19); 6,02 u. 6,09 (2x d, J=11 Hz, je 1H, H-6 u. H-7); 6,11 u. 6,32 (2x s, je 1H, H-21); 7,23-7,69 (m, 20H, Si-phenyl); 9,58 (s, 1H, H-22)

**56) (5*Z*,7*E*,22*E*)-(1*S*,3*R*)-Bis[[1,1-dimethylethyl(diphenyl)silyl]oxy]-N-methoxy-N-methyl-9,10-secochola-5,7,10(19),20,22-pentaen-24-amid 61**

Man setzt 1,2 g (1,4 mmol) **60** mit 3,75 g (9 mmol) N-Methoxy-N-methyl-2-(triphenylphosphoranyliden)acetamid analog **39)** um, wobei 1,07 g der Titelverbindung als farbloser Schaum anfallen.

$^1$H-NMR ($CDCl_3$): δ= 0,39 ppm (s, 3H, H-18); 0,93 u. 0,99 (2x s, je 9H, Si-t-butyl); 3,28 (s, 3H, N-Me); 3,75 (s, 3H, N-OMe); 4,25 (m, 1H, H-3); 4,55 (m, 1H, H-1); 4,84 u. 5,10 (2x s, je 1H, H-19); 5,32 u. 5,57 (2x s, je 1H, H-21); 6,04 u. 6,10 (d, J=11 Hz, je 1H, H-6 u. H-7); 6,65 (d, J=15,5 Hz, 1H, H-23); 7,23-7,70 (m, 21H, Si-phenyl u. H-22)

**57) (5*Z*,7*E*,22*E*)-(1*S*,3*R*)-Bis[[1,1-dimethylethyl(diphenyl)silyl]oxy]-9,10-secochola-5,7,10(19),20,22-pentaen-24-al 62**

Man setzt 750 mg (0,8 mmol) **61** mit 4 mmol DIBAH-Lösung analog 40) um, wobei man 500 mg der Titelverbindung als farblosen Schaum erhält.

δ= 0,40 ppm (s, 3H, H-18); 0,92 u. 1,00 (2x s, je 9H, Si-t-butyl); 4,22 (m, 1H, H-3); 4,56 (m, 1H, H-1); 4,82 u. 5,10 (2x s, je 1H, H-19); 5,51 u. 5,68 (2x s, je 1H, H-21); 6,02 u. 6,10 (2x d, J=11 Hz, je 1H, H-6 u. H-7); 6,36 (dd, J=16, 8 Hz, 1H, H-23); 7,18 (d, J=16 Hz, 1H, H-22); 7,22-7,68 (m, 20H, Si-phenyl); 9,60 (d, J=8 Hz, 1H, H-24)

**Beispiel 25**

**58) (5*Z*,7*E*,22*E*)-(1*S*,3*R*,24*R*)-1,3-Bis[[1,1-dimethylethyl(diphenyl)silyl]oxy]-24-hydroxy-9,10-secocholesta-5,7,10(19),20,22-pentaen-25-carbonsäure-1-methylethylester 63a und (5*Z*,7*E*,22*E*)-(1*S*,3*R*,24*S*)-1,3-Bis[[1,1-dimethylethyl(diphenyl)silyl]oxy]-24-hydroxy-9,10-secocholesta-5,7,10(19),20,22-pentaen-25-carbonsäure-1-methylethylester 63b**

Aus 385 mg (3,8 mmol) Diisopropylamin und 2,2 ml (3,4 mmol) n-Butyllithium (1,6 M in Hexan) in 30 ml Tetrahydrofuran wird unter Argon LDA bereitet und auf -78°C gekühlt. Man gibt nun 442 mg (3,4 mmol) Isobuttersäureisopropylester zu, rührt 30 Min. bei -78°C und addiert dann 650 mg (0,76 mmol) **62** in 5 ml Tetrahydrofuran. Es wird 3 Std. bei -78°C nachgerührt und anschließend auf Natriumchlorid-Lösung gegossen. Nach Extraktion mit Essigester, Waschen der organischen Phase mit Natriumchlorid-Lösung, Trocknen über Natriumsulfat und Entfernen des Lösungsmittels wird der Rückstand chromatographisch aufgetrennt, wobei nacheinander 207 mg der **63b** und 160 mg **63a** als farblose Schäume anfallen.

$^1$H-NMR ($CD_2Cl_2$): **63a:** δ= 0,37 ppm (s, 3H, H-18); 0,94 u. 0,96 (2x s, je 9H, Si-t-butyl); 1,13 u. 1,14 (2x s, je 3H, H-26 u. H-27); 1,22 (d, J=7 Hz, 6H, COOiPr); 4,14 (m, 1H, H-24); 4,24 (m, 1H, H-3); 4,54 (m, 1H, H-1); 4,81 u. 5,17 (2x s, je 1H, H-19); 4,98 (hept, J=7 Hz, 1H, COOiPr); 4,99 u. 5,07 (2x s, je 1H, H-21); 5,78 (dd, J=15,5, 6 Hz, H-23); 6,02 u. 6,12 (2x d, J=11 Hz, je 1H, H-6 u. H-7); 6,24 (d, J=15,5 Hz, 1H, H-22); 7,22-7,65 (m, 20H, Si-phenyl) $^1$H-NMR ($CD_2Cl_2$): **63b:** δ= 0,37 ppm (s, 3H, H-18); 0,94 u. 0,96 (2x s, je 9H, Si-t-butyl); 1,15 u. 1,18 (2x s, je 3H, H-26 u. H-27); 1,23 (d, J=7 Hz, 6H, COOiPr); 4,14 (m, 1H, H-24); 4,24 (m, 1H, H-3); 4,54 (m, 1H, H-1); 4,82 u. 5,19 (2x s, je 1H, H-19); 4,99 (hept, J=7 Hz, 1H, COOiPr); 5,00 u. 5,08 (2x s, je 1H, H-21); 5,80 (dd, J=15,5, 6 Hz, H-23); 6,02 u. 6,12 (2x d, J=11 Hz, je 1H, H-6 u. H-7); 6,28 (d, J=15,5 Hz, 1H, H-22); 7,22-7,65 (m, 20H, Si-phenyl)

**59) (5*Z*,7*E*,22*E*)-(1*S*,3*R*,24*R*)-1,3,24-Trihydroxy-9,10-secocholesta-5,7,10(19),20,22-pentaen-25-carbonsäure-1-methylethylester 64a**

Man rührt 160 mg (0,16 mmol) **63a** mit 162 mg (1,6 mmol) Dihydropyran und einer Spatelspitze Pyridinium-p-toluolsulfonat in 5 ml Methylenchlorid unter Argon für 24 Std. bei Raumtemperatur. Dann wird mit Natriumchlorid-Lösung gewaschen, über Natriumsulfat getrocket, das Lösungsmittel entfernt und der Rückstand chromatographisch gereinigt. Das so erhaltene Diastereomerengemisch wird in 20 ml Tetrahydrofuran gelöst und mit 408 mg (1,3 mmol) Tetrabutylammoniumfluorid bei 60°C unter Argon gerührt. Man gießt auf Natriumchlorid-Lösung, extrahiert mit Essigester, wäscht mit Natriumchlorid-Lösung und engt ein. Der Rückstand wird nun mit 420 mg Amberlite (aktiviert) in 5 ml Methanol/Methylenchlorid (9:1) bei Raumtemperatur gerührt. Anschließend filtriert man, wäscht das Filtrat mit Natriumhydrogencarbonat-Lösung und Natriumchlorid-Lösung, trocknet über Natriumsulfat und entfernt das Solvens. Das Rohprodukt wird chromatographisch an Kieselgel mit Hexan/Essigester gereinigt, wobei man 23 mg der Titelverbindung als farblosen Schaum erhält.

$^1$H-NMR ($CD_2Cl_2$): δ= 0,42 ppm (s, 3H, H-18); 1,11 u. 1,12 (2x s, je 3H, H-26 u. H-27); 1,22 (d, J=7 Hz, 6H, COOiPr); 4,15 (m, 2H, H-3 u. H-24); 4,37 (m, 1H, H-1); 4,95 u. 5,30 (2x s, je 1H, H-19); 4,97 (hept, J=7 Hz, 1H, COOiPr); 5,00 u. 5,17 (2x s, je 1H, H-21); 5,78 (dd, J=15,5, 6 Hz, H-23); 6,03 u. 6,34 (2x d, J=11 Hz,je 1H, H-6 u. H-7); 6,22 (d, J=15,5 Hz, 1H, H-22)

**60) (5*Z*,7*E*,22*E*)-(1*S*,3*R*,24*S*)-1,3,24-Trihydroxy-9,10-secocholesta-5,7,10(19),20,22-pentaen-25-carbonsäure-1-methylethylester 64b**

Man setzt 207 mg (0,21 mmol) **63b** analog **59)** um und erhält 28 mg der Titelverbindung als farblosen Schaum.

$^1$H-NMR ($CD_2Cl_2$): δ= 0,42 ppm (s, 3H, H-18); 1,12 u. 1,15 (2x s, je 3H, H-26 u. H-27); 1,22 (d, J=7 Hz, 6H, COOiPr); 4,14 (m, 2H, H-3 u. H-24); 4,36 (m, 1H, H-1); 4,95 u. 5,30 (2x s, je 1H, H-19); 4,96 (hept, J=7 Hz, 1H, COOiPr); 4,98 u. 5,16 (2x s, je 1H, H-21); 5,80 (dd, J=15,5, 6 Hz, H-23); 6,03 u. 6,34 (2x d, J=11 Hz, je 1H, H-6 u. H-7); 6,25 (d, J=15,5 Hz, 1H, H-22)

**Beispiel 26**

**61) (5*Z*,7*E*,22*E*)-(1*S*,3*R*,4*R*)-1,3-Bis[[1,1-dimethylethyl(diphenyl)silyl]oxy]-24-hydroxy-9,10-secocholesta-5,7,10(19),20,22-pentaen-25-carbonsäuremethylestär 65a und (5*Z*,7*E*,22*E*)-(1*S*,3*R*,24*S*)-1,3-Bis[[1,1-dimethylethyl(diphenyl)silyl]oxy]-24-hydroxy-9,10-secocholesta-5,7,10(19),20,22-pentaen-25-carbonsäuremethylester 65b**

Man setzt 400 mg (0,47 mmol) **62** analog **58)** mit 1,2 mmol LDA und 0,18 ml (1,18 mmol) Isobuttersäuremethylester um und erhält nach Trennung der Diastereomeren über HPLC (Normalphase, Methylenchlorid/Methanol) 134 mg **65a** sowie 128 mg **65b** als farblose Schäume.

$^1$H-NMR ($CDCl_3$): **65a**: δ= 0,36 ppm (s, 3H, H-18); 0,92 u. 0,95 (2x s, je 9H, Si-t-butyl); 1,14 (s, 6H, H-26 u. H-27); 2,48 (d, J=5 Hz, 1H, OH); 3,66 (s, 3H, COOMe); 4,16 (dd, J=6, 5Hz, 1H, H-24); 4,24 (m, 1H, H-3); 4,53 (m, 1H, H-1); 4,81 u. 5,18 (2x s, je 1H, H-19); 4,99 u. 5,06 (2x s, je 1H, H-21); 5,77 (dd, J=15,5, 6 Hz, 1H, H-23); 6,02 u. 6,12 (2x d, J=11 Hz, je 1H, H-6 u. H-7); 6,24 (d, J=15,5 Hz, 1H, H-22); 7,21-7,66 (m, 20H, Si-phenyl)

$^1$H-NMR ($CDCl_3$): **65b:** δ= 0,36 ppm (s, 3H, H-18); 0,93 u. 0,96 (2x s, je 9H, Si-t-butyl); 1,15 (s, 6H, H-26 u. H-27); 2,48 (d, J=5 Hz, 1H, OH); 3,65 (s, 3H, COOMe); 4,17 (dd, J=6, 5 Hz, 1H, H-24); 4,24 (m, 1H, H-3); 4,53 (m, 1H, H-1); 4,82 u. 5,18 (2x s, je 1H, H-19); 4,99 u. 5,06 (2x s, je 1H, H-21); 5,79 (dd, J=15,5, 6 Hz, 1H, H-23); 6,02 u. 6,12 (2x d, J=11 Hz, je 1H, H-6 u. H-7); 6,27 (d, J=15,5 Hz, 1H, H-22); 7,23-7,70 (m, 20H, Si-phenyl)

**62) (5*Z*,7*E*,22*E*)-(1*S*,3*R*,24*R*)-1,3,24-Trihydroxy-9,10-secocholesta-5,7,10(19),20,22-pentaen-25-carbonsäure-methylester 66a**

Man setzt 134 mg (0,14 mmol) **65a** analog **59)** um und erhält 14 mg der Titelverbindung als farblosen Schaum.

$^1$H-NMR ($CD_2Cl_2$): δ= 0,43 ppm (s, 3H, H-18); 1,17 (s, 6H, H-26 u. H-27); 2,52 (d, J=6 Hz, 1H, OH); 3,68 (s, 3H, COOMe); 4,17 (dd, J=7, 6 Hz, 1H, H-24); 4,17 (m, 1H, H-3); 4,38 (m, 1H, H-1); 4,97 u. 5,30 (2x s, je 1H, H-19); 5,01 u. 5,20 (2x s, je 1H, H-21); 5,77 (dd, J=15,5, 6 Hz, 1H, H-23); 6,03 u. 6,36 (2x d, J=11 Hz, je 1H, H-6 u. H-7); 6,25 (d, J=15,5 Hz, 1H, H-22)

**63) (5*Z*,7*E*,22*E*)-(1*S*,3*R*,24*S*)-1,3,24-Trihydroxy-9,10-secocholesta-5,7,10(19),20,22-pentaen-25-carbonsäure-methylester 66b**

Man setzt 128 mg (0,13 mmol) **65b** analog **59)** um und erhält 19 mg der Titelverbindung als farblosen Schaum.

$^1$H-NMR ($CD_2Cl_2$): δ= 0,44 ppm (s, 3H, H-18); 1,15 u. 1,16 (2x s, je 3H, H-26 u. H-27); 2,48 (d, J=6 Hz, 1H, OH); 3,66 (s, 3H, COOMe); 4,18 (dd, J=7, 6 Hz, 1H, H-24); 4,17 (m, 1H, H-3); 4,37 (m, 1H, H-1); 4,96 u. 5,31 (2x s, je 1H, H-19); 5,00 u. 5,18 (2x s, je 1H, H-21); 5,79 (dd, J=15,5, 6 Hz, 1H, H-23); 6,04 u. 6,36 (2x d, J=11 Hz, je 1H, H-6 u. H-7); 6,28 (d, J=15,5 Hz, 1H, H-22)

**Beispiel 27**

**64) (5*Z*,7*E*,22*E*)-(1*S*,3*R*,24*R*)-1,3-Bis[[1,1-dimethylethyl(diphenyl)silyl]oxy]-24-hydroxy-9,10-secocholesta-5,7,10(19),20,22-pentaen-25-carbonsäureethylester 67a und (5*Z*,7*E*,22*E*)-(1*S*,3*R*,24*S*)-1,3-Bis[[1,1-dimethylethyl(diphenyl)silyl]oxyl-24-hydroxy-9,10-secocholesta-5,7,10(19),20,22-pentaen-25-carbonsäureethylester 67b**

Man setzt 400 mg (0,47 mmol) **62** analog **58)** mit 1,2 mmol LDA und 0,16 ml (1,18 mmol) Isobuttersäureethylester um und erhält nach Trennung der Diastereomeren über HPLC (Normalphase, Methylenchlorid/Methanol) 105 mg **67a** sowie 90 mg **67b** als farblose Schäume.

$^1$H-NMR ($CDCl_3$): **67a:** δ= 0,38 ppm (s, 3H, H-18); 0,95 u. 1,00 (2x s, je 9H, Si-t-butyl); 1,20 (s, 6H, H-26 u. H-27); 1,29 (t, J=7 Hz, 3H, COOEt); 4,18 (q, J=7 Hz, 2H, COOEt); 4,20 (m, 2H, H-3 u. H-24); 4,54 (m, 1H, H-1); 4,83 u. 5,20 (2x s, je 1H, H-19); 5,01 u. 5,09 (2x s, je 1H, H-21); 5,80 (dd, J=15,5, 6 Hz, 1H, H-23); 6,02 u. 6,09 (2x d, J=11 Hz, je 1H, H-6 u. H-7); 6,29 (d, J=15,5 Hz, 1H, H-22) ; 7,20-7,65 (m, 20H, Si-phenyl)

$^1$H-NMR ($CDCl_3$): **67b:** δ= 0,39 ppm (s, 3H, H-18); 0,94 u. 1,00 (2x s, je 9H, Si-t-butyl); 1,18 u. 1,22 (2x s, je 3H, H-26 u. H-27); 1,28 (t, J=7 Hz, 3H, COOEt); 4,18 (q, J=7 Hz, 2H, COOEt); 4,21 (m, 2H, H-3 u. H-24); 4,54 (m, 1H, H-1); 4,83 u. 5,20 (2x s, je 1H, H-19); 5,00 u. 5,08 (2x s, je 1H, H-21); 5,82 (dd, J=15,5, 6 Hz, 1H, H-23); 6,02 u. 6,10 (2x d, J=11 Hz, je 1H, H-6 u. H-7); 6,30 (d, J=15,5 Hz, 1H, H-22); 7,20-7,65 (m, 20H, Si-phenyl)

**65) (5*Z*,7*E*,22*E*)-(1*S*,3*R*,24*R*)-1,3,24-Trihydroxy-9,10-secocholesta-5,7,10(19),20,22-pentaen-25-carbonsäure-ethylester 68a**

Man setzt 100 mg (0,11 mmol) **67a** analog **59)** um und erhält 15 mg der Titelverbindung als farblosen Schaum.

$^1$H-NMR ($CD_2Cl_2$): δ= 0,43 ppm (s, 3H, H-18); 1,15 (s, 6H, H-26 u. H-27); 1,22 (t, J=7 Hz, 3H, COOEt); 4,10 (q, J=7 Hz, 2H, COOEt); 4,18 (m, 2H, H-3 u. H-24); 4,39 (m, 1H, H-1); 4,98 u. 5,30 (2x s, je 1H, H-19); 5,01 u. 5,20 (2x s, je 1H, H-21); 5,78 (dd, J=15,5, 6 Hz, 1H, H-23); 6,03 u. 6,37 (2x d, J=11 Hz, je 1H, H-6 u. H-7); 6,25 (d, J=15,5 Hz, 1H, H-22)

**66) (5*Z*,7*E*,22*E*)-(1*S*,3*R*,24*S*)-1,3,24-Trihydroxy-9,10-secocholesta-5,7,10(19),20,22-pentaen-25-carbonsäure-ethylester 68b**

Man setzt 89 mg (0,11 mmol) **67b** analog **59)** um und erhält 13 mg der Titelverbindung als farblosen Schaum.

$^1$H-NMR ($CD_2Cl_2$): δ= 0,43 ppm (s, 3H, H-18); 1,17 u. 1,18 (2x s, je 3H, H-26 u. H-27); 1,23 (t, J=7 Hz, 3H, COOEt); 4,11 (q, J= 7 Hz, 2H, COOEt); 4,18 (m, 2H, H-3 u. H-24); 4,38 (m, 1H, H-1); 4,97 u. 5,30 (2x s, je 1H, H-19); 4,99 u. 5,20 (2x s, je 1H, H-21); 5,80 (dd, J=15,5, 6 Hz, 1H, H-23); 6,04 u. 6,35 (2x d, J=11 Hz, je 1H, H-6 u. H-7); 6,28 (d, J=15,5 Hz, 1H, H-22)

**Ausgangsmaterialien in der 19-Nor-Reihe mit Normalkonfiguration in 20-Position**

**67) [1*R*-[1α[*R**-(*E*)],3aβ,4α,7aα]]-N-Methoxy-4-[4-(methoxymethoxy)-7a-methyl-octahydro-1H-inden-1-yl]-N-methyl-2-pentenamid <u>70</u>**

Man setzt 10,0 g (39,3 mmol) [1*R*-[1α(*S**),3aβ,4α,7aα]]-α,7a-Dimethyl-4-(methoxy-methoxy)octahydro-1H-inden-1-acetaldehyd **<u>69</u>** [H.H. Inhoffen et al. Chem. Ber. 91, 780 (1958), Chem. Ber. **<u>92,</u>** 1772 (1959), W.G. Dauben et al. Tetrahedron Lett. **<u>30,</u>** 677 (1989)] mit 41,9 g (117,8 mmol) N-Methoxy-N-methyl-(2-triphenylphosphoranyliden)acetamid analog 39) um, wobei 10,6 g der Titelverbindung als farbloses Öl anfallen.

$^1$H-NMR ($CDCl_3$): δ= 0,89 ppm (s, 3H, H-18); 1,03 (d, J=7 Hz, 3H, H-21); 3,19 (s, 3H, N-Me); 3,30 (s, 3H, OMe); 3,65 (s, 3H, N-OMe); 3,80 (m, 1H, H-8); 4,48 u. 4,60 (2x d, J=6 Hz, je 1H, $OCH_2O$); 6,28 (d, J=15 Hz, 1H, H-23); 6,78 (d, J=15, 9 Hz, 1H, H-22)

**68) [1*R*-[1α[*R**-(*E*)],3aβ,4α,7aα]]-4-(4-Hydroxy-7a-methyloctahydro-1H-inden-1-yl)-N-methoxy-N-methyl-2-pentenamid <u>71</u>**

Man versetzt 5,3 g (15,6 mmol) **<u>70</u>** in 250 ml THF mit 4,45 g (23,5 mmol) p-Toluolsulfonsäure und rührt über Nacht bei 70°C. Nach dem Abkühlen wird Natriumchlorid-Lösung zugegeben und mit Essigester extrahiert. Die organische Phase wäscht man mit Natriumhydrogencarbonat-Lösung und Natriumchlorid-Lösung, trocknet über Natriumsulfat und entfernt das Solvens. Der Rückstand wird durch Chromatographie an Kieselgel mit Hexan/Essigester gereinigt, wobei man 3,08 g der Titelverbindung als farbloses Öl erhält.

$^1$H-NMR ($CDCl_3$): δ= 0,99 (s, 3H, H-18); 1,08 (d, J=7 Hz, 3H, H-21); 3,22 (s, 3H, N-Me); 3,70 (s, 3H, N-OMe); 4,08 (m, 1H, H-8); 6,30 (d, J=15 Hz, 1H, H-23); 6,81 (dd, J=15, 9 Hz, 1H, H-22)

**69) 1*R*-[1α[*R**-(*E*)],3aβ,7aα]]-N-Methoxy-N-methyl-4-(7a-methyl-4-oxooctahydro-4H-inden-1-yl)-2-pentenamid <u>72</u>**

Man rührt 3,08 g (10,5 mmol) **<u>71</u>** in 150 ml Methylenchlorid mit 3,16 g (14,7 mmol) Pyridiniumchlorochromat für 2 Stunden. Anschließend wird mit Diethylether verdünnt, über Celite filtriert, das Solvens entfernt und der Rückstand an Kieselgel mit Hexan/Essigester chromatographiert, wobei 2,07 g der Titelverbindung als farbloses Öl verbleiben.

$^1$H-NMR ($CDCl_3$): δ= 0,69 ppm (s, 3H, H-18); 1,13 (s, 3H, H-21); 2,48 (dd, J=10,5, 7,5 Hz, 1H, H-14); 3,23 (s, 3H, N-Me); 3,70 (s, 3H, N-OMe); 6,33 (d, J=15 Hz, 1H, H-23); 6,81 (dd, J=15, 9 Hz, 1H, H-22)

**70) (7*E*,22*E*)-(1*R*,3*R*)-1,3-Bis[[dimethyl(1,1-dimethylethyl)silyl]oxy]-N-methoxy-N-methyl-19-nor-9,10-secochola-5,7,22-trien-24-amid <u>73</u>**

Man löst 1,5 g (2,63 mmol) (3*R*-trans)-[2-[3,5-Bis[[dimethyl(1,1-dimethylethyl)silyl]oxy]-cyclohexyliden]ethyl] diphenylphosphinoxid [H.F. DeLuca et al. Tetrahedron Lett. **<u>32</u>**, 7663 (1991)] in 15 ml THF und kühlt unter Argon auf -70°C. Nun tropft man 1,94 ml (3,16 mmol) n-Butyllithium-Lösung (1,6 M in Hexan) zu. Nach 5 Min. werden nun 696 mg (2,39 mmol) **<u>72</u>** in 15 ml THF zugetropft und 30 Min. bei dieser Temperatur gerührt. Anschließend hydrolisiert man mit Kalium-Natriumtartrat/Kaliumhydrogencarbonat-Lösung, extrahiert mit Essigester, wäscht die organische Phase mit Natriumchlorid-Lösung, trocknet über Natriumsulfat und entfernt das Solvens. Der Rückstand wird an Kieselgel mit Hexan/Essigester chromatographiert, wobei man 390 mg der Titelverbindung als farblosen Schaum erhält.

$^1$H-NMR ($CDCl_3$): δ= 0,05 u. 0,06 ppm (2x s, je 6H, SiMe); 0,58 (s, 3H, H-18); 0,86 u. 0,87 (2x s, je 3H, Si-t-butyl); 1,13 (d, J=7 Hz, 3H, H-21); 3,23 (s, 3H, N-Me); 3,70 (s, 3H, N-OMe); 4,08 (m, 2H, H-1 u. H-3); 5,81 u. 6,17 (2x d, J=11 Hz, je 1H, H-6 u. H-7); 6,32 (d, J=15 Hz, 1H, H-23); 6,85 (dd, J=15, 9 Hz, 1H, H-22)

**71) (7*E*,22*E*)-(1*R*,3*R*)-1,3-Bis[[dimethyl(1,1-dimethylethyl)silyl]oxy]-19-nor-9,10-secochola-5,7,22-trien-24-al 74**

Man setzt 380 mg (0,59 mmol) **<u>73</u>** mit 1,48 mmol DIBAH-Lösung analog **40)** um und erhält 305 mg der Titelverbindung als farblosen Schaum.

$^1$H-NMR ($CDCl_3$): δ= 0,05 u. 0,06 ppm (2x s, je 6H, SiMe); 0,60 (s, 3H, H-18); 0,87 u. 0,88 (2x s, je 3H, Si-t-butyl); 1,17 (d, J=7 Hz, 3H, H-21); 4,08 (m, 2H, H-1 u. H-3); 5,82 u. 6,17 (2x d, J=11 Hz, je 1H, H-6 u. H-7); 6,07 (dd, J=15, 7 Hz, 1H, H-23); 6,72 (dd, J=15, 9 Hz, 1H, H-22); 9,49 (d, J=7 Hz, 1H, H-24)

**Beispiel 28**

**72) (7*E*,22*E*)-(1*R*,3*R*,24*R*)-1,3-Bis[[dimethyl(1,1-dimethylethyl)silyl)oxy)-24-hydroxy-19-nor-9,10-secocholesta-5,7,22-trien-25-carbonsäure-1-methylethylester 75a und (7*E*,22*E*)-(1*R*,3*R*,24*S*)-1,3-Bis[[dimethyl(1,1-dimethylethyl)silyl]oxy]-24-hydroxy-19-nor-9,10-secocholesta-5,7,22-trien-25-carbonsäure-1-methylethylester 75b**

Analog 41) werden 305 mg (0,52 mmol) **74** mit 2,1 mmol LDA und 0,28 ml (2,07 mmol) Isobuttersäureisopropylester umgesetzt, wobei nach Aufreinigung über HPLC (Normalphase, Laufmittel: Methylenchlorid/Methanol) 62 mg **75a** sowie 115 mg **75b** als farblose Schäume anfallen.

$^1$H-NMR ($CD_2Cl_2$): **75a:** δ= 0,02 ppm (s, 12H, SiMe); 0,51 (s, 3H, H-18); 0,82 u. 0,83 (2x s, je 9H, Si-t-butyl); 1,01 (d, J=7 Hz, 3H, H-21); 1,08 u. 1,09 (2x s, je 3H, H-26 u. H-27); 1,20 (d, J=7 Hz, 6H, COOiPr); 2,45 (d, J=6 Hz, 1H, OH); 4,02 (m, 3H, H-1, H-3 u. H-24); 4,96 (hept, J=7 Hz, 1H, COOiPr); 5,31 (dd, J=15, 7 Hz, 1H, H-23); 5,48 (dd, J=15, 9 Hz, 1H, H-22); 5,78 u. 6,12 (2x d, J=11 Hz, je 1H, H-6 u. H-7)

$^1$H-NMR ($CD_2Cl_2$): **75b:** δ= 0,02 ppm (s, 12H, SiMe); 0,50 (s, 3H, H-18); 0,82 u. 0,83 (2x s, je 9H, Si-t-butyl); 1,00 (d, J=7 Hz, 3H, H-21); 1,07 u. 1,08 (2x s, je 3H, H-26 u. H-27); 1,19 (d, J=7 Hz, 6H, COOiPr); 4,02 (m, 3H, H-1, H-3 u. H-24); 4,93 (hept, J=7 Hz, 1H, COOiPr); 5,33 (dd, J=15, 7 Hz, 1H, H-23); 5,52 (dd, J=15, 9 Hz, 1H, H-22); 5,78 u. 6,13 (2x d, J=11 Hz, je 1H, H-6 u. H-7)

**73) (7*E*,22*E*)-(1*R*,3*R*,24*R*)-1,3,24-Trihydroxy-19-nor-9,10-secocholesta-5,7,22-trien-25-carbonsäure-1-methylethylester 76a**

Man rührt 59 mg (0,082 mmol) **75a** mit 600 mg Dowex in 6 ml Methanol/Methylenchlorid 9:1 für 3 Tage. Nach Filtration entfernt man das Solvens und chromatographiert den Rückstand an Kieselgel mit Hexan/Essigester, wobei 22 mg der Titelverbindung als farbloser Schaum erhalten werden.

$^1$H-NMR ($CD_2Cl_2$): δ= 0,58 ppm (s, 3H, H-18); 1,03 (d, J=7 Hz, 3H, H-21); 1,11 u. 1,12 (2x s, je 3H, H-26 u. H-27); 1,22 (d, J=7 Hz, 6H, COOiPr); 2,51 (d, J=6 Hz, 1H, OH); 4,02 (m, 3H, H-1, H-3 u. H-24); 4,98 (hept, J=7 Hz, 1H, COOiPr); 5,35 (dd, J=15, 7 Hz, 1H, H-23); 5,51 (dd, J=15 Hz, 1H, H-22); 5,84 u. 6,28 (2x d, J=11 Hz, je 1H, H-6 u. H-7)

**74) (7*E*,22*E*)-(1*R*,3*R*,24*S*)-1,3,24-Trihydroxy-19-nor-9,10-secocholesta-5,7,22-trien-25-carbonsäure-1-methylethylester 76b**

Man setzt 112 mg (0,156 mmol) **75b** analog **73)** um, wobei 81 mg der Titelverbindung als farbloser Schaum erhalten werden.

$^1$H-NMR ($CD_2Cl_2$): δ= 0,57 ppm (s, 3H, H-18); 1,03 (d, J=7 Hz, 3H, H-21); 1,12 u. 1,13 (2x s, je 3H, H-26 u. H-27); 1,22 (d, J=7 Hz, 6H, COOiPr); 2,50 (d, J=6 Hz, 1H, OH); 4,02 (m, 3H, H-1, H-3 u. H-24); 4,98 (hept, J=7 Hz, 1H, COOiPr); 5,38 (dd, J=15, 7 Hz, 1H, H-23); 5,57 (dd, J=15 Hz, 1H, H-22); 5,84 u. 6,28 (2x d, J=11 Hz, je 1H, H-6 u. H-7)

**Beispiel 29**

**75) (7*E*,22*E*)-(1*R*,3*R*,24*R*)-1,3-Bis[[dimethyl(1,1-dimethylethyl)silyl]oxy]-24-hydroxy-19-nor-9,10-secocholesta-5,7,22-trien-25-carbonsäureethylester 77a und (7*E*,22*E*)-(1*R*,3*R*,24*S*)-1,3-Bis[[dimethyl(1,1-dimethylethyl)silyl]oxy]-24-hydroxy-19-nor-9,10-secocholesta-5,7,22-trien-25-carbonsäureethylester 77b**

Analog **41)** werden 280 mg (0,48 mmol) **74** mit 2 mmol LDA und 0,26 mg (1,9 mmol) Isobuttersäureethylester umgesetzt und analog **72)** aufgereinigt, wobei 58 mg **77a** sowie 86 mg **77b** als farblose Schäume anfallen.

$^1$H-NMR ($CD_2Cl_2$): **77a:** δ= 0,02 ppm (s, 12H, SiMe); 0,51 (s, 3H, H-18); 0,82 u. 0,84 (2x s, je 9H, Si-t-butyl); 1,02 (d, J=7 Hz, 3H, H-21); 1,08 u. 1,09 (2x s, je 3H, H-26 u. H-27); 1,27 (t, J=7 Hz, 3H, COOEt); 2,46 (d, J=6 Hz, 1H, OH); 4,02 (m, 3H, H-1, H-3 u. H-24); 4,05 (q, J=7 Hz, 2H, COOEt); 5,32 (dd, J=15, 7 Hz, 1H, H-23); 5,49 (dd, J=15, 9 Hz, 1H, H-22); 5,78 u. 6,12 (2x d, J=11 Hz, je 1H, H-6 u. H-7)

$^1$H-NMR ($CD_2Cl_2$): **77b:** δ= 0,02 ppm (s, 12H, SiMe); 0,51 (s, 3H, H-18); 0,82 u. 0,83 (2x s, je 9H, Si-t-butyl); 1,01 (d, J=7 Hz, 3H, H-21); 1,07 u. 1,08 (2x s, je 3H, H-26 u. H-27); 1,28 (t, J=7 Hz, 3H, COOEt); 4,02 (m, 3H, H-1, H-3 u. H-24); 4,05 (q, J=7 Hz, 2H, COOEt); 5,34 (dd, J=15, 7 Hz, 1H, H-23); 5,54 (dd, J=15, 9 Hz, 1H, H-22); 5,78 u. 6,13 (2x d, J=11 Hz, je 1H, H-6 u.H-7)

**76) (7*E*,22*E*)-(1*R*,3*R*,24*R*)-1,3,24-Trihydroxy-19-nor-9,10-secocholesta-5,7,22-trien-25-carbonsäureethylester 78a**

Man setzt 58 mg (0,0,82 mmol) **77a** analog **73)** um, wobei 20 mg der Titelverbindung als farbloser Schaum erhalten werden.

$^1$H-NMR ($CD_2Cl_2$): δ= 0,57 ppm (s, 3H. H-18); 1,04 (d, J=7 Hz, 3H, H-21); 1,11 u. 1,12 (2x s, je 3H, H-26 u. H-27); 1,29 (t, J=7 Hz, 3H, COOEt); 2,53 (d, J=6 Hz, 1H, OH); 4,03 (m, 3H, H-1, H-3 u. H-24); 4,07 (q, J=7 Hz, 2H, COOEt); 5,36 (dd, J=15, 7 Hz, 1H, H-23); 5,52 (dd, J=15 Hz, 1H, H-22); 5,85 u. 6,28 (2x d, J=11 Hz, je 1H, H-6 u. H-7)

**77) (7*E*,22*E*)-(1*R*,3*R*,24*S*)-1,3,24-Trihydroxy-19-nor-9,10-secocholesta-5,7,22-trien-25-carbonsäureethylester 78b**

Man setzt 72 mg (0,102 mmol) **77b** analog **73)** um, wobei 51 mg der Titelverbindung als farbloser Schaum erhalten werden.

$^1$H-NMR ($CD_2Cl_2$): δ= 0,58 ppm (s; 3H, H-18); 1,04 (d, J=7 Hz, 3H, H-21); 1,12 u. 1,13 (2x s, je 3H, H-26 u. H-27); 1,28 (t, J=7 Hz, 3H, COOEt); 2,52 (d, J=6 Hz, 1H, OH); 4,03 (m, 3H, H-1, H-3 u. H-24); 4,06 (q, J=7 Hz, 2H, COOEt); 5,39 (dd, J=15, 7 Hz, 1H, H-23); 5,58 (dd, J=15 Hz, 1H, H-22); 5,85 u. 6,28 (2x d, J=11 Hz, je 1H, H-6 u. H-7)

**Beispiel 30**

**78) (7*E*,22*E*)-(1*R*,3*R*)-1,3-Bis[[dimethyl(1,1-dimethylethyl)silyl]oxy]-24-oxo-19-nor-9,10-secocholesta-5,7,22-trien-25-carbonsäureethylester 79**

Man legt bei -50°C 0,04 ml (0,4 mmol) Oxalylchlorid in 5 ml Methylenchlorid vor, tropft 0,06 ml (0,8 mmol) DMSO in 1 ml Methylenchlorid zu und rührt 2 Min nach. Anschließend werden 80 mg (0,11 mmol) **75a/75b**-Gemisch in 1 ml Methylenchlorid zugetropft. Nach 15 Min. bei -50°C gibt man dann 0,26 ml Triethylamin hinzu und läßt die Reaktionsmischung auf Raumtemperatur kommen. Man hydrolysiert mit Natriumchlorid-Lösung, extrahiert mit Essigester, wäscht mit Natriumchlorid-Lösung, trocknet über Natriumsulfat und entfernt das Lösungsmittel. Der Rückstand wird an Kieselgel mit Hexan/Essigester chromatographiert, wobei 51 mg der Titelverbindung als farbloser Schaum verbleiben.

$^1$H-NMR ($CD_2Cl_2$): δ= 0,01 u. 0,05 ppm (2x s, je 6H, SiMe); 0,51 (s, 3H, H-18); 0,83 (s, 18H, Si-t-butyl); 1,05 (d, J=7 Hz, 3H, H-21); 1,18 (d, J=7 Hz, 6H, COOiPr); 1,29 (s, 6H, H-26 u. H-27); 4,05 (m, 2H, H-1 und H-3); 4,98 (hept, J=7 Hz, 1H, COOiPr); 5,78 u. 6,11 (2x d, J=11 Hz, je 1H, H-6 u. H-7); 6,10 (J=15 Hz, 1H, H-23); 6,77 (dd, J=15, 9,5 Hz, 1H, H-22)

**79) (7*E*,22*E*)-(1*R*,3*R*)-1,3-Dihydroxy-24-oxo-19-nor-9,10-secocholesta-5,7,22-trien--25-carbonsäure-1-methylethylester 80**

Man setzt 48 mg (0,067 mmol) **79** analog **73)** um, wobei 19 mg der Titelverbindung als farbloser Schaum erhalten werden.

$^1$H-NMR ($CD_2Cl_2$): δ= 0,51 ppm (s, 3H, H-18); 1,08 (d, J=7 Hz, 3H, H-21); 1,20 (d, J=7 Hz, 6H, COOiPr); 1,31 (s, 6H, H-26 u. H-27); 3,98 u. 4,08 (2x m, je 1H, H-1 und H-3); 5,00 (hept, J=7 Hz, 1H, COOiPr); 5,83 u. 6,28 (2x d, J=11 Hz, je 1H, H-6 u. H-7); 6,15 (J=15 Hz, 1H, H-23); 6,80 (dd, J=15, 9,5 Hz, 1H, H-22)

**Ausgangsmaterialien in der 19-Nor-Reihe mit 20-Modifikationen**

**80) [1*S*-(1α,3aβ,4α,7aα)]-1-[4-(Methoxymethoxy)-7a-methyloctahydro-1H-inden-1-yl]-1-ethanon 81**

Man rührt 12,2 g (47,9 mmol) [1*R*-[1α(*S**),3aβ,4α,7aα]]-α,7a-Dimethyl-4-(methoxymethoxy)octahydro-1H-inden-1-acetaldehyd **69** [H.H. Inhoffen et al. Chem. Ber. **91,** 780 (1958), Chem. Ber. **92,** 1772 (1959), W.G. Dauben et al. Tetrahedron Lett. **30,** 677 (1989)] in 600 ml DMF mit 4,78 g (41,9 mmol) DABCO, 720 mg (3,6 mmol) Kupfer(II)acetat und 574 mg (3,6 mmol) Bipyridyl unter Lufteinleitung bei 70°C für 24 Std. Nach dem Abkühlen der Mischung wird mit Natriumchlorid-Lösung versetzt, mit Essigester extrahiert und die organische Phase gründlich mit Natriumchlorid-Lösung gewaschen. Man trocknet über Natriumsulfat, entfernt das Solvens und reinigt den Rückstand chromatographisch an Kieselgel mit Hexan/Essigester, wobei 7,1 g der Titelverbindung als farbloses Öl verbleiben.

$^1$H-NMR ($CDCl_3$): δ= 0,82 ppm (s, 3H, H-18); 2,10 (s, 3H, H-21); 2,49 (t, J=9 Hz, 1H, H-17); 3,34 (s, 3H, OMe); 4,52 u. 4,63 (2x d, J=6 Hz, je 1H, $OCH_2O$)

**81) [1*S*-[1α(*R*)*,3aβ,4α,7aα]]-1-[4-(Methoxymethoxy)-7a-methyloctahydro-1H-inden -1-yl]-1-methyloxiran** **82**

Man setzt 6,1 g (25,38 mmol) **81** analog **52)** um, wobei man 6,4 g der Titelverbindung als farbloses Öl erhält.

$^1$H-NMR ($CDCl_3$): δ= 1,03 ppm (s, 3H, H-18); 1,38 (s, 3H, H-21); 2,31 u. 2,50 (2x d, J=5 Hz, je 1H, H-22); 3,35 (s, 3H, OMe); 3,85 (m, 1H, H-8); 4,54 u. 4,64 (2x d, J=6 Hz, je 1H, $OCH_2O$)

**82) [1*S*-(1α,3aβ,4α,7aα)]-4-(Methoxymethoxy)-7a-methyl-β-methylenoctahydro-1H-inden-1-ethanol** **83**

Man löst 12,1 g (47 mmol) **82** und 19,03 g (94 mmol) Aluminiumisopropylat in 400 ml Toluol und erhitzt für 3 Std. zum Sieden. Nach dem Abkühlen wird mit Isopropanol/Wasser gerührt und anschließend durch Filtration von den ausgefallenen Aluminiumsalzen abgetrennt. Man entfernt das Solvens und reinigt den Rückstand durch Chromatographie an Kieselgel mit Hexan/Essigester, wobei man 11,5 g der Titelverbindung als farbloses Öl erhält.

$^1$H-NMR ($CDCl_3$): δ= 0,80 ppm (s, 3H, H-18); 3,37 (s, 3H, OMe); 3,89 (m, 1H, H-8); 4,00 u. 4,10 (dbr, J=14 Hz, je 1H, H-22); 4,56 u. 4,65 (2x d, J=6 Hz, je 1H, $OCH_2O$); 4,94 u. 5,21 (2x s, je 1H, H-21)

**83) [1*S*-(1α,3aβ,4α,7aα)]-2-[4-(Methoxymethoxy)-7a-methyloctahydro-1H-inden-1-yl]-2-propenal** **84**

Man setzt 12,0 g (47 mmol) **83** analog **55)** um, wobei 7,87 g der Titelverbindung als farbloses Öl anfallen.

$^1$H-NMR ($CDCl_3$): δ= 0,76 ppm (s, 3H, H-18); 2,78 (t, J=9,5 Hz, H-17); 3,37 (s, 3H, OMe); 3,90 (m, 1H, H-8); 4,55 u. 4,66 (2x d, J=6 Hz, je 1H, $OCH_2O$); 6,12 u. 6,28 (2x s, je 1H, H-21); 9,54 (s, 1H, H-22)

**84) [1*R*-[1α(*E*),3aβ,4α,7aα]]-N-Methoxy-4-[4-(methoxymethoxy)-7a-methyloctahydro-1H-inden-1-yl]-N-methyl-2,4-pentadienamid** **85**

Man setzt 6,6 g (31,06 mmol) **84** mit 25,5 g (71,6 mmol) N-Methoxy-N-methyl-(2-triphenylphosphoranyliden)acetamid analog **39)** um und erhält 6,8 g der Titelverbindung als farbloses Öl.

$^1$H-NMR($CDCl_3$): δ= 0,70 ppm (s, 3H, H-18); 2,41 (t, J=10 Hz, 1H, H-17); 3,20 (s, 3H, N-Me); 3,30 (s, 3H, OMe); 3,64 (s, 3H, N-OMe); 3,84 (m, 1H, H-8); 4,49 u. 4,60 (2x d, J=6 Hz, je 1H, $OCH_2O$); 5,22 u. 5,51 (2x s, je 1H, H-21); 6,53 (d, J=15 Hz, 1H, H-23); 7,30 (d, J=15 Hz, 1H, H-22)

**85) [1*R*-[1α(*E*),3aβ,4α,7aα]]-4-(4-Hydroxy-7a-methyloctahydro-1H-inden-1-yl)-N-methoxy-N-methyl-2,4-pentadienamid** **86**

Man setzt 1,75 g (5,19 mmol) **85** analog **68)** um, wobei man 806 mg der Titelverbindung als farbloses Öl erhält.

$^1$H-NMR ($CDCl_3$): δ= 0,75 ppm (s, 3H, H-18); 2,42 (t, J=10 Hz, 1H, H-17); 3,20 (s, 3H, N-Me); 3,65 (s, 3H, N-OMe); 4,05 (m, 1H, H-8); 5,20 u. 5,52 (s, je 1H, H-21); 6,53 (d, J=15 Hz, 1H, H-23); 7,30 (d, J=15 Hz, 1H, H-22)

**86) [1*R*-[1α(*E*),3aβ,7aα]]-N-Methoxy-N-methyl-4-(7a-methyl-4-oxooctahydro-4H-inden-1-yl)-2,4-pentadienamid** **87**

Man setzt 800 mg **86** analog **69)** um und erhält 640 mg der Titelverbindung als farbloses Öl.

$^1$H-NMR ($CDCl_3$): δ= 0,52 ppm (s, 3H, H-18); 2,67 (dd, J=10,5, 7,5 Hz, 1H, H-14); 2,80 (t, J=10,5 Hz, 1H, H-17); 3,29 (s, 3H, N-Me); 3,74 (s, 3H, N-OMC); 5,32 u. 5,60 (2x s, je 1H, H-21); 6,65 (d, J=15 Hz, 1H, H-23); 7,38 (d, J=15 Hz, 1H, H-22)

**87) (7*E*,22*E*)-(1*R*,3*R*)-1,3-Bis[[dimethyl(1,1-dimethylethyl)silyl]oxy]-N-methyl-N-methoxy-19-nor-9,10-secochola-5,7,20,22-tetraen-24-amid** **88**

Man setzt 640 mg (2,2 mmol) **87** analog **70)** um und erhält 275 mg der Titelverbindung als farblosen Schaum.

$^1$H-NMR ($CDCl_3$): δ= 0,05 ppm (s, 12H, SiMe); 0,43 (s, 3H, H-18); 0,88 u. 0,89 (2x s, je 9H, Si-t-butyl); 3,28 (s, 3H, N-Me); 3,73 (s, 3H, N-OMe); 4,08 (m, 2H, H-1 u. H-3); 5,33 u. 5,55 (2x s, je 1H, H-21); 5,85 u. 6,18 (2x d, J=11 Hz, je 1H, H-6 u. H-7); 6,63 (d, J=15 Hz, 1H, H-23); 7,39 (d, J=15 Hz, 1H, H-22)

**88) (7*E*,22*E*)-(1*R*,3*R*)-1,3-Bis[[dimethyl(1,1-dimethylethyl)silyl]oxy]-19-nor-9,10-seco-chola-5,7,20,22-tetraen-24-al** **89**

Man setzt 265 mg (0,41 mmol) **88** mit 1,03 mmol DIBAH-Lösung analog **40)** um, wobei man 220 mg der Titelverbindung als farbiosen Schaum erhält.

$^1$H-NMR ($CDCl_3$): δ= 0,04 ppm (s, 12H, SiMe); 0,43 (s, 3H, H-18); 0,87 (s, 18H, Si-t-butyl); 4,09 (m, 2H, H-1 u. H-3); 5,51 u. 5,68 (2x s, je 1H, H-21); 5,85 u. 6,15 (2x d, J=11 Hz, je 1H, H-6 u. H-7); 6,34 (dd, J=15, 7,5 Hz, 1H, H-23); 7,18 (d, J=15 Hz, 1H, H-22); 9,58 (d, J=7,5 Hz, 1H, H-24)

**Beispiel 31**

**89) (7*E*,22*E*)-(1*R*,3*R*,24*R*)-1,3-Bis[[dimethyl(1,1-dimethylethyl)silyl]oxyl-24-hydroxy-19-nor-9,10-secocholesta-5,7,20,22-tetraen-25-carbonsaure-1-methylethylester <u>90a</u> und (7*E*,22*E*)-(1*R*,3*R*,24*S*)-1,3-Bis[[dimethyl(1,1-dimethylethyl)silyl)oxy]-24-hydroxy-19-nor 9,10-secocholesta-5,7,20,22-tetraen-25-carbonsäure-1-methylethylester <u>90b</u>**

Analog **41)** werden 305 mg (0,52 mmol) **<u>89</u>** mit 2,1 mmol LDA und 0,28 ml (2,07 mmol) Isobuttersäureisopropylester umgesetzt, wobei nach Aufreinigung über HPLC (Normalphase, Laufmittel: Methylenchlorid/Methanol) 64 mg **<u>90a</u>** sowie 67 mg **<u>90b</u>** als farblose Schäume anfallen.

$^1$H-NMR ($CD_2Cl_2$): **<u>90a:</u>** δ= 0,05 ppm (s, 12H, SiMe); 0,41 (s, 3H, H-18); 0,85 u. 0,86 (2x s, je 9H, Si-t-butyl); 1,14 u. 1,15 (2x s, je 3H, H-26 u. H-27); 1,22 (d, J=7 Hz, 6H, COOiPr); 2,61 (d, J=6 Hz, 1H, OH); 4,07 (m, 2H, H-1 u. H-3); 4,13 (dd, J=7, 6 Hz, 1H, H-24); 4,98 (hept, J=7 Hz, 1H, COOiPr); 5,01 u. 5,20 (2x s, je 1 H, H-21); 5,84 u. 6,16 (2x d, J= 11 Hz, je 1H, H-6 u. H-7); 5,76 (dd, J=15, 7 Hz, 1H, H-23); 6,21 (d, J=15 Hz, 1H, H-22)

$^1$H-NMR ($CD_2Cl_2$): **<u>90b:</u>** δ= 0,05 ppm (s, 12H, SiMe); 0,41 (s, 3H, H-18); 0,86 (s, 18H, Si-t-butyl); 1,14 u. 1,16 (2x s, je 3H, H-26 u. H-27); 1,20 (d, J=7 Hz, 6H, COOiPr); 2,62 (d, J=7 Hz, 1H, OH); 4,08 (m, 2H, H-1 u. H-3); 4,18 (t, J=7 Hz, 1H, H-24); 4,98 (hept, J=7 Hz, 1H, COOiPr); 4,99 u. 5,19 (2x s, je 1 H, H-21); 5,84 u. 6,17 (2x d, J= 11 Hz, je 1H, H-6 u. H-7); 5,80 (dd, J=15, 7 Hz, 1H, H-23); 6,26 (d, J=15 Hz, 1H, H-22)

**90) (7*E*,22*E*)-(1*R*,3*R*,24*R*)-1,3,24-Trihydroxy-19-nor-9,10-secocholesta-5,7,20,22-tetraen-25-carbonsäure-1-methylethylester <u>91a</u>**

Man setzt 64 mg (0,09 mmol) **<u>90a</u>** analog **73)** um, wobei man 22 mg der Titelverbindung als farblosen Schaum erhält.

$^1$H-NMR ($CD_2Cl_2$): δ= 0,42 ppm (s, 3H, H-18); 1,14 u. 1,15 (2x s, je 3H, H-26 u. H-27); 1,22 (d, J=7 Hz, 6H, COOiPr); 3,98 u. 4,08 (2x m, je 1H, H-1 u. H-3); 4,15 (d, J=7 Hz, 1H, H-24); 4,98 (hept, J=7 Hz, 1H, COOiPr); 5,00 u. 5,20 (2x s, je 1 H, H-21); 5,88 u. 6,28 (2x d, J= 11 Hz, je 1H, H-6 u. H-7); 5,79 (dd, J=15, 7 Hz, 1H, H-23); 6,26 (d, J=15 Hz, 1H, H-22)

**91) (7*E*,22*E*)-(1*R*,3*R*,24*S*)-1,3,24-Trihydroxy-19-nor-9,10-secocholesta-5,7,20,22-tetraen-25-carbonsäure-1-methylethylester <u>91b</u>**

Man setzt 67 mg (0,09 mmol) **<u>90b</u>** analog **73)** um, wobei man 27 mg der Titelverbindung als farblosen Schaum erhält.

$^1$H-NMR ($CD_2Cl_2$): δ= 0,42 ppm (s, 3H, H-18); 1,14 u. 1,17 (2x s, je 3H, H-26 u. H-27); 1,22 (d, J=7 Hz, 6H, COOiPr); 3,98 u. 4,07 (2x m, je 1H, H-1 u. H-3); 4,18 (t, J=7 Hz, 1H, H-24); 4,99 (hept, J=7 Hz, 1H, COOiPr); 5,00 u. 5,20 (2x s, je 1 H, H-21); 5,88 u. 6,28 (2x d, J= 11 Hz, je 1H, H-6 u. H-7); 5,80 (dd, J=15, 7 Hz, 1H, H-23); 6,28 (d, J=15 Hz, 1H, H-22)

**Beispiel 32**

**92) (7*E*,22*E*)-(1*R*,3*R*,24*R*)-1,3-Bis[[dimethyl(1,1-dimethylethyl)silyl]oxy]-24-hydroxy-19-nor-9,10-secocholesta-5,7,20,22-tetraen-25-carbonsäureethylester <u>92a</u> und (7*E*,22*E*)-(1*R*,3*R*,24*S*)-1,3-Bis[[dimethyl(1,1-dimethylethyl)silyl]oxy]-24-hydroxy-19-nor-9,10-secocholesta-5,7,20,22-tetraen-25-carbonsäureethylester <u>92b</u>**

Analog **41)** werden 300 mg (0,51 mmol) **<u>89</u>** mit 2 mmol LDA und 0,26 ml (1,9 mmol) Isobuttersäureethylester umgesetzt, wobei nach Aufreinigung über HPLC (Normalphase, Laufmittel: Methylenchlorid/Methanol) 68 mg **<u>92a</u>** sowie 85 mg **<u>92b</u>** als farblose Schäume anfallen.

$^1$H-NMR ($CD_2Cl_2$): **<u>92a:</u>** δ= 0,04 ppm (s, 12H, SiMe); 0,40 (s, 3H, H-18); 0,84 u. 0,86 (2x s, je 9H, Si-t-butyl); 1,13 u. 1,14 (2x s, je 3H, H-26 u. H-27); 1,27 (t, J=7 Hz, 3H, COOEt); 2,59 (d, J=6 Hz, 1H, OH); 4,05 (m, 2H, H-1 u. H-3); 4,11 (dd, J=7, 6 Hz, 1H, H-24); 4,12 (q, J=7 Hz, 2H, COOEt); 5,01 u. 5,19 (2x s, je 1 H, H-21); 5,84 u. 6,16 (2x d, J= 11 Hz, je 1H, H-6 u. H-7); 5,75 (dd, J=15, 7 Hz, 1H, H-23); 6,20 (d, J=15 Hz, 1H, H-22)

$^1$H-NMR ($CD_2Cl_2$): **92b:** δ= 0,04 ppm (s, 12H, SiMe); 0,40 (s, 3H; H-18); 0,85 (s, 18H, Si-t-butyl); 1,13 u. 1,15 (2x s, je 3H, H-26 u. H-27); 1,28 (t, J=7 Hz, 3H, COOEt); 2,60 (d, J=7 Hz, 1H, OH); 4,06 (m, 2H, H-1 u. H-3); 4,12 (t, J=7 Hz, 2H, COOEt); 4,14 (q, J=7 Hz, 2H, COOEt); 4,99 u. 5,18 (2x s, je 1 H, H-21); 5,84 u. 6,16 (2x d, J= 11 Hz, je 1H, H-6 u. H-7); 5,80 (dd, J=15, 7 Hz, 1H, H-23); 6,25 (d, J=15 Hz, 1H, H-22)

**93) (7*E*,22*E*)-(1*R*,3*R*,24*R*)-1,3,24-Trihydroxy-19-nor-9,10-secocholesta-5,7,20,22-tetraen-25-carbonsäureethylester 93a**

Man setzt 65 mg (0,093 mmol) **93a** analog **73)** um, wobei man 23 mg der Titelverbindung als farblosen Schaum erhält.

$^1$H-NMR ($CD_2Cl_2$): δ= 0,41 ppm (s, 3H, H-18); 1,13 u. 1,15 (2x s, je 3H, H-26 u. H-27); 1,26 (t, J=7 Hz, 3H, COOEt); 3,98 u. 4,07 (2x m, je 1H, H-1 u. H-3); 4,14 (q, J=7 Hz, 2H, COOEt); 4,15 (d, J=7 Hz, 1H, H-24); 5,00 u. 5,20 (2x s, je 1 H, H-21); 5,87 u. 6,28 (2x d, J= 11 Hz, je 1H, H-6 u. H-7); 5,78 (dd, J=15, 7 Hz, 1H, H-23); 6,25 (d, J=15 Hz, 1H, H-22)

**94) (7*E*,22*E*)-(1*R*,3*R*,24*S*)-1,3,24-Trihydroxy-19-nor-9,10-secocholesta-5,7,20,22-tetraen-25-carbonsäureethylester 93b**

Man setzt 80 mg (0,14 mmol) **92b** analog **73)** um, wobei man 31 mg der Titelverbindung als farblosen Schaum erhält.

$^1$H-NMR ($CD_2Cl_2$): δ= 0,41 ppm (s, 3H, H-18); 1,14 u. 1,16 (2x s, je 3H, H-26 u. H-27); 1,28 (t, J=7 Hz, 3H, COOEt); 3,98 u. 4,06 (2x m, je 1H, H-1 u. H-3); 4,15 (q, J=7 Hz, 2H, COOEt); 4,17 (t, J=7 Hz, 1H, H-24); 5,00 u. 5,19 (2x s, je 1 H, H-21); 5,87 u. 6,28 (2x d, J= 11 Hz, je 1H, H-6 u. H-7); 5,80 (dd, J=15, 7 Hz, 1H, H-23); 6,28 (d, J=15 Hz, 1H, H-22)

**Ausgangsmaterialien in der 20-Fluor-19-nor-Reihe**

**95) [1*S*-(1α,3aβ,4α,7aα)]-4[[Dimethyl(1,1-dimethylethyl)silyl]oxy]-7a-methyl-1-[1-methyl-2-[(trimethylsilyl)oxy]-1-ethenyl]octahydro-1H-inden 95**

Zu 37,7 g (170 mmol) Trimethylsilyltrifluormethansulfonat in 270 ml Methylenchlorid werden bei Raumtemperatur 21,9 g (170 mmol) Diisopropylethylamin und bei 5°C 9 g (27 mmol) [1*R*-[1α(*S**),3aβ,4α,7aα]]-α,7a-Dimethyl-4[[dimethyl(1,1-dimethylethyl)silyl]oxy]-octahydro-1H-inden-1-acetaldehyd **94** [H.H. Inhoffen et al. Chem. Ber. **91,** 780 (1958), Chem. Ber. **92,** 1772 (1959), W.G. Dauben et al. Tetrahedron Lett. **30,** 677 (1989)] in 600 ml Methylenchlorid getropft. Man rührt eine Std. bei Raumtemperatur nach und entfernt das Solvens anschließend im Vakuum. Der Rückstand wird in Hexan aufgenommen, über Natriumsulfat filtriert und erneut vom Solvens befreit, wobei 13 g der Titelverbindung als farbloses Öl anfallen (*E/Z*-Gemisch ca. 3:1).

$^1$H-NMR ($CD_2Cl_2$): δ = 0,00 ppm (s, 6H, SiMe); 0,13 (s, 9H, $SiMe_3$); 0,78 (s, 3H, H-18); 0,88 (s, 9H, Si-t-butyl); 1,50/1,57 (sbr, 3H, H-21); 4,02 (m, 1H, H-8); 6,02/6,18 (m; 1H, H-22)

**96) [1*S*-[1α(*S**),3aβ,4α,7aα]]-α,7a-Dimethyl-4-[[dimethyl(1,1-dimethylethyl)silyl]oxy]-α-fluoroctahydro-1H-inden-1-acetaldehyd 96 und**
**[1*S*-[1α(*R**),3aβ,4α,7aα]]-α,7a-Dimethyl-4-[[dimethyl(1,1-dimethylethyl)silyl]oxy]-α-fluoroctahydro-1H-inden-1-acetaldehyd 97**

Man löst 15 g (37,81 mmol) **95** in 300 ml Methylenchlorid und tropft bei 5°C 26 g (82 mmol) N-Fluordiphenylsulfonimid in 250 ml Methylenchlorid hinzu. Es wird über Nacht bei Raumtemperatur gerührt. Danach gießt man das Reaktionsgemisch in Wasser, trennt die organische Phase ab, trocknet über Natriumsulfat, engt ein und chromatographiert den Rückstand an Kieselgel mit Hexan/Essigester, wobei 3,85 g **96** und 2,08 g **97** als farblose Öle anfallen.

$^1$H-NMR ($CDCl_3$): **96:** δ= 0,00 u. 0,01 ppm (2x s, je 3H, SiMe); 0,88 (s, 9H, Si-t-butyl); 1,02 (d, J=2 Hz, 3H, H-18); 1,42 (d, J=21 Hz, 3H, H-21); 4,01 (m, 1H, H-8); 9,75 (d, J=7 Hz, 1H, H-22)
**97:** δ= 0,01 ppm (s, 6H, SiMe); 0,88 (s, 9H, Si-t-butyl); 1,08 (d, J=4,5 Hz, 3H, H-18); 1,46 (d, J=21 Hz, 3H, H-21); 4,01 (m, 1H, H-8); 9,87 (d, J=6 Hz, 1H, H-22)

**Ausgangsmaterialien in der 20-β-Fluor-Reihe**

**97) [1*S*-[1α[*R**-(*E*)],3aβ,4α,7aα]]-4-[4-[[Dimethyl(1,1-dimethylethyl)silyl]oxy]-7a-methyl-octahydro-1H-inden-1-yl]-4-fluor-N-methoxy-N-methyl-2-pentenamid 98**

Man setzt 342 mg (1 mmol) **96** analog **39)** um und erhält 380 mg der Titelverbindung als farbloses Öl.

$^1$H-NMR ($CDCl_3$): δ= 0,00 u. 0,01 ppm (2x s, je 3H, SiMe); 0,88 (s, 9H, Si-t-butyl); 1,05 (d, J=2 Hz, 3H, H-18); 1,50 (d, J=21 Hz, 3H, H-21); 3,27 (s, 3H, N-Me); 3,70 (s, 3H, N-OMe); 4,01 (m, 1H, H-8); 6,55 (d, J= 15,5 Hz, 1H, H-23); 6,93 (dd, 3=21, 15,5 Hz, 1H, H-22)

**98) [1*S*-[1α[*R**-(*E*)],3aβ,4α,7aα]]-4-Fluor-4-(4-hydroxy-7a-methyloctahydro-1H-inden-1-yl)-N-methoxy-N-methyl-2-pentenamid 99**

Man rührt 100 mg (0,23 mmol) **98** in 1,9 ml Acetonitril und 1,5 ml THF mit 1,4 ml Fluorwasserstoffsäure (40%) für 90 Min. Anschließend gießt man auf Natriumchlorid-Lösung, extrahiert mit Essigester, wäscht die organische Phase mit Natriumhydrogencarbonat- und Natriumchlorid-Lösung, trocknet über Natriumsulfat und engt ein. Der Rückstand wird chromatographisch an Kieselgel mit Hexan/Essigester gereinigt, wobei 53 mg der Titelverbindung als farbloses Öl anfallen.

$^1$H-NMR ($CDCl_3$): δ= 1,08 ppm (d, J=2 Hz, 3H, H-18); 1,50 (d, J=21 Hz, 3H, H-21); 3,25 (s, 3H, N-Me); 3,68 (s, 3H, N-OMe); 4,07 (m, 1H, H-8); 6,54 (d, J= 15,5 Hz, 1H, H-23); 6,89 (dd, J=21, 15,5 Hz, 1H, H-22)

**99) [1*S*-[1α[*R**-(*E*)],3aβ,7aα]]-4-Fluor-N-methoxy-N-methyl-4-(7a-methyl-4-oxo-octahydro-4H-inden-1-yl)-2-pentenamid 100**

Man löst 427 mg (1,36 mmol) **99** in 12 ml Methylenchlorid, gibt 63 mg (0,76 mmol) Natriumacetat zu und kühlt auf 0°C. Anschließend versetzt man mit 414 mg (1,92 mmol) Pyridiniumchlorochromat und rührt 3 Std. bei Raumtenperatur nach. Nach Verdünnung mit Diethylether wird über Celite filtriert, eingeengt und an Kieselgel mit Hexan/Essigester chromatographiert, wobei man 357 mg der Titelverbindung erhält.

$^1$H-NMR ($CDCl_3$): δ= 0,77 ppm (d, J=2 Hz, 3H, H-18); 1,53 (d, J=21 Hz, 3H, H-21); 2,46 (dd, J=10,5, 7,5 Hz, 1H, H-14); 3,26 (s, 3H, N-Me); 3,72 (s, 3H, N-OMe); 6,56 (d, J= 15,5 Hz, 1H, H-23); 6,93 (dd, J=21, 15,5 Hz, 1H, H-22)

**100) (7*E*,22*E*)-(1*R*,3*R*,20*S*)-1,3-Bis[[dimethyl(1,1-dimethylethyl)silyl]oxy]-20-fluor-N-methoxy-N-methyl-19-nor-9,10-secochola-5,7,22-trien-24-amid 101**

Man setzt 350 mg (1,15 mmol) **100** analog **70)** um und erhält 460 mg der Titelverbindung als farblosen Schaum.

$^1$H-NMR ($CDCl_3$): δ= 0,03 ppm (s, 12H, SiMe); 0,62 (d, J=2 Hz, 3H, H-18); 0,81 u. 0,82 (2x s, je 9H, Si-t-butyl); 1,50 (d, J=21 Hz, 3H, H-21); 3,22 (s, 3H, N-Me); 3,68 (s, 3H, N-OMe); 4,03 (m, 2H, H-1 u. H-3); 5,77 u. 6,11 (2x d, J=11 Hz, je 1H, H-6 u. H-7); 6,53 (d, J=15,5 Hz, 1H, H-23); 6,90 (dd, J=21, 15,5 Hz, 1H, H-22)

**101) (7*E*,22*E*)-(1*R*,3*R*,20*S*)-1,3-Bis[[dimethyl(1,1-dimethylethyl)silyl]oxy]-20-fluor-19-nor-9,10-secochola-5,7,22-trien-24-al 102**

Man setzt 440 mg (0,66 mmol) **101** mit 3,3 mmol DIBAH-Lösung analog **3**, um und erhält 340 mg der Titelverbindung als farblosen Schaum.

$^1$H-NMR ($CDCl_3$): δ= 0,02 ppm (s, 12H, SiMe); 0,62 (d, J=2 Hz, 3H, H-18); 0,83 u. 0,84 (2x s, je 9H, Si-t-butyl); 1,52 (d, J=21 Hz, 3H, H-21); 4,03 (m, 2H, H-1 u. H-3); 5,78 u. 6,11 (2x d, J=11 Hz, je 1H, H-6 u. H-7); 6,22 (dd, J=15,5, 8 Hz, 1H, H-23); 6,78 (dd, J=21, 15,5 Hz, 1H, H-22); 9,54 (dbr, J=8 Hz, 1H, H-24)

**Beispiel 33**

**102) (7*E*,22*E*)-(1*R*,3*R*,20*S*)-1,3-Bis[[dimethyl(1,1-dimethylethyl)silyl]oxy]-20-fluor-24-hydroxy-19-nor-9,10-secocholesta-5,7,22-trien-25-carbonsäureethylester 103**

Man setzt 700 mg (1,15 mmol) **102** analog **41)** mit 7,8 mmol LDA und 1,2 ml (7,8 mmol) Isobuttersäureethylester um und erhält 200 mg der Titelverbindung als farblosen Schaum (1:1 Diastereomere bzgl. C-24).

$^1$H-NMR ($CDCl_3$): δ= 0,02 ppm (s, 12H, SiMe); 0,62 (d, J=2 Hz, 3H, H-18); 0,83 u. 0,84 (2x s, je 9H, Si-t-butyl); 1,12/1,16 u. 1,22 (2x s, je 3H, H-26 u. H-27); 1,22 (t, J=7 Hz, 3H, COOEt); 1,47 (d, J=21 Hz, 3H, H-21); 4,04 (m, 2H, H-1 u. H-3); 4,13 (q, J= 7 Hz, 2H, COOEt); 4,13 (m, 1H, H-24); 5,61 (dd, J=15,5, 7 Hz, 1H, H-23); 5,75/5,78 (dd; J=21, 15,5 Hz, 1H, H-22); 5,78 u. 6,12 (2x d, J=11 Hz, je 1H, H-6 u. H-7)

**103) (7*E*,22*E*)-(1*R*,3*R*,20*S*)-20-Fluor-1,3,24-trihydroxy-19-nor-9,10-secocholesta-5,7,22-trien-25-carbonsäureethylester 104**

Man rührt 150 mg (0,2 mmol) **103** in 20 ml THF mit 630 mg (2 mmol) Tetrabutylammoniumfluorid über Nacht bei

Raumtemperatur und erhitzt anschließend für 30 Min. auf 40°C. Nach dem Abkühlen gibt man Natriumchlorid-Lösung zu, extrahiert mit Essigester, wäscht mit Natriumchlorid-Lösung, trocknet über Natriumsulfat und engt ein. Der Rückstand wird mehrmals an Kieselgel mit Hexan/Essigester chromatographiert, wobei 17,3 mg der Titelverbindung als farbloser Schaum anfallen (1:1-Diastereomere bzgl. C-24).

$^1$H-NMR ($CD_2Cl_2$): δ= 0,60 ppm (d, J=2 Hz, 3H, H-18); 1,07 u. 1,09/1,12 (2x s, je 3H, H-26 u. H-27); 1,18 (t, J=7 Hz, 3H, COOEt); 1,40 (d, J=21 Hz, 3H, H-21); 3,99 (m, 2H, H-1 u. H-3); 4,05 (q, J= 7 Hz, 2H, COOEt); 4,08 (m, 1H, H-24); 5,55/5,56 (dd, J=15,5, 7 Hz, 1H, H-23); 5,71/5,72 (dd, J=21, 15,5 Hz, 1H, H-22); 5,78 u. 6,20 (2x d, J=11 Hz, je 1H, H-6 u. H-7)

**Ausgangsmaterialien in der 20-α-Fluor-Reihe**

**104) [1*S*-[1α[*S**-(*E*)],3aβ,4α,7aα]]-4-[4-[[Dimethyl(1,1-dimethylethyl)silyl]oxy]-7a-methyl-octahydro-1H-inden-1-yl]-4-fluor-N-methoxy-N-methyl-2-pentenamid** <u>**105**</u>

Man setzt 2,03 g (5,9 mmol) <u>**97**</u> analog **39)** um und erhält 2,4 g der Titelverbindung als farbloses Öl.

$^1$H-NMR ($CDCl_3$): δ= 0,02 ppm (s, 6H, SiMe); 0,88 (s, 9H, Si-t-butyl); 1,00 (d, J=4,5 Hz, 3H, H-18); 1,40 (d, J=21 Hz, 3H, H-21); 3,29 (s, 3H, N-Me); 3,72 (s, 3H, N-OMe); 4,01 (m, 1H, H-8); 6,62 (d, J= 15,5 Hz, 1H, H-23); 6,99 (dd, J=25, 15,5 Hz, 1H, H-22)

**105) [1*S*-[1α[*S**-(*E*)],3aβ,4α,7aα]]-4-Fluor-4-(4-hydroxy-7a-methyloctahydro-1H-inden-1-yl)-N-methoxy-N-methyl-2-pentenamid** <u>**106**</u>

Man setzt 2,4 g (5,6 mmol) <u>**105**</u> analog **98)** um und erhält 1 g der Titelverbindung als farbloses Öl.

$^1$H-NMR ($CDCl_3$): δ= 0,97 ppm (d, J=4,5 Hz, 3H, H-18); 1,40 (d, J=21 Hz, 3H, H-21); 3,25 (s, 3H, N-Me); 3,72 (s, 3H, N-OMe); 4,02 (m, 1H, H-8); 6,57 (d, J= 15,5 Hz, 1H, H-23); 6,92 (dd, J=25, 15,5 Hz, 1H, H-22)

**106) [1*S*-[1α[*S**-(*E*)],3aβ,7aα]]-4-Fluor-N-methoxy-N-methyl-4-(7a-methyl-4-oxo-octahydro-4H-inden-1-yl)-2-pentenamid** <u>**107**</u>

Man setzt 1 g (3,19 mmol) <u>**106**</u> analog **99)** um und erhält 800 mg der Titelverbindung als farbloses Öl.

$^1$H-NMR ($CDCl_3$): δ= 0,70 ppm (d, J=4,5 Hz, 3H, H-18); 1,41 (d, J=21 Hz, 3H, H-21); 2,44 (dd, J=10,5, 7,5 Hz, 1H, H-14); 3,26 (s, 3H, N-Me); 3,73 (s, 3H, N-OMe); 6,62 (d, J= 15,5 Hz, 1H, H-23); 6,98 (dd, J=25, 15,5 Hz, 1H, H-22)

**107) (7*E*,22*E*)-(1*R*,3*R*,20*R*)-1,3-Bis[[dimethyl(1,1-dimethylethyl)silyl]oxy]-20-fluor-N-methoxy-N-methyl-19-nor-9,10-secochola-5,7,22-trien-24-amid** <u>**108**</u>

Man setzt 600 mg (2,5 mmol) <u>**107**</u> analog **70)** um und erhält 470 mg der Titelverbindung als farblosen Schaum.

$^1$H-NMR ($CDCl_3$): δ= 0,03 ppm (s, 12H, SiMe); 0,60 (d, J=4,5 Hz, 3H, H-18); 0,81 u. 0,82 (2x s, je 9H, Si-t-butyl); 1,45 (d, J=21 Hz, 3H, H-21); 3,24 (s, 3H, N-Me); 3,72 (s, 3H, N-OMe); 4,03 (m, 2H, H-1 u. H-3); 5,77 u. 6,12 (2x d, J=11 Hz, je 1H, H-6 u. H-7); 6,57 (d, J=15,5 Hz, 1H, H-23); 6,98 (dd, J=25, 15,5 Hz, 1H, H-22)

**108) (7*E*,22*E*)-(1*R*,3*R*,20*R*)-1,3-Bis[[dimethyl(1,1-dimethylethyl)silyl]oxy]-20-fluor-19-nor-9,10-secochola-5,7,22-trien-24-al** <u>**109**</u>

Man setzt 440 mg (0,66 mmol) <u>**108**</u> mit 3,3 mmol DIBAH-Lösung analog **40)** um und erhält 294 mg der Titelverbindung als farblosen Schaum.

$^1$H-NMR ($CDCl_3$): δ= 0,02 ppm (s, 12H, SiMe); 0,58 (d, J=4,5 Hz, 3H, H-18); 0,82 u. 0,83 (2x s, je 9H, Si-t-butyl); 1,45 (d, J=21 Hz, 3H, H-21); 4,03 (m, 2H, H-1 u. H-3); 5,78 u. 6,11 (2x d, J=11 Hz, je 1H, H-6 u. H-7); 6,29 (dd, J=15,5, 8 Hz, 1H, H-23); 6,84 (dd, J=25, 15,5 Hz, 1H, H-22); 9,62 (dbr, J=8 Hz, 1H, H-24)

**Beispiel 34**

**109) (7*E*,22*E*)-(1*R*,3*R*,20*R*,24*S*)-1,3-Bis[[dimethyl(1,1-dimethylethyl)silyl]oxy]-20-fluor-24-hydroxy-19-nor-9,10-secocholesta-5,7,22-trien-25-carbonsäureethylester** <u>**110**</u>

Man setzt 240 mg (0,39 mmol) <u>**109**</u> analog **41)** mit 3,9 mmol LDA und 0,52 ml (3,9 mmol) Isobuttersäureethylester um und erhält 160 mg der Titelverbindung als farblosen Schaum.

$^1$H-NMR ($CD_2Cl_2$): δ= 0,01 ppm (s, 12H, SiMe); 0,55 (d, J=4,5 Hz, 3H, H-18); 0,82 u. 0,83 (2x s, je 9H, Si-t-butyl);

1,11 u. 1,13 (2x s, je 3H, H-26 u. H-27); 1,22 (t, J=7 Hz, 3H, COOEt); 1,46 (d, J=21 Hz, 3H, H-21); 4,02 (m, 2H, H-1 u. H-3); 4,08 (q, J= 7 Hz, 2H, COOEt); 4,13 (m, 1H, H-24); 5,63 (dd, J=15,5, 7 Hz, 1H, H-23); 5,83 (dd, J=25, 15,5 Hz, 1H, H-22); 5,79 u. 6,13 (2x d, J=11 Hz, je 1H, H-6 u. H-7)

**110) (7*E*,22*E*)-(1*R*,3*R*,20*R*,24*S*)-20-Fluor-1,3,24-trihydroxy-19-nor-9,10-secocholesta-5,7,22-trien-25-carbonsäureethylester 111**

Man setzt 150 mg (0,2 mmol) **110** analog **103)** um und erhält 25 mg der Titelverbindung als farblosen Schaum.

$^1$H-NMR ($CD_2Cl_2$): δ= 0,60 ppm (d, J=4,5 Hz, 3H, H-18); 1,15 u. 1,18 (2x s, je 3H, H-26 u. H-27); 1,23 (t, J=7 Hz, 3H, COOEt); 1,35 (d, J=21 Hz, 3H, H-21); 3,99 u. 4,08 (2x m, je 1H, H-1 u. H-3); 4,12 (q, J= 7 Hz, 2H, COOEt); 4,18 (d, J=7 Hz, 1H, H-24); 5,68 (dd, J=15,5, 7 Hz, 1H, H-23); 5,88 (dd, J=25, 15,5 Hz, 1H, H-22); 5,83 u. 6,28 (2x d, J=11 Hz, je 1H, H-6 u. H-7)

**Derivate in der Normalreihe (Ergänzung zu EM 50741)**

**Beispiel 35**

**111) (5*E*,7*E*,22*E*)-(1*S*,3*R*)-1,3-Bis[[dimethyl(1,1-dimethylethyl)silyl]oxy]-24-hydroxy-9,10-secocholesta-5,7,10(19),22-tetraen-25-carbonsäurebutylester 112**

Man setzt 500 mg (0,91 mmol) (5*E*,7*E*,22*E*)-(1*S*,3*R*)-1,3-Bis[[dimethyl-(1,1-dimethylethyl)silyl]oxy]-9,10-secochola-5,7,10(19),22-trien-24-al **3** mit 4,5 mmol LDA und 0.76 ml (4,5 mmol) Isobuttersäurebutylester analog **41)** um und erhält 677 mg der Titelverbindung als farblosen Schaum (1:1 Diastereomere bzgl. C-24).

$^1$H-NMR ($CDCl_3$): δ= 0,04 ppm (s, 12H, SiMe); 0,53 (s, 3H, H-18); 0,88 u. 0,90 (2x s, je 9H, Si-t-butyl); 0,99 (t, J=7 Hz, 3H, COOBu); 1,02 (d, J=7 Hz, 3H, H-21); 1,13/1,14 u. 1,15/1,16 (2x s, je 3H, C-26 u. C-27); 4,07 (t, J=7 Hz, 2H, COOBu); 4,09 (m, 1H, H-24); 4,19 (m, 1H, H-3); 4,51 (m, 1H, H-1); 4,92 u. 4,97 (2x s, je 1H, H-19); 5,36/5,38 (dd, J=15, 7 Hz, 1H, H-23); 5,52/5,57 (dd, J=15, 9 Hz, 1H, H-22); 5,80 u. 6,43 (2x d, J=11 Hz, je 1H, H-6 u. H-7)

**112) (5*Z*,7*E*,22*E*)-(1*S*,3*R*,24*R*)-1,3-Bis[[dimethyl(1,1-dimethylethyl)silyl]oxy]-24-hydroxy-9,10-secocholesta-5,7,10(19),22-tetraen-25-carbonsäurebutylester 113a und (5*Z*,7*E*,22*E*)-(1*S*,3*R*,24*S*)-1,3-Bis[[dimethyl(1,1-dimethylethyl)silyl]oxy]-24-hydroxy-9,10-secocholesta-5,7,10(19),22-tetraen-25-carbonsäurebutylester 113b**

Man löst 670 mg (0,90 mmol) **112** in 80 ml Toluol und bestrahlt die Lösung in Gegenwart von 180 mg (0,96 mmol) Anthracen sowie 0,1 ml Triethylamin in einem Pyrex-Tauchreaktor mit einer Quecksilberhochdrucklampe (Philips HPK 125) unter Stickstoff für 10 Min. Das Lösungsmittel wird entfernt und der Rückstand chromatographisch an Kieselgel mit Hexan/Essigester gereinigt, wobei 102 mg **113a** und 158 mg **113b** als farblose Schäume anfallen.

$^1$H-NMR ($CDCl_3$): **113a**: δ= 0,06 ppm (s, 12H, SiMe); 0,54 (s, 3H, H-18); 0,88 (s, 18H, Si-t-butyl); 0,96 (t, J=7 Hz, 3H, COOBu); 1,05 (d, J=7 Hz, 3H, H-21); 1,16 u, 1,17 (2x s, je 3H, H-26 u. H-27); 2,60 (d, J=6 Hz, 1H, OH); 4,06 (dd, J=7, 6 Hz, 1H, H-24); 4,10 (t, J=7 Hz, 2H, COOBu); 4,20 (m, 1H, H-3); 4,38 (m, 1H, H-1); 4,87 u. 5,18 (2x s, je 1H, H-19); 5,36 (dd, J=15, 7 Hz, 1H, H-23); 5,52 (dd, J=15, 9 Hz, 1H, H-22); 6,01 u. 6,23 (2x d, J=11 Hz, je 1H, H-6 u. H-7)

**113b:** δ= 0,06 ppm (s, 12H, SiMe); 0,53 (s, 3H, H-18); 0,88 (s, 18H, Si-t-butyl); 0,94 (t, J=7 Hz, 3H, COOBu); 1,03 (d, J=7 Hz, 3H, H-21); 1,17 u, 1,18 (2x s, je 3H, H-26 u. H-27); 2,58 (d, J=6 Hz, 1H, OH); 4,10 (dd, J=7, 6 Hz, 1H, H-24); 4,10 (t, J=7 Hz, 2H, COOBu); 4,18 (m, 1H, H-3); 4,37 (m, 1H, H-1); 4,87 u. 5,18 (2x s, je 1H, H-19); 5,38 (dd, J=15, 7 Hz, 1H, H-23); 5,59 (dd, J=15, 9 Hz, 1H, H-22); 6,01 u. 6,23 (2x d, J=11 Hz, je 1H, H-6 u. H-7)

**113) (5*Z*,7*E*,22*E*)-(1*S*,3*R*,24*R*)-1,3,24-Trihydroxy-9,10-secocholesta-5,7,10(19),22-tetraen-25-carbonsäurebutylester 114a**

Man setzt 100 mg (0,13 mmol) **113a** analog **73)** um und erhält 39 mg der Titelverbindung als farblosen Schaum.

$^1$H-NMR ($CD_2Cl_2$): δ= 0,57 ppm (s, 3H, H-18); 0,93 (t, J=7 Hz, 3H, COOBu); 1,04 (d, J=7 Hz, 3H, H-21); 1,12 (s, 3H, H-26 u. H-27); 2,45 (d, J=6 Hz, 1H, OH); 4,02 (dd, J=7, 6 Hz; 1H, H-24); 4,06 (t, J=7 Hz, 2H, COOBu); 4,17 (m, 1H, H-3); 4,38 (m, 1H, H-1); 4,95 u. 5,28 (2x s, je 1H, H-19); 5,35 (dd, J=15, 7 Hz, 1H, H-23); 5,51 (dd, J=15, 9 Hz, 1H, H-22); 6,01 u. 6,36 (2x d, J=11 Hz, je 1H, H-6 u. H-7)

**114) (5*Z*,7*E*,22*E*)-(1*S*,3*R*,24*S*)-1,3,24-Trihydroxy-9,10-secocholesta-5,7,10(19),22-tetraen-25-carbonsäurebutylester 114b**

Man setzt 155 mg (0,21 mmol) **113b** analog **73)** um und erhält 69 mg der Titelverbindung als farblosen Schaum.

$^1$H-NMR ($CD_2Cl_2$): δ= 0,56 ppm (s, 3H, H-18); 0,94 (t, J=7 Hz, 3H, COOBu); 1,03 (d, J=7 Hz, 3H, H-21); 1,12 (s, 3H, H-26 u. H-27); 2,46 (d, J=6 Hz, 1H, OH); 4,08 (m, 1H, H-24); 4,08 (t, J=7 Hz, 2H, COOBu); 4,17 (m, 1H, H-3); 4,39 (m, 1H, H-1); 4,96 u. 5,29 (2x s, je 1H, H-19); 5,37 (dd, J=15, 7 Hz, 1H, H-23); 5,57 (dd, J=15, 9 Hz, 1H, H-22); 6,01 u. 6,36 (2x d,J=11 Hz, je 1H, H-6 u.H-7)

**Beispiel 36**

**115) (5*E*,7*E*,22*E*)-(1*S*,3*R*)-1,3-Bis[[dimethyl(1,1-dimethylethyl)silyl]oxy]-24-hydroxy-9,10-secocholesta-5,7,10(19),22-tetraen-25-carbonsäure-2-methylpropylester 115**

Man setzt 500 mg (0,91 mmol) (5*E*,7*E*,22*E*)-(1*S*,3*R*)-1,3-Bis[[dimethyl-(1,1-dimethylethyl)silyl]oxy]-9,10-secochola-5,7,10(19),22-tri en-24-al **3** mit 4,5 mmol LDA und 0,76 ml (4,5 mmol) Isobuttersäureisobutylester analog **41)** um und erhält 400 mg der Titelverbindung als farblosen Schaum (1:1 Diastereomere bzgl. C-24).

$^1$H-NMR ($CDCl_3$): δ= 0,03 ppm (s, 12H, SiMe); 0,57 (s, 3H, H-18); 0,89 (s, 18H, Si-t-butyl); 0,90 u. 0,92 (2x d, J=7 Hz, je 6H, COOiBu); 1,04/1,05 (d, J=7 Hz, 3H, H-21); 1,17 u, 1,18 (2x s, je 3H, H-26 u. H-27); 3,88 (m, 2H, COOiBu); 4,10 (m, 1H, H-24); 4,23 (m, 1H, H-3); 4,53 (m, 1H, H-1); 4,94 u. 4,99 (2x s, je 1H, H-19); 5,37/5,39 (dd, J=15, 7 Hz, 1H, H-23); 5,53/5,59 (dd, J=15, 7 Hz, 1H, H-22); 5,81 u. 6,46 (2x d, J=11 Hz, je 1H, H-6 u. H-7)

**116) (5*Z*,7*E*,22*E*)-(1*S*,3*R*,24*R*)-1,3-Bis[[dimethyl(1,1-dimethylethyl)silyl]oxy]-24-hydroxy-9,10-secocholesta-5,7,10(19),22-tetraen-25-carbonsäure-2-methylpropylester 116a und (5*Z*,7*E*,22*E*)-(1*S*,3*R*,24*S*)-1,3-Bis[[dimethyl(1,1-dimethylethyl)silyl]oxy]-24-hydroxy-9,10-secocholesta-5,7,10(19),22-tetraen-25-carbonsäure-2-methylpropylester 116b**

Man setzt 400 mg (0,54 mmol) **115** analog **112)** um und erhält 102 mg **116a** sowie 200 mg **116b** als farblose Schäume.

$^1$H-NMR ($CDCl_3$): **116a:** δ= 0,05 ppm (s, 12H, SiMe); 0,55 (s, 3H, H-18); 0,89 (s, 18H, Si-t-butyl); 0,95 (d, J=7 Hz, 6H, COOiBu); 1,05 (d, J=7 Hz, 3H, H-21); 1,19 u, 1,20 (2x s, je 3H, H-26 u. H-27); 2,60 (d, J=6 Hz, 1H, OH); 3,89 (d, J=7 Hz, 2H, COOiBu); 4,08 (dd, J=7, 6 Hz, 1H, H-24); 4,19 (m, 1H, H-3); 4,38 (m, 1H, H-1); 4,87 u. 5,18 (2x s, je 1H, H-19); 5,37 (dd, J=15, 7 Hz, 1H, H-23); 5,52 (dd, J=15, 9 Hz, 1H, H-22); 6,02 u. 6,23 (2x d, J=11 Hz, je 1H, H-6 u. H-7)

**116b:** δ= 0,05 ppm (s, 12H, SiMe); 0,54 (s, 3H, H-18); 0,89 (s, 18H, Si-t-butyl); 0,96 (d, J=7 Hz, 6H, COOiBu); 1,04 (d, J=7 Hz, 3H, H-21); 1,18 u, 1,19 (2x s, je 3H, H-26 u. H-27); 2,59 (d, J=7 Hz, 1H, OH); 3,89 (d, J=7 Hz, 2H, COOiBu); 4,10 (t, J=7 Hz, 1H, H-24); 4,19 (m, 1H, H-3); 4,38 (m, 1H, H-1); 4,87 u. 5,18 (2x s, je 1H, H-19); 5,39 (dd, J=15, 7 Hz, 1H, H-23); 5,59 (dd, J=15, 9 Hz, 1H, H-22); 6,02 u. 6,23 (2x d, J=11 Hz, je 1H, H-6 u. H-7)

**117) (5*Z*,7*E*,22*E*)-(1*S*,3*R*,24*R*)-1,3,24-Trihydroxy-9,10-secocholesta-5,7,10(19),22-tetraen-25-carbonsäure-2-methylpropylester 117a**

Man setzt 98 mg (0,13 mmol) **116a** analog **73)** um und erhält 48 mg der Titelverbindung als farblosen Schaum.

$^1$H-NMR ($CD_2Cl_2$): δ= 0,57 ppm (s, 3H, H-18); 0,95 (d, J=7 Hz, 6H, COOiBu); 1,04 (d, J=7 Hz, 3H, H-21); 1,12 u. 1,13 (2x s, je 3H, H-26 u. H-27); 2,44 (d, J=6 Hz, 1H, OH); 3,85 (d, J=7 Hz, 2H, COOiBu); 4,04 (dd, J=7, 6 Hz, 1H, H-24); 4,17 (m, 1H, H-3); 4,38 (m, 1H, H-1); 4,95 u. 5,28 (2x s, je 1H, H-19); 5,36 (dd, J=15, 7 Hz, 1H, H-23); 5,52 (dd, J=15, 9 Hz, 1H, H-22); 6,01 u. 6,37 (2x d, J=11 Hz, je 1H, H-6 u. H-7)

**118) (5*Z*,7*E*,22*E*)-(1*S*,3*R*,24*S*)-1,3,24-Trihydroxy-9,10-secocholesta-5,7,10(19),22-tetraen-25-carbonsäure-2-methylpropylester 117b**

Man setzt 193 mg (0,26 mmol) **116b** analog **73**, um und erhält 105 mg der Titelverbindung als farblosen Schaum.

$^1$H-NMR ($CD_2Cl_2$): δ= 0,56 ppm (s, 3H, H-18); 0,95 (d, J=7 Hz, 6H, COOiBu); 1,03 (d, J=7 Hz, 3H, H-21); 1,14 (s, 6H, H-26 u. H-27); 2,46 (d, J=6 Hz, 1H, OH); 3,84 (d, J=7 Hz, 2H, COOiBu); 4,08 (dd, J=7, 6 Hz, 1H, H-24); 4,17 (m, 1H, H-3); 4,38 (m, 1H, H-1); 4,96 u. 5,29 (2x s, jc 1H, H-19); 5,38 (dd, J=15, 7 Hz, 1H, H-23); 5,58 (dd, J=15, 9 Hz, 1H, H-22); 6,00 u. 6,37 (2x d, J=11 Hz, je 1H, H-6 u. H-7)

**Beispiel 37**

**119) (5*E*,7*E*,22*E*)-(1*S*,3*R*)-1,3-Bis[[dimethyl(1,1-dimethylethyl)silyl]oxy]-24-hydroxy-9,10-secocholesta-5,7,10(19),22-tetraen-25-carbonsäurepentylester 118**

Man setzt 500 mg (0,91 mmol) (5*E*,7*E*,22*E*)-(1*S*,3*R*)-Bis[[dimethyl-(1,1-dimethylethyl)silyl]oxy]-9,10-secochola-5,7,10(19),22-Tien-24-al **3** mit 4,5 mmol LDA und 0,76 ml (4,5 mmol) Isobuttersäurepentylester analog **41)** um und erhält 550 mg der Titelverbindung als farblosen Schaum (1:1 Diastereomere bzgl. C-24).

$^1$H-NMR ($CDCl_3$): δ= 0,03 ppm (s, 12H, SiMe); 0,52 (s, 3H, H-18); 0,88 (t, J=7 Hz, 3H, COOPent); 0,90 (s, 18H, Si-t-butyl); 1,01/1,02 (d, J=7 Hz, 3H, H-21); 1,14 u. 1,15 (2x s, je 3H, H-26 u. H-27); 2,51 (quint, J=7 Hz, 2H, COOPent); 4,02 (t, J=7 Hz, 2H, COOPent); 4,08 (m, 1H, H-24); 4,20 (m, 1H, H-3); 4,52 (m, 1H, H-1); 4,93 u. 4,98 (2x s, je 1H, H-19); 5,35/5,39 (dd, J=15, 7 Hz, 1H, H-23); 5,52/5,57 (dd, J=15, 7 Hz, 1H, H-22); 5,80 u. 6,43 (2x d, J=11 Hz, je 1H, H-6 u. H-7)

**120) (5*Z*,7*E*,22*E*)-(1*S*,3*R*,24*R*)-1,3-Bis[[dimethyl(1,1-dimethylethyl)silyl]oxy]-24-hydroxy-9,10-secocholesta-5,7,10(19),22-tetraen-25-carbonsäurepentylester 119a und (5*Z*,7*E*,22*E*)-(1*S*-3*R*,24*S*)-1,3-Bis[[dimethyl(1,1-dimethylethyl)silyl]oxy]-24-hydroxy-9,10-secocholesta-5,7,10(19),22-tetraen-25-carbonsäurepentylester 119b**

Man setzt 550 mg (0,71 mmol) **118** analog **112)** um und erhält 156 mg **119a** sowie 174 mg **119b** als farblose Schäume.

$^1$H-NMR ($CDCl_3$): **119a:** δ= 0,03 ppm (s, 12H, SiMe); 0,53 (s, 3H, H-18); 0,87 (s, 18H, Si-t-butyl); 0,89 (t, J=7 Hz, 3H, COOPent); 1,02 (d, J=7 Hz, 3H, H-21); 1,17 u. 1,18 (2x s, je 3H, H-26 u. H-27); 2,60 (d, J=6 Hz, 1H, OH); 4,08 (m, 1H, H-24); 4,09 (t, J=7 Hz, 2H, COOPent); 4,18 (m, 1H, H-3); 4,35 (m, 1H, H-1); 4,85 u. 5,16 (2x s, je 1H, H-19); 5,36 (dd, J=15, 7 Hz, 1H, H-23); 5,53 (dd, J=15, 9 Hz, 1H, H-22); 6,00 u. 6,23 (2x d, J=11 Hz, je 1H, H-6 u. H-7)
**119b:** δ= 0,03 ppm (s, 12H, SiMe); 0,52 (s, 3H, H-18); 0,86 (s, 18H, Si-t-butyl); 0,88 (t, J=7 Hz, 3H, COOPent); 1,01 (d, J=7 Hz, 3H, H-21); 1,15 u. 1,16 (2x s, je 3H, H-26 u. H-27); 2,57 (d, J=6 Hz, 1H, OH); 4,08 (m, 1H, H-24); 4,08 (t, J=7 Hz, 2H, COOPent); 4,18 (m, 1H, H-3); 4,34 (m, 1H, H-1); 4,83 u. 5,14 (2x s, je 1H, H-19); 5,37 (dd, J=15, 7 Hz, 1H, H-23); 5,56 (dd, J=15, 9 Hz, 1H, H-22); 5,98 u. 6,22 (2x d, J=11 Hz, je 1H, H-6 u. H-7)

**121) (5*Z*,7*E*,22*E*)-(1*S*,3*R*,24*R*)-1,3,24-Trihydroxy-9,10-secocholesta-5,7,10(19),22-tetraen-25-carbonsäurepentylester 120a**

Man setzt 150 mg (0,2 mmol) **119a** analog **73)** um und erhält 75 mg der Titelverbindung als farblosen Schaum.

$^1$H-NMR ($CD_2Cl_2$): δ= 0,58 ppm (s, 3H, H-18); 0,90 (t, J=7 Hz, 3H, COOPent); 1,04 (d, J=7 Hz, 3H, H-21); 2,43 (d, J=6 Hz, 1H, OH); 4,05 (t, J=7 Hz, 2H, COOPent); 4,08 (m, 1H, H-24); 4,17 (m, 1H, H-3); 4,38 (m, 1H, H-1); 4,95 u. 5,28 (2x s, je 1H, H-19); 5,35 (dd, J=15, 7 Hz, 1H, H-23); 5,52 (dd, J=15, 9 Hz, 1H, H-22); 6,01 u. 6,37 (2x d, J=11 Hz, je 1H, H-6 u. H-7)

**122) (5*Z*,7*E*,22*E*)-(1*S*,3*R*,24*S*)-1,3,24-Trihydroxy-9,10-secocholesta-5,7,10(19),22-tetraen-25-carbonsäurepentylester 120b**

Man setzt 169 mg (0,22 mmol) **119b** analog **73)** um und erhält 86 mg der Titelverbindung als farblosen Schaum.

$^1$H-NMR ($CD_2Cl_2$): δ= 0,57 ppm (s, 3H, H-18); 0,91 (t, J=7 Hz, 3H, COOPent); 1,02 (d, J=7 Hz, 3H, H-21); 2,44 (d, J=6 Hz, 1H, OH); 4,03 (t, J=7 Hz, 2H, COOPent); 4,06 (m, 1H, H-24); 4,18 (m, 1H, H-3); 4,38 (m, 1H, H-1); 4,95 u. 5,29 (2x s, je 1H, H-19); 5,38 (dd, J=15, 7 Hz, 1H, H-23); 5,56 (dd, J=15, 9 Hz, 1H, H-22); 6,01 u. 6,37 (2x d, J=11 Hz, je 1H, H-6 u. H-7)

**Cyclobutyl-Reihe**

**Beispiel 38**

**123) (5*E*,7*E*,22*E*)-(1*S*,3*R*)-1,3-Bis[[dimethyl(1,1-dimethylethyl)silyl]oxy]-24-hydroxy-26a,27-cyclo-26a-homo-9,10-secocholesta-5,7,10(19),22-tetraen-25-carbonsäureethyl-ester 121**

Man setzt 400 mg (0,72 mmol) (5*E*,7*E*,22*E*)-(1*S*,3*R*)-Bis[[dimethyl-(1,1-dimethylethyl)silyl]oxy]-9,10-secochola-5,7,10(19),22-trien-24-al 3 mit 2,2 mmol LDA und 281 mg (2,2 mmol) Cyclobutancarbonsäureethylester analog **41)** um und erhält 300 mg der Titelverbindung als farblosen Schaum (1:1 Diastereomere bzgl. C-24).

$^1$H-NMR ($CDCl_3$): δ= 0,02 ppm (s, 12H, SiMe); 0,50 (s, 3H, H-18); 0,83 u. 0,86 (2x s, je 9H, Si-t-butyl); 0,98/0,99 (d, J=7 Hz, 3H, H-21); 1,25 (t, J=7 Hz, 3H, COOEt); 4,17 (q, J=7 Hz, 2H, COOEt); 4,18 (m, 2H, H-3 u. H-24); 4,49 (m, 1H, H-1); 4,89 u. 4,92 (2x s, je 1H, H-19); 5,35/5,38 (dd, J=15, 6 Hz, 1H, H-23); 5,52 u. 5,57 (dd, J=15, 7 Hz, 1H, H-22); 5,76 u. 6,40 (2x d, J=11 Hz, je 1H, H-6 u. H-7)

**124) (5*Z*,7*E*,22*E*)-(1*S*,3*R*,24*R*)-1,3-Bis[[dimethyl(1,1-dimethylethyl)silyl]oxy]-24-hydroxy-26a,27-cyclo-26a-homo-9,10-secocholesta-5,7,10(19),22-tetraen-25-carbonsäureethylester 122a und (5*Z*,7*E*,22*E*)-(1*S*,3*R*,24*S*)-1,3-Bis[[dimethyl(1,1-dimethylethyl)silyl]oxy]-24-hydroxy-26a,27-cyclo-26a-homo-9,10-secocholesta-5,7,10(19),22-tetraen-25-carbonsäure-ethylester 122b**

Man setzt 300 mg (0,41 mmol) **121** analog **112)** um und erhält 43 mg 122a sowie 67 mg **122b** als farblose Schäume.

$^1$H-NMR ($CDCl_3$): **122a:** δ= 0,06 ppm (s, 12H, SiMe); 0,53 (s, 3H, H-18); 0,89 (s, 18H, Si-t-butyl); 1,02 (d, J=7 Hz, 3H, H-21); 1,30 (t, J=7 Hz, 3H, COOEt); 2,67 (d, J=6 Hz, 1H, OH); 4,19 (q, J=7 Hz, 2H, COOEt); 4,20 (m, 2H, H-3 u. H-24); 4,37 (m, 1H, H-1); 4,85 u. 5,18 (2x s, je 1H, H-19); 5,38 (dd, J=15, 7 Hz, 1H, H-23); 5,58 (dd, J=15, 7 Hz, 1H, H-22); 6,00 u. 6,23 (2x d, J=11 Hz, je 1H, H-6 u. H-7)

**122b:** δ= 0,06 ppm (s, 12H, SiMe); 0,53 (s, 3H, H-18); 0,89 (s, 18H, Si-t-butyl); 1,02 (d, J=7 Hz, 3H, H-21); 1,30 (t, J=7 Hz, 3H, COOEt); 2,64 (d, J=6 Hz, 1H, OH); 4,18 (q, J=7 Hz, 2H, COOEt); 4,20 (m, 1H, H-3); 4,22(m, 1H, H-24); 4,37 (m, 1H, H-1); 4,85 u. 5,18 (2x s, je 1H, H-19); 5,40 (dd, J=15, 7 Hz, 1H, H-23); 5,61 (dd, J=15, 7 Hz, 1H, H-22); 6,00 u. 6,23 (2x d, J=11 Hz, je 1H, H-6 u. H-7)

**125) (5*Z*,7*E*,22*E*)-(1*S*,3*R*,24*R*)-1,3,24-Trihydroxy-26a,27-cyclo-26a-homo-9,10-secocholesta-5,7,10(19),22-tetraen-25-carbonsäureethylester 123a**

Man setzt 43 mg (0,059 mmol) **122a** analog **73)** um und erhält 15 mg der Titelverbindung als farblosen Schaum.

$^1$H-NMR ($CD_2Cl_2$): δ= 0,52 ppm (s, 3H, H-18); 1,01 (d, J=7 Hz, 3H, H-21); 1,23 (t, J=7 Hz, 3H, COOEt); 4,13 (q, J=7 Hz, 2H, COOEt); 4,13 (m, 2H, H-3 u. H-24); 4,33 (m, 1H, H-1); 4,91 u. 5,25 (2x s, je 1H, H-19); 5,34 (dd, J=15, 7 Hz, 1H, H-23); 5,53 (dd, J=15, 7 Hz, 1H, H-22); 5,96 u. 6,32 (2x d, J=11 Hz, je 1H, H-6 u. H-7)

**126) (5*Z*,7*E*,22*E*)-(1*S*,3*R*,24*S*)-1,3,24-Trihydroxy-26a,27-cyclo-26a-homo-9,10-secocholesta-5,7,10(19),22-tetraen-25-carbonsäureethylester 123b**

Man setzt 43 mg (0,059 mmol) **122b** analog **73)** um und erhält 26 mg der Titelverbindung als farblosen Schaum.

$^1$H-NMR ($CD_2Cl_2$): δ= 0,52 ppm (s, 3H, H-18); 1,00 (d, J=7 Hz, 3H, H-21); 1,24 (t, J=7 Hz, 3H, COOEt); 4,12 (q, J=7 Hz, 2H, COOEt); 4,13 (m, 2H, H-3 u. H-24); 4,34 (m, 1H, H-1); 4,92 u. 5,26 (2x s, je 1H, H-19); 5,38 (dd, J=15, 7 Hz, 1H, H-23); 5,57 (dd, J=15, 7 Hz, 1H, H-22); 5,97 u. 6,33 (2x d, J=11 Hz, je 1H, H-6 u. H-7)

**C-24-Ketone (Normalreihe)**

**Beispiel 39**

**127) (5*Z*,7*E*,22*E*)-(1*S*,3*R*)-1,3-Bis[[dimethyl(1,1-dimethylethyl)silyl]oxy]-24-oxo-9,10-secocholesta-5,7,10(19), 22-tetraen-25-carbonsäuremethylester 124**

Man setzt 200 mg (0,3 mmol) **14a** analog **78)** um und erhält 135 mg der Titelverbindung als farblosen Schaum.

$^1$H-NMR ($CDCl_3$): δ= 0,01 ppm (s, 12H, SiMe); 0,50 (s, 3H, H-18); 0,82 (s, 18H, Si-t-butyl); 1,02 (d, J=7 Hz, 3H, H-21); 1,33 (s, 6H, C-26 u. C-27); 3,64 (s, 3H, COOMe); 4,12 (m, 1H, H-3); 4,31 (m, 1H, H-1); 4,89 u. 5,12 (2x s, je 1H, H-19); 5,95 u. 6,18 (2x d, J=11 Hz, je 1H, H-6 u. H-7); 6,08 (d, J=15 Hz, 1H, H-23); 6,78 (dd, J=15, 9,5 Hz, 1H, H-22)

**128) (5*Z*,7*E*,22*E*)-(1*S*,3*R*)-1,3-Dihydroxy-24-oxo-9,10-secocholesta-5,7,10(19),22-tetraen-25-carbonsäuremethylester 125**

Man setzt 130 mg (0,19 mmol) **124** analog **73)** um und erhält 31 mg der Titelverbindung als farblosen Schaum.

$^1$H-NMR ($CD_2Cl_2$): δ= 0,55 ppm (s, 3H, H-18); 1,06 (d, J=7 Hz, 3H, H-21); 1,33 (3, 6H, C-26 u. C-27); 3,65 (s, 3H, COOMe); 4,14 (m, 1H, H-3); 4,35 (m, 1H, H-1); 4,93 u. 5,25 (2x s, je 1H, H-19); 5,98 u. 6,34 (2x d, J=11 Hz, je 1H, H-6 u. H-7); 6,13 (d, J=15 Hz, 1H, H-23); 6,76 (dd, J=15, 9 Hz, 1H, H-22)

**Beispiel 40**

**129) (5*Z*,7*E*,22*E*)-(1*S*,3*R*)-1,3-Bis[[dimethyl(1,1-dimethylethyl)silyl]oxy]-24-oxo-9,10-secocholesta-5,7,10(19), 22-tetraen-25-carbonsäureethylester 126**

Man setzt 420 mg (0,59 mmol) **5a** analog **78)** um und erhält 350 mg der Titelverbindung als farblosen Schaum.
$^1$H-NMR ($CDCl_3$): δ= 0,01 ppm (s, 12H, SiMe); 0,50 (s, 3H, H-18); 0,82 (s, 18H, Si-t-butyl); 1,02 (d, J=7 Hz, 3H, H-21); 1,18 (t, J=7 Hz, 3H, COOEt); 1,20 u. 1,30 (2x s, je 3H, C-26 u. C-27); 4,10 (q, J=7 Hz, 2H, COOEt); 4,12 (m, 1H, H-3); 4,30 (m, 1H, H-1); 4,89 u. 5,10 (2x s, je 1H, H-19); 5,95 u. 6,18 (2x d, J=11 Hz, je 1H, H-6 u. H-7); 6,08 (d, J=15 Hz, 1H, H-23); 6,78 (dd, J=15, 9,5 Hz, 1H, H-22)

**130) (5*Z*,7*E*,22*E*)-(1*S*,3*R*)-1,3-Dihydroxy-24-oxo-9,10-secocholesta-5,7,10(19),22-tetraen-25-carbonsäureethylester 127**

Man setzt 50 mg (0,07 mmol) **126** analog **73)** um und erhält 23 mg der Titelverbindung als farblosen Schaum.
$^1$H-NMR ($CD_2Cl_2$): δ= 0,55 ppm (s, 3H, H-18); 1,06 (d, J=7 Hz, 3H, H-21); 1,30 (t, J=7 Hz, 3H, COOEt); 1,32 (s, 6H, C-26 u. C-27); 4,13 (q, J=7 Hz, 3H, COOEt); 4,14 (m, 1H, H-3); 4,35 (m, 1H, H-1); 4,93 u. 5,27 (2x s, je 1H, H-19); 5,98 u. 6,34 (2x d, J=11 Hz, je 1H, H-6 u. H-7); 6,13 (d, J=15 Hz, 1H, H-23); 6,78 (dd, J=15, 9 Hz, 1H, H-22)

**Beispiel 41**

**131) (5*Z*,7*E*,22*E*)-(1*S*,3*R*)-1,3-Bis[[dimethyl(1,1-dimethylethyl)silyl]oxy]-24-oxo-9,10-secocholesta-5,7,10(19), 22-tetraen-25-carbonsäurepropylester 128**

Man setzt 204 mg (0,3 mmol) Verb. **18a** (EM 50741) analog **78)** um und erhält 155 mg der Titelverbindung als farblosen Schaum.
$^1$H-NMR ($CDCl_3$): δ= 0,01 ppm (s, 12H, SiMe); 0,50 (s, 3H, H-18); 0,83 (s, 18H, Si-t-butyl); 0,84 (t, J=7 Hz, 3H, COOPr); 1,02 (d, J=7 Hz, 3H, H-21); 1,19 u. 1,30 (2x s, je 3H, C-26 u. C-27); 4,02 (m, Hz, 2H, COOPr); 4,13 (m, 1H, H-3); 4,30 (m, 1H, H-1); 4,90 u. 5,11 (2x s, je 1H, H-19); 5,95 u. 6,19 (2x d, J=11 Hz, je 1H, H-6 u. H-7); 6,08 (d, J=15 Hz, 1H, H-23); 6,77 (dd, J=15, 9,5 Hz, 1H, H-22)

**132) (5*Z*,7*E*,22*E*)-(1*S*,3*R*)-1,3-Dihydroxy-24-oxo-9,10-secocholesta-5,7,10(19),22-tetraen-25-carbonsäurepropylester 129**

Man setzt 145 mg (0,2 mmol) **128** analog **73)** um und erhält 56 mg der Titelverbindung als farblosen Schaum.
$^1$H-NMR ($CD_2Cl_2$): δ= 0,55 ppm (s, 3H, H-18); 0,80 (t, J=7 Hz, 3H, COOPr); 1,08 (d, J=7 Hz, 3H, H-21); 1,32 (3, 6H, C-26 u. C-27); 4,03 (t, J=7 Hz, 2H, COOPr); 4,17 (m, 1H, H-3); 4,38 (m, 1H, H-1); 4,95 u. 5,29 (2x s, je 1H, H-19); 6,00 u. 6,35 (2x d, J=11 Hz, je 1H, H-6 u. H-7); 6,15 (d, J=15 Hz, 1H; H-23); 6,79 (dd, J=15, 9 Hz, 1H, H-22)

**Beispiel 42**

**133) (5*Z*,7*E*,22*E*)-(1*S*,3*R*)-1,3-Bis[[dimethyl(1,1-dimethylethyl)silyl]oxyl-24-oxo-9,10-secocholesta-5,7,10(19), 22-tetraen-25-carbonsäure-1-methylethylester 130**

Man setzt 800 mg (1,14 mmol) **12a** analog **78)** um und erhält 630 mg der Titelverbindung als farblosen Schaum.
$^1$H-NMR ($CDCl_3$): δ= 0,01 ppm (s, 12H, SiMe); 0,50 (s, 3H, H-18); 0,83 (s, 18H, Si-t-butyl); 1,03 (d, J=7 Hz, 3H, H-21); 1,18 (d, J=7 Hz, 6H, COOiPr); 1,31 (s, 6H, C-26 u. C-27); 4,13 (m, 1H, H-3); 4,32 (m, 1H, H-1); 4,95 (hept, J=7 Hz, 1H, COOiPr); 4,89 u. 5,12 (2x s, je 1H, H-19); 5,95 u. 6,17 (2x d, J=11 Hz, je 1H, H-6 u. H-7); 6,08 (d, J=15 Hz, 1H, H-23); 6,79 (dd, J=15,9,5 Hz, 1H,H-22)

**134) (5*Z*,7*E*,22*E*)-(1*S*,3*R*)-1,3-Dihydroxy-24-oxo-9,10-secocholesta-5,7,10(19),22-tetraen-25-carbonsäure-1-methylethylester 131**

Man setzt 130 mg (0,18 mmol) **130** analog **73)** um und erhält 64 mg der Titelverbindung als farblosen Schaum.
$^1$H-NMR ($CD_2Cl_2$): δ= 0,55 ppm (s, 3H, H-18); 1,06 (d, J=7 Hz, 3H, H-21); 1,22 (d, J=7 Hz, 6H, COOiPr); 1,31 (3, 6H, C-26 u. C-27); 4,14 (m, 1H, H-3); 4,36 (m, 1H, H-1); 4,99 (hept, J=7 Hz, 1H, COOiPr); 4,94 u. 5,28 (2x s, je 1H, H-19); 5,99 u. 6,35 (2x d, J=11 Hz, je 1H, H-6 u. H-7); 6,13 (d, J=15 Hz, 1H, H-23); 6,78 (dd, J=15, 9 Hz, 1H, H-22)

**Beispiel 43**

**135) (5*Z*,7*E*,22*E*)-(1*S*,3*R*)-1,3-Bis[[dimethyl(1,1-dimethylethyl)silyl]oxy]-24-oxo-9,10-secocholesta-5,7,10(19),22-tetraen-25-carbonsäurebutylester <u>132</u>**

Man setzt 180 mg (0,23 mmol) **<u>113a/113b</u>** analog **78)** um und erhält 100 mg der Titelverbindung als farblosen Schaum.

$^1$H-NMR ($CDCl_3$): δ= 0,04 ppm (s, 12H, SiMe); 0,53 (s, 3H, H-18); 0,89 (s, 18H, Si-t-butyl); 0,90 (t, J= 7 Hz, 3H, COOBu); 1,06 (d, J=7 Hz, 3H, H-21); 1,36 (s, 6H, C-26 u. C-27); 4,08 (t, J=7 Hz, 3H, COOBu); 4,18 (m, 1H, H-3); 4,38 (m, 1H, H-1); 4,85 u. 5,18 (2x s, je 1H, H-19); 6,00 u. 6,22 (2x d, J=11 Hz, je 1H, H-6 u. H-7); 6,14 (d, J=15 Hz, 1H, H-23); 6,82 (dd, J=15, 9,5 Hz, 1H, H-22)

**136) (5*Z*,7*E*,22*E*)-(1*S*,3*R*)-1,3-Dihydroxy-24-oxo-9,10-secocholesta-5,7,10(19),22-tetraen-25-carbonsäurebutylester <u>133</u>**

Man setzt 95 mg (0,13 mmol) **<u>132</u>** analog **73)** um und erhält 35 mg der Titelverbindung als farblosen Schaum.

$^1$H-NMR ($CD_2Cl_2$): δ= 0,56 ppm (s, 3H, H-18); 0,89 (t, J=7 Hz, 3H, COOBu); 1,08 (d, J=7 Hz, 3H, H-21); 1,32 (3, 6H, C-26 u. C-27); 4,08 (tbr, J=7 Hz, 2H, COOBu); 4,17 (m, 1H, H-3); 4,38 (m, 1H, H-1); 4,95 u. 5,29 (2x s, je 1H, H-19); 6,00 u. 6,36 (2x d, J=11 Hz, je 1H, H-6 u. H-7); 6,15 (d, J=15 Hz, 1H, H-23); 6,80 (dd, J=15, 9 Hz, 1H, H-22)

**Beispiel 44**

**137) (5*Z*,7*E*,22*E*)-(1*S*,3*R*)-1,3-Bis[[dimethyl(1,1-dimethylethyl)silyl]oxy]-24-oxo-9,10-secocholesta-5,7,10(19),22-tetraen-25-carbonsäure-2-methylpropylester <u>134</u>**

Man setzt 205 mg (0,28 mmol) **<u>116a/116b</u>** analog **78)** um und erhält 120 mg der Titelverbindung als farblosen Schaum.

$^1$H-NMR ($CDCl_3$): δ = 0,01 ppm (s, 12H, SiMe); 0,50 (s, 3H, H-18); 0,82 (s, 18H, Si-t-butyl); 0,86 (d, J= 7 Hz, 6H, COOiBu); 1,04 (d, J=7 Hz, 3H, H-21); 1,32 (s, 6H, C-26 u. C-27); 3,87 u. 3,90 (2x dd, J=10, 7 Hz, je 1H, COOiBu); 4,14 (m, 1H, H-3); 4,33 (m, 1H, H-1); 4,89 u. 5,11 (2x s, je 1H, H-19); 5,95 u. 6,17 (2x d, J=11 Hz, je 1H, H-6 u. H-7); 6,09 (d, J=15 Hz, 1H, H-23); 6,78 (dd, J=15, 9,5 Hz, 1H, H-22)

**138) (5*Z*,7*E*,22*E*)-(1*S*,3*R*)-1,3-Dihydroxy-24-oxo-9,10-secocholesta-5,7,10(19),22-tetraen-25-carbonsäure-2-methylpropylester <u>135</u>**

Man setzt 115 mg (0,15 mmol) **<u>134</u>** analog **73)** um und erhält 54 mg der Titelverbindung als farblosen Schaum.

$^1$H-NMR ($CD_2Cl_2$): δ= 0,56 ppm (s, 3H, H-18); 0,89 (d, J=7 Hz, 6H, COOiBu); 1,08 (d, J=7 Hz, 3H, H-21); 1,34 (3, 6H, C-26 u. C-27); 3,84 u. 3,89 (2x dd, J=10, 6 Hz, je 1H, COOiBu); 4,17 (m, 1H, H-3); 4,38 (m, 1H, H-1); 4,95 u. 5,29 (2x s, je 1H, H-19); 6,00 u. 6,35 (2x d, J=11 Hz, je 1H, H-6 u. H-7); 6,16 (d, J=15 Hz, 1H, H-23); 6,80 (dd, J=15, 9 Hz, 1H, H-22)

**Beispiel 45**

**139) (5*Z*,7*E*,22*E*)-(1*S*,3*R*)-1,3-Bis[[dimethyl(1,1-dimethylethyl)silyl]oxy]-24-oxo-9,10-secocholesta-5,7,10(19),22-tetraen-25-carbonsäurepentylester <u>136</u>**

Man setzt 190 mg (0,25 mmol) **<u>119a/119b</u>** analog **78)** um und erhält 108 mg der Titelverbindung als farblosen Schaum.

$^1$H-NMR ($CDCl_3$): δ= 0,04 ppm (s, 12H, SiMe); 0,55 (s, 3H, H-18); 0,89 (s, 18H, Si-t-butyl); 0,89 (t, J= 7 Hz, 3H, COOPent); 1,08 (d, J=7 Hz, 3H, H-21); 1,36 (s, 6H, C-26 u. C-27); 4,09 (t, J=7 Hz, 3H, COOPent); 4,19 (m, 1H, H-3); 4,38 (m, 1H, H-1); 4,85 u. 5,18 (2x s, je 1H, H-19); 6,01 u. 6,22 (2x d, J=11 Hz, je 1H, H-6 u. H-7); 6,14 (d, J=15 Hz, 1H, H-23); 6,83 (dd, J=15, 9,5 Hz, 1H, H-22)

**140) (5*Z*,7*E*,22*E*)-(1*S*,3*R*)-1,3-Dihydroxy-24-oxo-9,10-secocholesta-5,7,10(19),22-tetraen-25-carbonsäurepentylester <u>137</u>**

Man setzt 103 mg (0,14 mmol) **<u>136</u>** analog **73)** um und erhält 42 mg der Titelverbindung als farblosen Schaum.

$^1$H-NMR ($CD_2Cl_2$): δ= 0,56 ppm (s, 3H, H-18); 0,88 (t, J=7 Hz, 3H, COOPent); 1,08 (d, J=7 Hz, 3H, H-21); 1,32

(3, 6H, C-26 u. C-27); 4,08 (t, J=7 Hz, 2H, COOPent); 4,17 (m, 1H, H-3); 4,38 (m, 1H, H-1); 4,95 u. 5,30 (2x s, je 1H, H-19); 6,00 u. 6,35 (2x d, J=11 Hz, je 1H, H-6 u. H-7); 6,15 (d, J=15 Hz, 1H, H-23); 6,80 (dd, J=15, 9 Hz, 1H, H-22)

## Patentansprüche

**1.** 25-Carbonsäure-Derivate der allgemeinen Formel **I**,

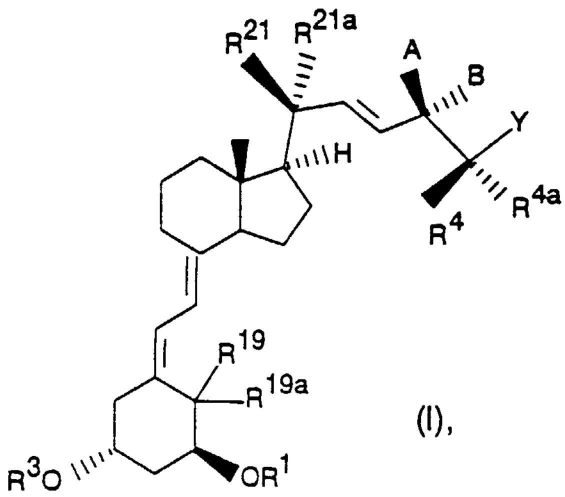

(I),

worin $R^1$ und $R^3$ unabhängig voneinander je ein Wasserstoffatom, eine gerad- oder verzweigtkettige gesättigte Alkanoylgruppe mit 1 bis 9 Kohlenstoffatomen oder eine Aroylgruppe,

$R^{19}$ und $R^{19a}$ je ein Wasserstoffatom oder gemeinsam eine exocyclische Methylengruppe,
A und B gemeinsam ein Ketosauerstoffatom oder A eine Gruppe $OR^{24}$ und B ein Wasserstoffatom oder A ein Wasserstoffatom und B eine Gruppe $OR^{24}$, wobei $R^{24}$ ein Wasserstoffatom oder eine gerad- oder verzweigtkettige gesättigte Alkanoylgruppe mit bis zu 9 Kohlenstoffatomen oder eine Aroylgruppe ist,
$R^{21}$ und $R^{21a}$ unabhängig voneinander ein Wasserstoffatom, ein Chlor- oder Fluoratom, eine Alkylgruppe mit 1 bis 4 Kohlenstoffatomen, gemeinsam eine Methylengruppe, gemeinsam mit dem Kohlenstoffatom 20 einen 3-7 gliedrigen, gesättigten oder ungesättigten carbocyclischen Ring,
$R^4$ und $R^{4a}$ gleichzeitig je ein Wasserstoffatom, ein Chlor- oder Fluoratom, eine Trifluormethylgruppe, einen gerad- oder verzweigtkettigen, gesättigten oder ungesättigten Kohlenwasserstoffrest mit bis zu 4 Kohlenstoffatomen oder $R^4$ und $R^{4a}$ gemeinsam mit dem Kohlenstoffatom 25 einen 3- bis 7-gliedrigen, gesättigten oder ungesättigten carbocylischen Ring sowie Y einen der Reste $-C(O)NR^5R^{5'}$,$-C(O)OR^6$, -CN, wobei $R^5$ und $R^{5'}$ unabhängig voneinander und $R^6$ je für ein Wasserstoffatom oder eine lineare oder verzweigte Alkylgruppe mit bis zu 8 Kohlenstoffatomensowie $R^6$ zusätzlich für einen ungesättigten, linearen oder verzweigten Kohlenwasserstoffrest mit 3 bis 8 Kohlenstoffatomen oder für die Gruppe $-(CH_2)_m-CH(CH_2)_n$ mit m=0 oder 1 und n=2, 3, 4, 5 oder 6 und wenn m = 1 zusätzlich mit n = 1
stehen,
bedeuten.

**2.** Verbindungen der allgemeinen Formel I nach Anspruch 1, worin $R^1$, $R^3$ und $R^{24}$ je ein Wasserstoffatom bedeuten.

**3.** Verbindungen der allgemeinen Formel I nach Anspruch 1, worin $R^1$, $R^3$ und $R^{24}$ je für einen Acetyl-, Propionyl-, n-Butyryl-, iso-Butyryl-, Pivaloyl- oder Valerylrest stehen.

**4.** Verbindungen der allgemeinen Formel I nach Anspruch 1, worin $R^1$, $R^3$ und $R^{24}$ je für einen Benzoylrest stehen.

**5.** Verbindungen der allgemeinen Formel I nach Anspruch 1, worin $R^4$ und $R^{4a}$ je für einen Methyl-, Ethyl-, n-Propyl-, n-Butyl-, i-Propyl-, i-Butyl- oder tert.-Butylrest stehen.

**6.** Verbindungen der allgemeinen Formel I nach Anspruch 1, worin $R^{21}$ für ein Wasserstoffatom und $R^{21a}$ für eine

Methylgruppe stehen.

**7.** Verbindungen der allgemeinen Formel I nach Anspruch 1, worin der Substituent $R^{21}$ oder. $R^{21a}$ für einen Methyl-, Ethyl-, n-Propyl-, n-Butyl-, i-Propyl-, i-Butyl- oder tert.-Butylrest stehen und der andere Substituent für eine Methylgruppe steht.

**8.** Verbindungen der allgemeinen Formel I nach Anspruch 1, worin $R^{21}$ und $R^{21a}$. gemeinsam mit dem Kohlenstoffatom 20 einen Cyclopropylring bilden.

**9.** Verbindungen der allgemeinen Formel I nach Anspruch 1, worin $R^{21}$ und $R^{21a}$ gemeinsam für eine Methylengruppe stehen.

**10.** Verbindungen der allgemeinen Formel I nach Anspruch 1, worin $R^{21}$ ein Fluoratom und $R^{21a}$ eine Methylgruppe oder $R^{21}$ eine Methylgruppe und $R^{21a}$ ein Fluoratom bedeuten.

**11.** Verbindungen der allgemeinen Formel I nach Anspruch 1, worin $R^{4}$ und $R^{4a}$ jeweils eine Methyl- oder Ethylgruppe bedeuten oder gemeinsam mit dem tertiären Kohlenstoffatom 25 einen Cyclopropyl-, -butyl-, -pentyl- oder -hexylring bilden.

**12.** Verbindungen der allgemeinen Formel I nach Anspruch 1, worin $R^{5}$, $R^{5'}$ und/oder $R^{6}$ eine Methyl-, Ethyl-, n-Propyl-, n-Butyl-, i-Propyl-, i-Butyl- oder tert.-Butylgruppe ist.

**13.** Verbindungen der allgemeinen Formel I nach Anspruch 1, worin $R^{19}$ und $R^{19a}$ jeweils für ein Wasserstoffatom stehen.

**14.** Verbindungen nach Anspruch 13, worin $R^{21}$ für ein Wasserstoffatom und $R^{21a}$ für eine Methylgruppe stehen.

**15.** Verbindungen nach Anspruch 13, worin $R^{21}$ und $R^{21a}$ gemeinsam für eine Methylengruppe stehen.

**16.** Verbindungen nach Anspruch 13, worin $R^{21}$ und $R^{21a}$ je für eine Methylgruppe stehen.

**17.** Verbindungen nach Anspruch 13, worin $R^{21}$ für ein Wasserstoffatom und $R^{21a}$ für ein Fluoratom oder umgekehrt stehen.

**18.** Verbindungen der allgemeinen Formel I nach Anspruch 1, worin $R^{19}$ und $R^{19a}$ gemeinsam für eine Methylengruppe stehen.

**19.** Verbindungen nach Anspruch 18, worin $R^{21}$ für ein Wasserstoffatom und $R^{21a}$ für eine Methylgruppe stehen.

**20.** Verbindungen nach Anspruch 18, worin $R^{21}$ und $R^{21a}$ gemeinsam für eine Methylengruppe stehen.

**21.** Verbindungen nach Anspruch 18, worin $R^{21}$ und $R^{21a}$ je für eine Methylgruppe stehen.

**22.** Verbindungen der allgemeinen Formel I, nämlich

*(5Z,7E,22E)-(1S,3R,24R)*-1,3,24-Trihydroxy-9,10-secocholesta-5,7,10(19),22-tetraen-25-carbonsäureethylester
*(5Z,7E,22E)-(1S,3R,24R)*-26,27-Dimethyl-1,3,24-trihydroxy-9,10-secocholesta-5,7,10(19),22-tetraen-25-carbonsäuremethylester
*(5Z,7E,22E)-(1S,3R,24R)-1,3,24-Trihydroxy*-9,10-*secocholesta-5,7,10(19),22-tetraen-25*-carbonsäure-1-methylethylester
*(5Z,7E,22E)-(1S,3R,24R)*-1,3,24-Trihydroxy-9,10-secocholesta-5,7,10(19),22-tetraen-25-carbonsäuremethylester
*(5Z,7E,22E)-(1S,3R,24S)*-1,3,24-Trihydroxy-9,10-secochola-5,7,10(19),22-tetraen-24-essigsäuremethylester
*(5Z,7E,22E)-(1S,3R,24R )*-1,3,24-Trihydroxy-9,10-secocholesta-5,7,10(19),22-tetraen-25-carbonsäurenitril
*(5Z, 7E,22E)-(1S,3R,24R)*-1,3,24-Trihydroxy-9,10-secocholesta-5,7,10(19),22-tetraen-25-carbonsäurepropylester

*(5Z,7E,22E)-(1S,3R,24R)*-1,3,24-Trihydroxy-9,10-secocholesta-5,7,10(19),22-tetraen-25-carbonsäure
*(5Z,7E,22E)-(1S,3R,24R)*-1,3,24-Trihydroxy-26,27-cyclo-9,10-secocholesta-5,7,10(19),22-tetraen-25-carbonsäuremethylester
*(5Z,7E,22E)-(1S,3R,24R)*-1,3,24-Trihydroxy-26,27-cyclo-9,10-secocholesta-5,7,10(19),22-tetraen-25-carbonsäureethylester
*(5Z,7E,22E)-(1S,3R,24R)*-1,3,24-Trihydroxy-26,27-cyclo-9,10-secocholesta-5,7,10(19),22-tetraen-25-carbonsäurepropylester
*(5Z,7E,22E)-(1S,3R,24R)*-1,3,24-Trihydroxy-26,27-cyclo-9,10-secocholesta-5,7,10(19),22-tetraen-25-carbonsäurebutylester
*(5Z,7E,22E)-(1S,3R,24R)*- 1,3,24-Trihydroxy-26,27-cyclo-9,10-secocholesta-5,7,10(19),22-tetraen-25-carbonsäure-2-methylpropylester.
*(5Z,7E,22E)-(1S,3R,24R)*,1,3,24-Trihydroxy-26,27-cyclo-9,10-secocholesta-5,7,10(19),22-tetraen-25-carbonsäurepentylester
(5*Z*,7*E*,22*E*)-(1*S*,3*R*,24*R*)1,3,24-Trihydroxy-26,27-cyclo-9,10-secocholesta-5,7,10(19),22-tetraen-25-carbonsäurehexylester
(5*Z*,7*E*,22*E*)-(1*S*,3*R*,24*R*)-1,3,24-Trihydroxy-9,10-*secocholesta-5,7,10(19),22*-tetraen-25-carbonsäuredimethylamid
(5*Z*,7*E*,22*E*)-(1*S*,3*R*,24*R*)-1,3,24-Trihydroxy-9,10-secocholesta-5,7,10(19),22-tetraen-25-carbonsäurediethylamid
(5*Z*,7*E*,22*E*)-(1*S*,3*R*,24*R*)-1,3,24-Trihydroxy-26,27-cyclo-9,10-secocholesta-5,7,10(19),22-tetraen-25-carbonsäurediethylamid
(5*Z*,7*E*,22*E*)-(1*S*,3*R*)-1,3-Dihydroxy-24-oxo-26,27-cyclo-9,10-secocholesta-5,7,10(19),22-tetraen-25-carbonsäure-n-butylamid
(5*Z*,7*E*,22*E*)-(1*S*,3*R*,24*R*)-20-Methyl-1,3,24-trihydroxy-9,10-secocholesta-5,7,10(19),22-tetraen-25-carbonsäuremethylester
(5*Z*,7*E*,22*E*)-(1*S*,3*R*,24*S*)-20-Methyl-1,3,24-trihydroxy-9,10-secocholesta-5,7,10(19),22-tetraen-25-carbonsäuremethylester
(5*Z*,7*E*,22*E*)-(1*S*,3*R*,24*R*)-20-Methyl-1,3,24-trihydroxy-9,10-secocholesta-5,7,10(19),22-tetraen-25-carbonsäureethylester
(5*Z*,7*E*,22*E*)-(1*S*,3*R*,24*S*)-20-Methyl-1,3,24-trihydroxy-9,10-secocholesta-5,7,10(19),22-tetraen-25-carbonsäureethylester
(5*Z*,7*E*,22*E*)-(1*S*,3*R*,24*R*)-20-Methyl-1,3,24-trihydroxy-9,10-secocholesta-5,7,10(19),22-tetraen-25-carbonsäurepropylester
(5*Z*,7*E*,22*E*)-(1*S*,3*R*,24*S*)-20-Methyl-1,3,24-trihydroxy-9,10-secocholesta-5,7,10(19),22-tetraen-25-carbonsäurepropylester
(5*Z*,7*E*,22*E*)-(1*S*,3*R*,24*R*)-20-Methyl-1,3,24-trihydroxy-9,10-secocholesta-5,7,10(19),22-tetraen-25-carbonsäure-1-methylethylester
(5*Z*,7*E*,22*E*)-(1*S*,3*R*,24*S*)-20-Methyl-1,3,24-trihydroxy-9,10-secocholesta-5,7,10(19),22-tetraen-25-carbonsäure-1-methylethylester
(5*Z*,7*E*,22*E*)-(1*S*,3*R*,24*R*)-20-Methyl-1,3,24-trihydroxy-9,10-secocholesta-5,7,10(19),22-tetraen-25-carbonsäurebutylester
(5*Z*,7*E*,22*E*)-(1*S*,3*R*,24*S*)-20-Methyl-1,3,24-trihydroxy-9,10-secocholesta-5,7,10(19),22-tetraen-25-carbonsäurebutylester
(5*Z*,7*E*,22*E*)-(1*S*,3*R*,24*R*)-1,3,24-Trihydroxy-9,10-secocholesta-5,7,10(19),20,22-pentaen-25-carbonsäure-methylester
(5*Z*,7*E*,22*E*)-(1*S*,3*R*,24*S*)-1,3,24-Trihydroxy-9,10-secocholesta-5,7,10(19),20,22-pentaen-25-carbonsäure-methylester
(5*Z*,7*E*,22*E*)-(1*S*,3*R*,24*R*)-1,3,24-Trihydroxy-9,10-secocholesta-5,7,10(19),20,22-pentaen-25-carbonsäure-ethylester
(5*Z*,7*E*,22*E*)-(1*S*,3*R*,24*S*)-1,3,24-Trihydroxy-9,10-secocholesta-5,7,10(19),20,22-pentaen-25-carbonsäure-ethylester
(5*Z*,7*E*,22*E*)-(1*S*,3*R*,24*R*)-1,3,24-Trihydroxy-9,10-secocholesta-5,7,10(19),20,22-pentaen-25-carbonsäurepropylester
(5Z,7*E*,22*E*)-(1*S*,3*R*,24*S*)-1,3,24-Trihydroxy-9,10-secocholesta-5,7,10(19),20,22-pentaen-25-carbonsäurepropylester
(5*Z*,7*E*,22*E*)-(1*S*,3*R*,24*R*)-1,3,24-Trihydroxy-9,10-secocholesta-5,7,10(19),20,22-pentaen-25-carbonsäure-1-methylethylester
(5*Z*,7*E*,22*E*)-(1*S*,3*R*,24*S*)-1,3,24-Trihydroxy-9,10-secocholesta-5,7,10(19),20,22-pentaen-25-carbonsäure-1-methylethylester
(5*Z*,7*E*,22*E*)-(1*S*,3*R*,24*R*)-1,3,24-Trihydroxy-9,10-secocholesta-5,7,10(19),20,22-pentaen-25-carbonsäurebutylester
(5*Z*,7*E*,22*E*)-(1*S*,3*R*,24*S*)-1,3,24-Trihydroxy-9,10-secocholesta-5,7,10(19),20,22-pentaen-25-carbonsäurebutylester
(5*Z*,7*E*,22*E*)-(1*S*,3*R*,24*R*)-20,21-Methylen-1,3,24-trihydroxy-9,10-secocholesta-5,7,10(19),22-tetraen-25-carbonsäuremethylester
(5*Z*,7*E*,22*E*)-(1*S*,3*R*,24*S*)-20,21-Methylen-1,3,24-trihydroxy-9,10-secocholesta-5,7,10(19),22-tetraen-25-carbonsäuremethylester
(5*Z*,7*E*,22*E*)-(1*S*,3*R*,24*R*)-20,21-Methylen-1,3,24-trihydroxy-9,10-secocholesta-5,7,10(19),22-tetraen-25-carbonsäureethylester
(5*Z*,7*E*,22*E*)-(1*S*,3*R*,24*S*)-20,21-Methylen-1,3,24-trihydroxy-9,10-secocholesta-5,7,10(19),22-tetraen-25-carbonsäureethylester
(5*Z*,7*E*,22*E*)-(1*S*,3*R*,24*R*)-20,21-Methylen-1,3,24-trihydroxy-9,10-secocholesta-5,7,10(19),22-tetraen-25-carbonsäurepropylester
(5*Z*,7*E*,22*E*)-(1*S*,3*R*,24*S*)-20,21-Methylen-1,3,24-trihydroxy-9,10-secocholesta-5,7,10(19),22-tetraen-25-carbonsäurepropylester
(5*Z*,7*E*,22*E*)-(1*S*,3*R*,24*R*)-20,21-Methylen-1,3,24-trihydroxy-9,10-secocholesta-5,7,10(19),22-tetraen-25-carbonsäure-1-methylethylester
(5*Z*,7*E*,22*E*)-(1*S*,3*R*,24*S*)-20,21-Methylen-1,3,24-trihydroxy-9,10-secocholesta-5,7,10(19),22-tetraen-25-carbonsäure-1-methylethylester
(5*Z*,7*E*,22*E*)-(1*S*,3*R*,24*R*)-20,21-Methylen-1,3,24-trihydroxy-9,10-secocholesta-5,7,10(19),22-tetraen-25-carbonsäurebutylester
(5*Z*,7*E*,22*E*)-(1*S*,3*R*,24*S*)-20,21-Methylen-1,3,24-trihydroxy-9,10-secocholesta-5,7,10(19),22-tetraen-25-carbonsäurebutylester
(7*E*,22*E*)-(1*R*,3*R*,24*R*)-1,3,24-Trihydroxy-19-nor-9,10-secocholesta-5,7,22-trien-25-carbonsäuremethylester
(7*E*,22*E*)-(1*R*,3*R*,24*S*)-1,3,24-Trihydroxy-19-nor-9,10-secocholesta-5,7,22-trien-25-carbonsäuremethylester
(7*E*,22*E*)-(1*R*,3*R*,24*R*)-1,3,24-Trihydroxy-19-nor-9,10-secocholesta-5,7,22-trien-25-carbonsäureethylester
(7*E*,22*E*)-(1*R*,3*R*,24*S*)-1,3,24-Trihydroxy-19-nor-9,10-secocholesta-5,7,22-trien-25-carbonsäureethylester
(7*E*,22*E*)-(1*R*,3*R*,24*R*)-1,3,24-Trihydroxy-19-nor-9,10-secocholesta-5,7,22-trien-25-carbonsäurepropylester
(7*E*,22*E*)-(1*R*,3*R*,24*S*)-1,3,24-Trihydroxy-19-nor-9,10-secocholesta-5,7,22-trien-25-carbonsäurepropylester
(7*E*,22*E*)-(1*R*,3*R*,24*R*)-1,3,24-Trihydroxy-19-nor-9,10-secocholesta-5,7,22-trien-25-carbonsäure-1-methylethylester
(7*E*,22*E*)-(1*R*,3*R*,24*S*)-1,3,24-Trihydroxy-19-nor-9,10-secocholesta-5,7,22-trien-25-carbonsäure-1-methylethylester
(7*E*,22*E*)-(1*R*,3*R*,24*R*)-1,3,24-Trihydroxy-19-nor-9,10-secocholesta-5,7,22-trien-25-carbonsäurebutylester
(7*E*,22*E*)-(1*R*,3*R*,24*S*)-1,3,24-Trihydroxy-19-nor-9,10-secocholesta-5,7,22-trien-25-carbonsäurebutylester
(7*E*,22*E*)-(1*R*,3*R*,24*R*)-1,3,24-Trihydroxy-19-nor-9,10-secocholesta-5,7,20,22-tetraen-25-carbonsäuremethylester
(7*E*,22*E*)-(1*R*,3*R*,24*S*)-1,3,24-Trihydroxy-19-nor-9,10-secocholesta-5,7,20,22-tetraen-25-carbonsäuremethylester
(7*E*,22*E*)-(1*R*,3*R*,24*R*)-1,3,24-Trihydroxy-19-nor-9,10-secocholesta-5,7,20,22-tetraen-25-carbonsäureethylester
(7*E*,22*E*)-(1*R*,3*R*,24*S*)-1,3,24-Trihydroxy-19-nor-9,10-secocholesta-5,7,20,22-tetraen-25-carbonsäureethylester
(7*E*,22*E*)-(1*R*,3*R*,24*R*)-1,3,24-Trihydroxy-19-nor-9,10-secocholesta-5,7,20,22-tetraen-25-carbonsäurepropylester
(7*E*,22*E*)-(1*R*,3*R*,24*S*)-1,3,24-Trihydroxy-19-nor-9,10-secocholesta-5,7,20,22-tetraen-25-carbonsäurepropylester
(7*E*,22*E*)-(1*R*,3*R*,24*R*)-1,3,24-Trihydroxy-19-nor-9,10-secocholesta-5,7,20,22-tetraen-25-carbonsäure-1-methylethylester
(7*E*,22*E*)-(1*R*,3*R*,24*S*)-1,3,24-Trihydroxy-19-nor-9,10-secocholesta-5,7,20,22-tetraen-25-carbonsäure-1-methylethylester
(7*E*,22*E*)-(1*R*,3*R*,24*R*)-1,3,24-Trihydroxy-19-nor-9,10-secocholesta-5,7,20,22-tetraen-25-carbonsäurebutylester
(7*E*,22*E*)-(1*R*,3*R*,24*S*)-1,3,24-Trihydroxy-19-nor-9,10-secocholesta-5,7,20,22-tetraen-25-carbonsäurebutylester
(7*E*,22*E*)-(1*R*,3*R*)-1,3-Dihydroxy-24-oxo-19-nor-9,10-secocholesta-5,7,22-trien-25-carbonsäuremethylester

(7*E*,22*E*)-(1*R*,3*R*)-1,3-Dihydroxy-24-oxo-19-nor-9,10-secocholesta-5,7,22-trien-25-carbonsäureethylester
(7*E*,22*E*)-(1*R*,3*R*)-1,3-Dihydroxy-24-oxo-19-nor-9,10-secocholesta-5,7,22-trien-25-carbonsäurepropylester
(7*E*,22*E*)-(1*R*,3*R*)-1,3-Dihydroxy-24-oxo-19-nor-9,10-secocholesta-5,7,22-trien-25-carbonsäure-1-methylethylester
(7*E*,22*E*)-(1*R*,3*R*)-1,3-Dihydroxy-24-oxo-19-nor-9,10-secocholesta-5,7,22-trien-25-carbonsäurebutylester
(7*E*,22*E*)-(1*R*,3*R*)-1,3-Dihydroxy-24-oxo-19-nor-9,10-secocholesta-5,7,20,22-tetraen-25-carbonsäuremethylester
(7*E*,22*E*)-(1*R*,3*R*)-1,3-Dihydroxy-24-oxo-19-nor-9,10-secocholesta-5,7,20,22-tetraen-25-carbonsäureethylester
(7*E*,22*E*)-(1*R*,3*R*)-1,3-Dihydroxy-24-oxo-19-nor-9,10-secocholesta-5,7,20,22-tetraen-25-carbonsäurepropylester
(7*E*,22*E*)-(1*R*,3*R*)-1,3-Dihydroxy-24-oxo-19-nor-9,10-secocholesta-5,7,20,22-tetraen-25-carbonsäure-1-methylethylester
(7*E*,22*E*)-(1*R*,3*R*)-1,3-Dihydroxy-24-oxo-19-nor-9,10-secocholesta-5,7,20,22-tetraen-25-carbonsäurebutylester
(7*E*,22*E*)-(1*R*,3*R*,20*S*,24*R*)-20-Fluor-1,3,24-trihydroxy-19-nor-9,10-secocholesta-5,7,22-trien-25-carbonsäureethylester
(7*E*,22*E*)-(1*R*,3*R*,20*S*,24*S*)-20-Fluor-1,3,24-trihydroxy-19-nor-9,10-secocholesta-5,7,22-trien-25-carbonsäureethylester
(7*E*,22*E*)-(1*R*,3*R*,20*R*,24*R*)-20-Fluor-1,3,24-trihydroxy-19-nor-9,10-secocholesta-5,7,22-trien-25-carbonsäureethylester
(7*E*,22*E*)-(1*R*,3*R*,20*R*,24*S*)-20-Fluor-1,3,24-trihydroxy-19-nor-9,10-secocholesta-5,7,22-trien-25-carbonsäureethylester
(7*E*,22*E*)-(1*R*,3*R*,20*S*,24*R*)-20-Fluor-1,3,24-trihydroxy-19-nor-9,10-secocholesta-5,7,22-trien-25-carbonsäure-1-methylethylester
(7*E*,22*E*)-(1*R*,3*R*,20*S*,24*S*)-20-Fluor-1,3,24-trihydroxy-19-nor-9,10-secocholesta-5,7,22-trien-25-carbonsäure-1-methylethylester
(7*E*,22*E*)-(1*R*,3*R*,20*R*,24*R*)-20-Fluor-1,3,24-trihydroxy-19-nor-9,10-secocholesta-5,7,22-trien-25-carbonsäure-1-methylethylester
(7*E*,22*E*)-(1*R*,3*R*,20*R*,24*S*)-20-Fluor-1,3,24-trihydroxy-19-nor-9,10-secocholesta-5,7,22-trien-25-carbonsäureethyl-1-methylester
(5*Z*,7*E*,22*E*)-(1*S*,3*R*,24*R*)-1,3,24-Trihydroxy-9,10-secocholesta-5,7,10(19),22-tetraen-25-carbonsäurebutylester
(5*Z*,7*E*,22*E*)-(1*S*,3*R*,24*S*)-1,3,24-Trihydroxy-9,10-secocholesta-5,7,10(19),22-tetraen-25-carbonsäurebutylester
(5*Z*,7*E*,22*E*)-(1*S*,3*R*,24*R*)-1,3,24-Trihydroxy-9,10-secocholesta-5,7,10(19),22-tetraen-25-carbonsäure-2-methylpropylester
(5*Z*,7*E*,22*E*)-(1*S*,3*R*,24*S*)-1,3,24-Trihydroxy-9,10-secocholesta-5,7,10(19),22-tetraen-25-carbonsäure-2-methylpropylester
(5*Z*,7*E*,22*E*)-(1*S*,3*R*,24*R*)-1,3,24-Trihydroxy-9,10-secocholesta-5,7,10(19),22-tetraen-25-carbonsäurepentylester
(5*Z*,7*E*,22*E*)-(1*S*,3*R*,24*S*)-1,3,24-Trihydroxy-9,10-secocholesta-5,7,10(19),22-tetraen-25-carbonsäurepentylester
(5*Z*,7*E*,22*E*)-(1*S*,3*R*)-1,3-Dihydroxy-24-oxo-9,10-secocholesta-5,7,10(19),22-tetraen-25-carbonsäuremethylester
(5*Z*,7*E*,22*E*)-(1*S*,3*R*)-1,3-Dihydroxy-24-oxo-9,10-secocholesta-5,7,10(19),22-tetraen-25-carbonsäureethylester
(5*Z*,7*E*,22*E*)-(1*S*,3*R*)-1,3-Dihydroxy-24-oxo-9,10-secocholesta-5,7,10(19),22-tetraen-25-carbonsäurepropylester
(5*Z*,7*E*,22*E*)-(1*S*,3*R*)-1,3-Dihydroxy-24-oxo-9,10-secocholesta-5,7,10(19),22-tetraen-25-carbonsäure-1-methylethylester
(5*Z*,7*E*,22*E*)-(1*S*,3*R*)-1,3-Dihydroxy-24-oxo-9,10-secocholesta-5,7,10(19),22-tetraen-25-carbonsäurebutylester
(5*Z*,7*E*,22*E*)-(1*S*,3*R*)-1,3-Dihydroxy-24-oxo-9,10-secocholesta-5,7,10(19),22-tetraen-25-carbonsäure-2-methylpropylester
(5*Z*,7*E*,22*E*)-(1*S*,3*R*)-1,3-Dihydroxy-24-oxo-9,10-secocholesta-5,7,10(19),22-tetraen-25-carbonsäurepentylester
(5*Z*,7*E*,22*E*)-(1*S*,3*R*)-1,3-Dihydroxy-20-methyl-24-oxo-9,10-secocholesta-5,7,10(19),22-tetraen-25-carbonsäureethylester
(5*Z*,7*E*,22*E*)-(1*S*,3*R*)-1,3-Dihydroxy-20-methyl-24-oxo-9,10-secocholesta-5,7,10(19),22-tetraen-25-carbonsäure-1-methylethylester
(5*Z*,7*E*,22*E*)-(1*S*,3*R*,24*R*)-1,3,24-Trihydroxy-26a,27-cyclo-26a-homo-9,10-secocholesta-5,7,10(19),22-tetraen-25-carbonsäureethylester
(5*Z*,7*E*,22*E*)-(1*S*,3*R*,24*S*)-1,3,24-Trihydroxy-26a,27-cyclo-26a-homo-9,10-secocholesta-5,7,10(19),22-tetraen-25-carbonsäureethylester
(5*Z*,7*E*,22*E*)-(1*S*,3*R*,24*R*)-1,3,24-Trihydroxy-26a,27-cyclo-26a-homo-10-secocholesta-5,7,10(19),22-tetraen-25-carbonsäure-1-methylethylester
(5*Z*,7*E*,22*E*)-(1*S*,3*R*,24*S*)-1,3,24-Trihydroxy-26a,27-cyclo-26a-homo-9,10-secocholesta-5,7,10(19),22-tetraen-25-carbonsäure-1-methylethylester

**23.** Pharmazeutische Präparate enthaltend mindestens eine Verbindung der allgemeinen Formel I gemäß einem der Ansprüche 1 bis 22 sowie einen pharmazeutisch verträglichen Träger.

**24.** Verwendung der Verbindungen der allgemeinen Formel I gemäß den Ansprüchen 1 bis 22 zur Herstellung von Arzneimitteln.

## Claims

**1.** 25-Carboxylic acid derivatives of the general formula I

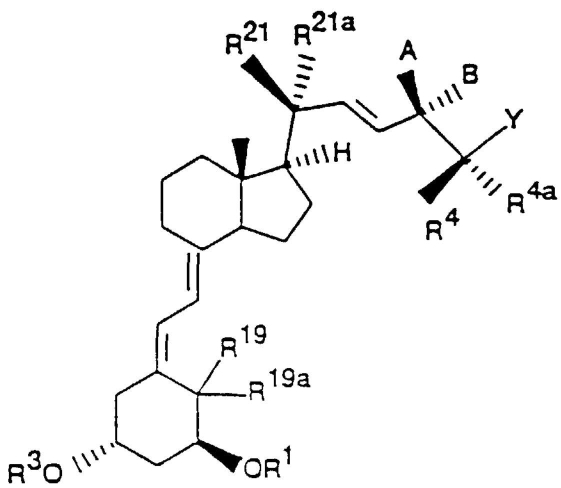

(I)

in which

$R^1$ and $R^3$ each independently of the other represents a hydrogen atom, a straight-or branched-chained saturated alkanoyl group having from 1 to 9 carbon atoms, or an aroyl group,
$R^{19}$ and $R^{19a}$ each represents a hydrogen atom or together represent an exocyclic methylene group,
A and B together represent a keto oxygen atom, or A represents a group $OR^{24}$ and
B represents a hydrogen atom, or A represents a hydrogen atom and B represents a group $OR^{24}$, wherein $R^{24}$ is a hydrogen atom or a straight- or branched-chained saturated alkanoyl group having up to 9 carbon atoms, or an aroyl group,
$R^{21}$ and $R^{21a}$ independently of each other represent a hydrogen atom, a chlorine or fluorine atom or an alkyl group having from 1 to 4 carbon atoms, or together represent a methylene group, or, together with carbon atom 20, form a 3- to 7-membered, saturated or unsaturated carbocyclic ring,
$R^4$ and $R^{4a}$ simultaneously each represents a hydrogen atom, a chlorine or fluorine atom, a trifluoromethyl group, or a straight- or branched-chained, saturated or unsaturated hydrocarbon radical having up to 4 carbon atoms, or $R^4$ and $R^{4a}$, together with carbon atom 25, form a 3- to 7-membered, saturated or unsaturated carbocyclic ring, and

Y represents one of the radicals $-C(O)NR^5R^{5'}$, $-C(O)OR^6$ and -CN, wherein $R^5$ and $R^{5'}$, independently of each other, and $R^6$ each represents a hydrogen atom or a linear or branched alkyl group having up to 8 carbon atoms, and $R^6$ additionally represents an unsaturated, linear or branched hydrocarbon radical having from 3 to 8 carbon atoms or represents the group $-(CH_2)_m-CH(CH_2)_n$, wherein m = 0 or 1 and n = 2, 3, 4, 5 or 6 and, when m = 1, additionally n = 1.

**2.** Compounds of the general formula I according to claim 1, in which $R^1$, $R^3$ and $R^{24}$ each represents a hydrogen atom.

**3.** Compounds of the general formula I according to claim 1, in which $R^1$, $R^3$ and $R^{24}$ each represents an acetyl, propionyl, n-butyryl, isobutyryl, pivaloyl or valeryl radical.

**4.** Compounds of the general formula I according to claim 1, in which $R^1$, $R^3$ and $R^{24}$ each represents a benzoyl radical.

**5.** Compounds of the general formula I according to claim 1, in which $R^4$ and $R^{4a}$ each represents a methyl, ethyl, n-propyl, n-butyl, isopropyl, isobutyl or tert.-butyl radical.

**6.** Compounds of the general formula I according to claim 1, in which $R^{21}$ represents a hydrogen atom and $R^{21a}$ represents a methyl group.

**7.** Compounds of the general formula I according to claim 1, in which the substituent $R^{21}$ or $R^{21a}$ represents a methyl, ethyl, n-propyl, n-butyl, isopropyl, isobutyl or tert.-butyl radical and the other substituent represents a methyl group.

**8.** Compounds of the general formula I according to claim 1, in which $R^{21}$ and $R^{21a}$, together with carbon atom 20, form a cyclopropyl ring.

**9.** Compounds of the general formula I according to claim 1, in which $R^{21}$ and $R^{21a}$ together represent a methylene group.

**10.** Compounds of the general formula I according to claim 1, in which $R^{21}$ represents a fluorine atom and $R^{21a}$ represents a methyl group, or $R^{21}$ represents a methyl group and $R^{21a}$ represents a fluorine atom.

**11.** Compounds of the general formula I according to claim 1, in which $R^4$ and $R^{4a}$ each represents a methyl or ethyl group, or $R^4$ and $R^{4a}$, together with tertiary carbon atom 25, form a cyclopropyl, cyclobutyl, cyclopentyl or cyclohexyl ring.

**12.** Compounds of the general formula I according to claim 1, in which $R^5$, $R^5$ and/or $R^6$ is/are a methyl, ethyl, n-propyl, n-butyl, isopropyl, isobutyl or tert.-butyl group.

**13.** Compounds of the general formula I according to claim 1, in which $R^{19}$ and $R^{19a}$ each represents a hydrogen atom.

**14.** Compounds according to claim 13, in which $R^{21}$ represents a hydrogen atom and $R^{21a}$ represents a methyl group.

**15.** Compounds according to claim 13, in which $R^{21}$ and $R^{21a}$ together represent a methylene group.

**16.** Compounds according to claim 13, in which $R^{21}$ and $R^{21a}$ each represents a methyl group.

**17.** Compounds according to claim 13, in which $R^{21}$ represents a hydrogen atom and $R^{21a}$ represents a fluorine atom, or *vice versa.*

**18.** Compounds of the general formula I according to claim 1, in which $R^{19}$ and $R^{19a}$ together represent a methylene group.

**19.** Compounds according to claim 18, in which $R^{21}$ represents a hydrogen atom and $R^{21a}$ represents a methyl group.

**20.** Compounds according to claim 18, in which $R^{21}$ and $R^{21a}$ together represent a methylene group.

**21.** Compounds according to claim 18, in which $R^{21}$ and $R^{21a}$ each represents a methyl group.

**22.** Compounds of the general formula I, namely

(5*Z*,7*E*,22*E*)-(1*S*,3*R*,24*R*)-1,3,24-trihydroxy-9,10-secocholesta-5,7,10(19),22-tetraene-25-carboxylic acid ethyl ester
(5*Z*,7*E*,22*E*)-(1*S*,3*R*,24*R*)-26,27-dimethyl-1,3,24-trihydroxy-9,10-secocholesta-5,7,10(19),22-tetraene-25-carboxylic acid methyl ester
(5*Z*,7*E*,22*E*)-(1*S*,3*R*,24*R*)-1,3,24-trihydroxy-9,10-secocholesta-5,7,10(19),22-tetraene-25-carboxylic acid 1-methylethylester
(5Z,7*E*,22*E*)-(1*S*,3*R*,24*R*)-1,3,24-trihydroxy-9,10-secocholesta-5,7,10(19),22-tetraene-25-carboxylic acid methyl ester
(5*Z*,7*E*,22*E*)-(1*S*,3*R*,24*S*)-1,3,24-trihydroxy-9,10-secochola-5,7,10(19),22-tetraene-24-acetic acid methyl ester
(5*Z*,7*E*,22*E*)-(1*S*,3*R*,24*R*)-1,3,24-trihydroxy-9,10-secocholesta-5,7,10(19),22-tetraene-25-carboxylic acid nitrile
(5*Z*,7*E*,22*E*)-(1*S*,3R,24R)-1,3,24-trihydroxy-9,10-secocholesta-5,7,10(19),22-tetraene-25-carboxylic acid propyl ester
(5*Z*,7*E*,22*E*)-(1*S*,3*R*,24*R*)-1,3,24-trihydroxy-9,10-secocholesta-5,7,10(19)22-tetraene-25-carboxylic acid
(5*Z*,7*E*,22*E*)-(1*S*,3*R*,24*R*)-1,3,24-trihydroxy-26,27-cyclo-9,10-secocholesta-5,7,10(1 9),22-tetraene-25-carboxylic acid methyl ester
(5*Z*,7*E*,22*E*)-(1*S*,3*R*,24*R*)-1,3,24-trihydroxy-26,27-cydo-9,10-secocholesta-5,7,10(19),22-tetraene-25-carboxylic acid ethyl ester
(5*Z*,7*E*,22*E*)-(1*S*,3*R*,24*R*)-1,3,24-trihydroxy-26,27-cyclo-9,10-secocholesta-5,7,10(19),22-tetraene-25-carboxylic acid propyl ester
(5*Z*,7*E*,22*E*)-(1*S*,3*R*,24*R*)-1,3,24-trihydroxy-26,27-cyclo-9,10-secocholesta-5,7,10(19),22-tetraene-25-carboxylic acid butyl ester
(5*Z*,7*E*,22*E*)-(1*S*,3*R*,24*R*)-1,3,24-trihydroxy-26,27-cyclo-9,10-secocholesta-5,7,10(19),22-tetraene-25-carboxylic acid 2-methylpropyl ester
(5*Z*,7*E*,22*E*)-(1*S*,3*R*,24*R*)-1,3,24-trihydroxy-26,27-cyclo-9,10-secocholesta-5,7,10(19),22-tetraene-25-carboxylic acid pentyl ester
(5*Z*,7*E*,22*E*)-(1*S*,3*R*,24*R*)-1,3,24-trihydroxy-26,27-cyclo-9,10-secocholesta-5,7,10(19),22-tetraene-25-carboxylic acid hexyl ester
(5*Z*,7*E*,22*E*)-(1*S*,3*R*,24*R*)-1,3,24-trihydroxy-9,10-secocholesta-5,7,10(19),22-tetraene-25-carboxylic acid dimethylamide
(5*Z*,7*E*,22*E*)-(1*S*,3*R*,24*R*)-1,3,24-trihydroxy-9,10-secocholesta-5,7,10(19),22-tetraene-25-carboxylic acid diethylamide
(5*Z*,7*E*,22*E*)-(1*S*,3*R*,24*R*)-1,3,24-trihydroxy-26,27-cyclo-9,10-secocholesta-5,7,10(1 9),22-tetraene-25-carboxylic acid diethylamide
(5*Z*,7*E*,22*E*)-(1*S*,3*R*)-1,3-dihydroxy-24-oxo-26,27-cyclo-9,10-secocholesta-5,7,10(19),22-tetraene-25-carboxylic acid n-butylamide
(5*Z*,7*E*,22*E*)-(1*S*,3*R*,24*R*)-20-methyl-1,3,24-trihydroxy-9,10-secocholesta-5,7,10(19),22-tetraene-25-carboxylic acid methyl ester
(5*Z*,7*E*,22*E*)-(1*S*,3*R*,24*S*)-20-methyl-1,3,24-trihydroxy-9,10-secocholesta-5,7,10(19),22-tetraene-25-carboxylic acid methyl ester
(5*Z*,7*E*,22*E*)-(1*S*,3*R*,24*R*)-20-methyl-1,3,24-trihydroxy-9,10-secocholesta-5,7,10(19),22-tetraene-25-carboxylic acid ethyl ester
(5*Z*,7*E*,22*E*)-(1*S*,3*R*,24*S*)-20-methyl-1,3,24-trihydroxy-9,10-secocholesta-5,7,10(19),22-tetraene-25-carboxylic acid ethyl ester
(5*Z*,7*E*,22*E*)-(1*S*,3*R*,24*R*)-20-methyl-1,3,24-trihydroxy-9,10-secocholesta-5,7,10(19),22-tetraene-25-carboxylic acid propyl ester
(5*Z*,7*E*,22*E*)-(1*S*,3*R*,24*S*)-20-methyl-1,3,24-trihydroxy-9,10-secocholesta-5,7,10(19),22-tetraene-25-carboxylic acid propyl ester
(5*Z*,7*E*,22*E*)-(1*S*,3*R*,24*R*)-20-methyl-1,3,24-trihydroxy-9,10-secocholesta-5,7,10(19),22-tetraene-25-carboxylic acid 1-methylethylester
(5*Z*,7*E*,22*E*)-(1*S*,3*R*,24*S*)-20-methyl-1,3,24-trihydroxy-9,10-secocholesta-5,7,10(19),22-tetraene-25-carboxylicacid 1-methylethylester
(5*Z*,7*E*,22*E*)-(1*S*,3*R*,24*R*)-20-methyl-1,3,24-trihydroxy-9,10-secocholesta-5,7,10(19),22-tetraene-25-carboxylic acid butyl ester
(5*Z*,7*E*,22*E*)-(1*S*,3*R*,24*S*)-20-methyl-1,3,24-trihydroxy-9,10-secocholesta-5,7,10(19),22-tetraene-25-carboxylic acid butyl ester
(5*Z*,7*E*,22*E*)-(1*S*,3*R*,24*R*)-1,3,24-trihydroxy-9,10-secccholesta-5,7,10(19),20,22-pentaene-25-carboxylic acid methyl ester
(5*Z*,7*E*,22*E*)-(1*S*,3*R*,24*S*)-1,3,24-trihydroxy-9,10-secocholesta-5,7,10(19),20,22-pentaene-25-carboxylic acid methyl ester
(5*Z*,7*E*,22*E*)-(1*S*,3*R*,24*R*)-1,3,24-trihydroxy-9,10-secocholesta-5,7,10(19),20,22-pentaene-25-carboxylic acid ethyl ester
(5*Z*,7*E*,22*E*)-(1*S*,3*R*,24*S*)-1,3,24-trihydroxy-9,10-secocholesta-5,7,10(19),20,22-pentaene-25-carboxylic acid ethyl ester
(5*Z*,7*E*,22*E*)-(1*S*,3*R*,24*R*)-1,3,24-trihydroxy-9,10-secocholesta-5,7,10(19),20,22-pentaene-25-carboxylic acid propyl ester
(5*Z*,7*E*,22*E*)-(1*S*,3*R*,24*S*)-1,3,24-trihydroxy-9,10-secocholesta-5,7,10(19),20,22-pentaene-25-carboxylic acid propyl ester
(5*Z*,7*E*,22*E*)-(1*S*,3*R*,24*R*)-1,3,24-trihydroxy-9,10-secocholesta-5,7,10(19),20,22-pentaene-25-carboxylic acid 1-methylethyl ester
(5*Z*,7*E*,22*E*)-(1*S*,3*R*,24*S*)-1,3,24-trihydroxy-9,10-secocholesta-5,7,10(19),20,22-pentaene-25-carboxylic acid 1-methylethyl ester
(5*Z*,7*E*,22*E*)-(1*S*,3*R*,24*R*)-1,3,24-trihydroxy-9,10-secocholesta-5,7,10(19),20,22-pentaene-25-carboxylic acid butyl ester
(5*Z*,7*E*,22*E*)-(1*S*,3*R*,24*S*)-1,3,24-trihydroxy-9,1 0-secocholesta-5,7,10(19),20,22-pentaene-25-carboxylic acid butyl ester
(5*Z*,7*E*,22*E*)-(1*S*,3*R*,24*R*)-20,21-methylene-1,3,24-trihydroxy-9,10-secocholesta-5,7,10(19),22-tetraene-25-carboxylic acid methyl ester
(5*Z*,7*E*,22*E*)-(1*S*,3*R*,24*S*)-20,21-methylene-1,3,24-trihydroxy-9,10-secocholesta-5,7,10(19),22-tetraene-25-carboxylic acid methyl ester
(5*Z*,7*E*,22*E*)-(1S,3R,24R)-20,21-methylene-1,3,24-trihydroxy-9,10-secocholesta-5,7,10(19),22-tetraene-25-carboxylic acid ethyl ester
(5*Z*,7*E*,22*E*)-(1*S*,3R,24S)-20,21-methylene-1,3,24-trihydroxy-9,10-secocholesta-5,7,10(19),22-tetraene-25-carboxylic acid ethyl ester
(5*Z*,7*E*,22*E*)-(1*S*,3*R*,24*R*)-20,21-methylene-1,3,24-trihydroxy-9,10-secocholesta-5,7,10(19),22-tetraene-25-carboxylic acid propyl ester
(5*Z*,7*E*,22*E*)-(1*S*,3*R*,24*S*)-20,21-methylene-1,3,24-trihydroxy-9,10-secocholesta-5,7,10(19),22-tetraene-25-carboxylic acid propyl ester
(5*Z*,7*E*,22*E*)-(1*S*,3*R*,24*R*)-20,21-methylene-1,3,24-trihydroxy-9,10-secocholesta-5,7,10(19),22-tetraene-25-carboxylic acid1-methylethylester
(5*Z*,7*E*,22*E*)-(1*S*,3*R*,24*S*)-20,21-methylene-1,3,24-trihydroxy-9,10-secocholesta-5,7,10(19),22-tetraene-25-carboxylic acid 1-methylethyl ester
(5*Z*,7*E*,22*E*)-(1*S*,3*R*,24*R*)-20,21-methylene-1,3,24-trihydroxy-9,10-secocholesta-5,7,10(19),22-tetraene-25-carboxylic acid butyl ester
(5*Z*,7*E*,22*E*)-(1*S*,3*R*,24*S*)-20,21-methylene-1,,3,24-trihydroxy-9,10-secocholesta-5,7,10(19),22-tetraene-25-carboxylic acid butyl ester
(7*E*,22*E*)-(1*R*,3*R*,24*R*)-1,3,24-trihydroxy-19-nor-9,10-secocholesta-5,7,22-triene-25-carboxylic acid methyl ester
(7*E*,22*E*)-(1*R*,3*R*,24*S*)-1,3,24-trihydroxy-19-nor-9,10-secocholesta-5,7,22-triene-25-carboxylic acid methyl ester
(7*E*,22*E*)-(1*R*,3*R*,24*R*)-1,3,24-trihydroxy-19-nor-9,10-secocholesta-5,7,22-triene-25-carboxylic acid ethyl ester
(7*E*,22*E*)-(1*R*,3*R*,24*S*)-1,3,24-trihydroxy-19-nor-9,10-secocholesta-5,7,22-triene-25-carboxylic acid ethyl ester
(7*E*,22*E*)-(1*R*,3*R*,24*R*)-1,3,24-trihydroxy-19-nor-9,10-secocholesta-5,7,22-triene-25-carboxylic acid propyl ester
(7*E*,22*E*)-(1*R*,3*R*,24*S*)-1,3,24-trihydroxy-19-nor-9,10-secocholesta-5,7,22-triene-25-carboxylic acid propyl ester
(7*E*,22*E*)-(1*R*,3*R*,24*R*)-1,3,24-trihydroxy-19-nor-9,10-secocholesta-5,7,22-triene-25-carboxylic acid 1-methylethyl ester
(7*E*,22*E*)-(1*R*,3*R*,24*S*)-1,3,24-trihydroxy-1 9-nor-9,10-secocholesta-5,7,22-triene-25-carboxylic acid 1-methylethyl ester
(7*E*,22*E*)-(1*R*,3*R*,24*R*)-1,3,24-trihydroxy-19-nor-9,10-secocholesta-5,7,22-triene-25-carboxylic acid butyl ester
(7*E*,22*E*)-(1*R*,3*R*,24*S*)-1,3,24-trihydroxy-19-nor-9,10-secocholesta-5,7,22-triene-25-carboxylic acid butyl ester
(7*E*,22*E*)-(1*R*,3*R*,24*R*)-1,3,24-trihydroxy-19-nor-9,10-secocholesta-5,7,20,22-tetraene-25-carboxylic acid methyl ester
(7*E*,22*E*)-(1 *R*,3*R*,24*S*)-1,3,24-trihydroxy-19-nor-9,1 0-secocholesta-5,7,20,22-tetraene-25-carboxylic acid methyl ester
(7*E*,22*E*)-(1*R*,3*R*,24*R*)-1,3,24-trihydroxy-19-nor-9,10-secocholesta-5,7,20,22-tetraene-25-carboxylic acid ethyl ester
(7*E*,22*E*)-(1*R*,3*R*,24*S*)-1,3,24-trihydroxy-19-nor-9,10-secocholesta-5,7,20,22-tetraene-25-carboxylic acid ethyl ester
(7*E*,22*E*)-(1*R*,3*R*,24*R*)-1,3,24-trihydroxy-19-nor-9,10-secocholesta-5,7,20,22-tetraene-25-carboxylic acid propyl ester
(7*E*,22*E*)-(1*R*,3*R*,24*S*)-1,3,24-trihydroxy-19-nor-9,10-secocholesta-5,7,20,22-tetraene-25-carboxylic acid propyl ester
(7*E*,22*E*)-(1*R*,3*R*,24*R*)-1,3,24-trihydroxy-19-nor-9,10-secocholesta-5,7,20,22-tetraene-25-carboxylic acid 1-methylethyl ester
(7*E*,22*E*)-(1*R*,3*R*,24*S*)-1,3,24-trihydroxy-19-nor-9,10-secocholesta-5,7,20,22-tetraene-25-carboxylic acid 1-methylethyl ester
(7*E*,22*E*)-(1*R*,3*R*,24*R*)-1,3,24-trihydroxy-19-nor-9,10-secocholesta-5,7,20,22-tetraene-25-carboxylic acid butyl ester
(7*E*,22*E*)-(1*R*,3*R*,24*S*)-1,3,24-trihydroxy-19-nor-9,10-secocholesta-5,7,20,22-tetraene-25-carboxylic acid butyl ester
(7*E*,22*E*)-(1*R*,3*R*)-1,3-dihydroxy-24-oxo-19-nor-9,10-secocholesta-5,7,22-triene-25-carboxylic acid methyl ester
(7*E*,22*E*)-(1*R*,3*R*)-1,3-dihydroxy-24-oxo-9-nor-9,10-secocholesta-5,7,22-triene-25-carboxylic acid ethyl ester
(7*E*,22*E*)-(1*R*,3*R*)-1,3-dihydroxy-24-oxo-19-nor-9,10-secocholesta-5,7,22-triene-25-carboxylic acid propyl ester
(7*E*,22*E*)-(1*R*,3*R*)-1,3-dihydroxy-24-oxo-9-nor-9,10-secocholesta-5,7,22-triene-25-carboxylic acid 1-methylethyl ester
(7*E*,22*E*)-(1*R*,3*R*)-1,3-dihydroxy-24-oxo-19-nor-9,10-secocholesta-5,7,22-triene-25-carboxylic acid butyl ester
(7*E*,22*E*)-(1*R*,3*R*)-1,3-dihydroxy-24-oxo-19-nor-9,10-secocholesta-5,7,20,22-tetraene-25-carboxylic acid methyl ester
(7*E*,22*E*)-(1*R*,3*R*)-1,3-dihydroxy-24-oxo-19-nor-9,10-secocholesta-5,7,20,22-tetraene-25-carboxylic acid ethyl ester
(7*E*,22*E*)-(1*R*,3*R*)-1,3-dihydroxy-24-oxo-19-nor-9,10-secocholesta-5,7,20,22-tetraene-25-carboxylic acid propyl ester
(7*E*,22*E*)-(1*R*,3*R*)-1,3-dihydroxy-24-oxo-19-nor-9,10-secocholesta-5,7,20,22-tetraene-25-carboxylic acid 1-methylethyl ester
(7*E*,22*E*)-(1*R*,3*R*)-1,3-dihydroxy-24-oxo-19-nor-9,10-secocholesta-5,7,20,22-tetraene-25-carboxylic acid butyl ester
(7*E*,22*E*)-(1*R*,3*R*,20*S*,24*R*)-20-fluoro-1,3,24-trihydroxy-19-nor-9,10-secocholesta-5,7,22-triene-25-carboxylic acid ethyl ester
(7*E*,22*E*)-(1*R*,3*R*,20*S*,24*S*)-20-fluoro-1,3,24-trihydroxy-19-nor-9,10-secocholesta-5,7,22-triene-25-carboxylic acid ethyl ester
(7*E*,22*E*)-(1*R*,3*R*,20*R*,24*R*)-20-fluoro-1,3,24-trihydroxy-19-nor-9,10-secocholesta-5,7,22-triene-25-carboxylic acid ethyl ester
(7*E*,22*E*)-(1*R*,3*R*,20*R*,24*S*)-20-fluoro-1,3,24-trihydroxy-19-nor-9,10-secocholesta-5,7,22-triene-25-carboxylic acid ethyl ester
(7*E*,22*E*)-(1*R*,3*R*,20*S*,24*R*)-20-fluoro-1,3,24-trihydroxy-19-nor-9,10-secocholesta-5,7,22-triene-25-carboxylic acid 1-methylethyl ester
(7*E*,22*E*)-(1*R*,3*R*,20*S*,24*S*)-20-fluoro-1,3,24-trihydroxy-9-nor-9,10-secocholesta-5,7,22-triene-25-carboxylic acid 1-methylethyl ester
(7*E*,22*E*)-(1*R*,3*R*,20*R*,24*R*)-20-fluoro-1,3,24-trihydroxy-9-nor-9,10-secocholesta-5,7,22-triene-25-carboxylic acid 1-methylethyl ester
(7*E*,22*E*)-(1*R*,3*R*,20*R*,24*S*)-20-fluoro-1,3,24-trihydroxy-19-nor-9,10-secocholesta-5,7,22-triene-25-carboxylic acid ethyl-1-methyl ester

(5*Z*,7*E*,22*E*)-(1*S*,3*R*,24*R*)-1,3,24-trihydroxy-9,10-secocholesta-5,7,10(19),22-tetraene-25-carboxylic acid butyl ester
(5*Z*,7*E*,22*E*)-(1*S*,3*R*,24*S*)-1,3,24-trihydroxy-9,10-secocholesta-5,7,10(19),22-tetraene-25-carboxylic acid butyl ester
(5*Z*,7*E*,22*E*)-(1*S*,3*R*,24*R*)-1,3,24-trihydroxy-9,10-secocholesta-5,7,10(19),22-tetraene-25-carboxylic acid 2-methylpropyl ester
(5*Z*,7*E*,22*E*)-(1*S*,3*R*,24*S*)-1,3,24-trihydroxy-9,10-secocholesta-5,7,10(19),22-tetraene-25-carboxylic acid 2-methylpropyl ester
(5*Z*,7*E*,22*E*)-(1*S*,3*R*,24*R*)-1,3,24-trihydroxy-9,10-secocholesta-5,7,10(19),22-tetraene-25-carboxylic acid pentyl ester
(5*Z*,7*E*,22*E*)-(1*S*,3*R*,24*S*)-1,3,24-trihydroxy-9,10-secocholesta-5,7,10(19)22-tetraene-25-carboxylic acid pentyl ester
(5*Z*,7*E*,22*E*)-(1*S*,3*R*)-1,3-dihydroxy-24-oxo-9,10-secocholesta-5,7,10(19),22-tetraene-25-carboxylic acid methyl ester
(5*Z*,7*E*,22*E*)-(1*S*,3*R*)-1,3-dihydroxy-24-oxo-9,10-secocholesta-5,7,10(19),22-tetraene-25-carboxylic acid ethyl ester
(5*Z*,7*E*,22*E*)-(1*S*,3*R*)-1,3-dihydroxy-24-oxo-9,10-secocholesta-5,7,10(19),22-tetraene-25-carboxylic acid propyl ester
(5*Z*,7*E*,22*E*)-(1*S*,3*R*)-1,3-dihydroxy-24-oxo-9,10-secocholesta-5,7,10(19),22-tetraene-25-carboxylic acid 1-methylethyl ester
(5*Z*,7*E*,22*E*)-(1*S*,3*R*)-1,3-dihydroxy-24-oxo-9,10-secocholesta-5,7,10(19),22-tetraene-25-carboxylic acid butyl ester
(5*Z*,7*E*,22*E*)-(1*S*,3*R*)-1,3-dihydroxy-24-oxo-9,10-secocholesta-5,7,10(19)22-tetraene-25-carboxylic acid 2-methylpropyl ester
(5*Z*,7*E*,22*E*)-(1*S*,3*R*)-1,3-dihydroxy-24-oxo-9,10-secocholesta-5,7,10(19)22-tetraene-25-carboxylic acid pentyl ester
(5*Z*,7*E*,22*E*)-(1*S*,3*R*)-1,3-dihydroxy-20-methyl-24-oxo-9,10-secocholesta-5,7,10(19),22-tetraene-25-carboxylic acid ethyl ester
(5*Z*,7*E*,22*E*)-(1*S*,3*R*)-1,3-dihydroxy-20-methyl-24-oxo-9,10-secocholesta-5,7,10(19),22-tetraene-25-carboxylic acid 1-methylethyl ester
(5*Z*,7*E*,22*E*)-(1*S*,3*R*,24*R*)-1,3,24-trihydroxy-26a,27-cyclo-26a-homo-9,10-secocholesta-5,7,10(19), 22-tetraene-25-carboxylic acid ethyl ester
(5*Z*,7*E*,22*E*)-(1*S*,3*R*,24*S*)-1,3,24-trihydroxy-26a,27-cyclo-26a-homo-9,10-secocholesta-5,7,10(19), 22-tetraene-25-carboxylic acid ethyl ester
(5*Z*,7*E*,22*E*)-(1*S*,3*R*,24*R*)-1,3,24-trihydroxy-26a,27-cyclo-26a-homo-9,10-secocholesta-5,7,10(19), 22-tetraene-25-carboxylic acid 1-methylethyl ester
(5*Z*,7*E*,22*E*)-(1*S*,3*R*,24*S*)-1,3,24-trihydroxy-26a,27-cyclo-26a-homo-9,10-secocholesta-5,7,10(19), 22-tetraene-25-carboxylicacid 1-methylethyl ester.

**23.** Pharmaceutical preparations comprising at least one compound of the general formula I according to any one of claims 1 to 22 as well as a pharmaceutically acceptable carrier.

**24.** Use of the compounds of the general formula I according to claims 1 to 22 in the preparation of medicaments.

**Revendications**

**1.** Dérivés d'acide 25-carboxylique de formule générale I

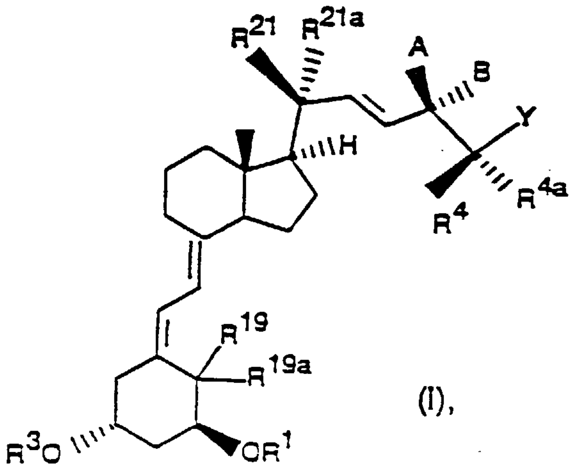

(I),

où $R^1$ et $R^3$, indépendamment l'un de l'autre, chacun représente un atome d'hydrogène, un groupe alcanoyle linéaire ou ramifié, saturé, avec 1 à 9 atomes de carbone, ou un groupe aroyle,
$R^{19}$ et $R^{19a}$, chacun, représente un atome d'hydrogène ou ensemble, un groupe méthylène exocyclique,
A et B ensemble, représentent un atome d'oxygène cétonique ou A représente un groupe $OR^{24}$ et B un atome d'hydrogène ou A représente un atome d'hydrogène et B un groupe $OR^{24}$, $R^{24}$ étant un atome d'hydrogène ou un groupe alcanoyle linéaire ou ramifié, saturé, avec jusqu'à 9 atomes de carbone ou un groupe aroyle,
$R^{21}$ et $R^{21a}$, indépendamment l'un de l'autre, représentent un atome d'hydrogène, un atome de chlore ou de fluor, un groupe alkyle avec 1 à 4 atomes de carbone, ensemble représentent un groupe méthylène, ensemble avec l'atome de carbone 20, représentent un cycle carbocyclique à 3 à 7 chaînons, saturé ou insaturé,
$R^4$ et $R^{4a}$, représente chacun simultanément un atome d'hydrogène, un atome de chlore ou de fluor, un groupe trifluorométhyle, un reste hydrocarboné linéaire ou ramifié, saturé ou insaturé, avec jusqu'à 4 atomes de carbone ou $R^4$ et $R^{4a}$, ensemble avec l'atome de carbone 25, forment un cycle carbocyclique à 3 à 7 chaînons, saturé ou insaturé, ainsi que Y représente les restes $-C(O)NR^5T^{5'}$, $-C(O)OR^6$, -CN, $R^5$ et $R^{5'}$, indépendamment l'un de l'autre, et $RT^6$, représentant chacun un atome d'hydrogène ou un groupe alkyle linéaire ou ramifié avec jusqu'à 8 atomes de carbone, $R^6$ représente de plus un reste hydrocarboné insaturé, linéaire ou ramifié, avec 3 à 8 atomes de carbone ou un groupe $-(CH_2)_m-CH(CH_2)_n$ avec m = 0 ou 1, n = 2, 3, 4, 5 ou 6 et lorsque m = 1, en plus avec n = 1.

2. Composés de formule générale I selon la revendication 1, où $R^1$, $R^3$ et $R^{24}$ représentent chacun un atome d'hydrogène.

3. Composés de formule générale I selon la revendication 1, où $R^1$, $R^3$ et $R^{24}$ représentent chacun un reste acétyle, propionyle, n-butyryle, iso-butyryle, pivaloyle ou valéryle.

4. Composés de formule générale I selon la revendication 1, où $R^1$, $R^3$ et $R^{24}$ représentent chacun un reste bezoyle.

5. Composés de formule générale I selon la revendication 1, où $R^4$ et $R^{4a}$ représentent chacun un reste méthyle, éthyle, n-propyle, n-butyle, 1-propyle, i-butyle ou tert-butyle.

6. Composés de formule générale I selon la revendication 1, où $R^{21}$ représente un atome d'hydrogène et $R^{21a}$ un groupe méthyle.

7. Composés de formule générale I selon la revendication 1, où le substituant $R^{21}$ ou $R^{21a}$ représentente un reste méthyle, éthyle, n-propyle, n-butyle, i-propyle, i-butyle ou tert-butyle, et l'autre substituant représente un groupe méthyle.

8. Composés de formule générale I selon la revendication 1, où $R^{21}$ et $R^{21a}$ ensemble avec l'atome de carbone 20 forment un cycle cyclopropyle.

9. Composés de formule générale I selon la revendication 1, où $R^{21}$ et $R^{21a}$ ensemble représentent un groupe méthylène.

**10.** Composés de formule générale I selon la revendication 1, où $R^{21}$ représente un atome de fluor et $R^{21a}$ un groupe méthyle ou $R^{21}$ représente un groupe méthyle et $R^{21a}$ un atome de fluor.

**11.** Composés de formule générale I selon la revendication 1, où $R^4$ et $R^{4a}$ chacun représente on groupe méthyle ou éthyle ou ensemble avec l'atome de carbone tertiaire 25, forment un cycle cyclopropyle, cyclobutyle, cyclopentyle ou cyclohexyle.

**12.** Composés de formule générale I selon la revendication 1, où $R^5$, $R^{5'}$ et/ou $R^6$ représentent un groupe méthyle, éthyle, n-propyle, n-butyle, i-propyle, i-butyle ou tert-butyle.

**13.** Composés de formule générale I selon la revendication 1, où $R^{19}$ et $R^{19a}$ chacun représentent un atome d'hydrogène.

**14.** Composés selon la revendication 13, où $R^{21}$ représente un atome d'hydrogène et $R^{21a}$ un groupe méthyle.

**15.** Composés selon la revendication 13, où $R^{21}$ et $R^{21a}$ représentent ensemble un groupe méthylène.

**16.** Composés selon la revendication 13, où $R^{21}$ et $R^{21a}$ représentent chacun un groupe méthyle.

**17.** Composés sselon la revendication 13, où $R^{21}$ représente un atome d'hydrogène et $R^{21a}$ un atome de fluor ou inversément.

**18.** Composés de formule générale I selon la revendication 1, où $R^{19}$ et $R^{19a}$ ensemble représentent un groupe méthylène.

**19.** Composés selon la revendication 18, où $R^{21}$ représente un atome d'hydrogène et $R^{21a}$ un groupe méthyle.

**20.** Composés selon la revendication 18, où $R^{21}$ et $R^{21a}$ ensemble, représentent un groupe méthylène.

**21.** Composés selon la revendication 18, où $R^{21}$ et $R^{21a}$ chacun représente un groupe méthyle.

**22.** Composés de formule générale I, à savoir

*(5Z,7E,22E)-(1S,3R,24R)*-1,3,24-trihydroxy-9,10-secocholesta-5,7,10(19)22-tétraène-25-carboxylate d'éthyle
*(5Z,7E,22E)-(1S,3R,24R)*-26,27-diméthyl-1,3,24-trihydroxy-9,10-seco-cholesta-5,7,10(19)22-tétraène-25-carboxylate de méthyle
*(5Z,7E,22E)-(1S,3R,24R)*-1,3,24-trihydroxy-9,10-secocholesta-5,7,10(19)22-tétraène-25-carboxylate de 1-méthyléthyle
*(5Z,7E,22E)-(1S,3R,24R)*-1,3,24-trihydroxy-9,10-secocholesta-5,7,10(19)22-tétraène-25-carboxylate de méthyléthyle
*(5Z,7E,22E)-(1S,3R,24R)*-1,3,24-trihydroxy-9,10-secocholesta-5,7,10(19)22-tétraène-24-acétate de méthyle
nitrile de l'acide *(5Z,7E,22E)-(1S,3R,24R)*-1,3,24-trihydroxy-9,10-secocholesta-5,7,10 (19)22-tétraène-25-carboxylique
*(5Z,7E,22E)-(1S,3R,24R)*-1,3,24-trihydroxy-9,10-secocholesta-5,7,10(19)22-tétraène-25-carboxylate de propyle
acide*(5Z,7E,22E)-(1S,3R,24R)*-1,3,24-trihydroxy-9,10-seco-cholesta-5,7,10(19)22-tétraène-25-carboxylique
*(5Z,7E,22E)-(1S,3R,24R)*-1,3,24-trihydroxy-26,27-cyclo-9,10-secocholesta-5,7,10(19)22-tétraène-25-carboxylate de méthyle
*(5Z,7E,22E)-(1S,3R,24R)*-1,3,24-trihydroxy-26,27-cyclo-9,10-secocholesta-5,7,10 (19)22-tétraène-25-carboxylate d'éthyle
*(5Z,7E,22E)-(1S,3R,24R)*-1,3,24-trihydroxy-26,27-cyclo-9,10-secocholesta-5,7,10(19)22-tétraène-25-carboxylate de propyle
*(5Z,7E,22E)-(1S,3R,24R)*-1,3,24-trihydroxy-26,27-cyclo-9,10-secocholesta-5,7,10(19)22-tétraène-25-carboxylate de butyle
*(5Z,7E,22E)-(1S,3R,24R)*-1,3,24-trihydroxy-26,27-cyclo-9,10-secocholesta-5,7,10(19)22-tétraène-25-carboxylate de 2-méthylpropyle

*(5Z,7E,22E)-(1S,3R,24R)*-1,3,24-trihydroxy-26,27-cyclo-9,10-secocholesta-5,7,10(19)22-tétraène-25-carboxylate de pentyle
*(5Z,7E,22E)-(1S,3R,24R)*-1,3,24-trihydroxy-26,27-cyclo-9,10-secocholesta-5,7,10(19)22-tétraène-25-carboxylate d'hexyle
diméthylamide de l'acide *(5Z,7E,22E)-(1S,3R,24R)*-1,3,24-trihydroxy-9,10-secocholesta-5,7,10(19)22-tétraène-25-carboxylique
diéthylamide de l'acide *(5Z,7E,22E)-(1S,3R,24R)*-1,3,24-trihydroxy-9,10-secocholesta-5,7,10(19)22-tétraène-25-carboxylique
diéthylamide de l'acide *(5Z,7E,22E)-(1S,3R,24R)*-1,3,24-trihydroxy-26,27-cyclo-9,10-secocholesta-5,7,10(19)22-tétraène-25-carboxylique
n-butylamide de l'acide *(5Z,7E,22E)-(1S,3R)*-1,3-dihydroxy-24-oxo-26,27-cyclo-9,10-secocholesta-5,7,10(19)22-tétraène-25-carboxylique
*(5Z,7E,22E)-(1S,3R,24R)*-20-méthyl-1,3,24-trihydroxy-9,10-secocholesta-5,7,10(19)22-tétraène-25-carboxylate de méthyle
*(5Z,7E,22E)-(1S,3R,24S)*-20-méthyl-1,3,24-trihydroxy-9,10-secocholesta-5,7,10(19)22-tétraène-25-carboxylate de méthyle
*(5Z,7E,22E)-(1S,3R,24R)*-20-méthyl-1,3,24-trihydroxy-9,10-secocholesta-5,7,10(19)22-tétraène-25-carboxylate d'éthyle
*(5Z,7E,22E)-(1S,3R,24S)*-20-méthyl-1,3,24-trihydroxy-9,10-secocholesta-5,7,10(19)22-tétraène-25-carboxylate d'éthyle
*(5Z,7E,22E)-(1S,3R,24R)*-20-méthyl-1,3,24-trihydroxy-9,10-secocholesta-5,7,10(19)22-tétraène-25-carboxylate de propyle
*(5Z,7E,22E)-(1S,3R,24S)*-20-méthyl-1,3,24-trihydroxy-9,10-secocholesta-5,7,10(19)22-tétraène-25-carboxylate de propyle
*(5Z,7E,22E)-(1S,3R,24R)*-20-méthyl-1,3,24-trihydroxy-9,10-secocholesta-5,7,10(19)22-tétraène-25-carboxylate de 1-méthyléthyle
*(5Z,7E,22E)-(1S,3R,24S)*-20-méthyl-1,3,24-trihydroxy-9,10-secocholesta-5,7,10(19)22-tétraène-25-carboxylate de 1-méthyléthyle
*(5Z,7E,22E)-(1S,3R,24R)*-20-méthyl-1,3,24-trihydroxy-9,10-secocholesta-5,7,10(19)22-tétraène-25-carboxylate de butyle
*(5Z,7E,22E)-(1S,3R,24S)*-20-méthyl-1,3,24-trihydroxy-9,10-secocholesta-5,7,10(19)22-tétraène-25-carboxylate de butyle
*(5Z,7E,22E)-(1S,3R,24R)*-1,3,24-trihydroxy-9,10-seco-cholesta-5,7,10(19)20,22-pentaène-25-carboxylate de méthyle
*(5Z,7E,22E)-(1S,3R,24S)*-1,3,24-trihydroxy-9,10-seco-cholesta-5,7,10(19)20,22-pentaène-25-carboxylate de méthyle
*(5Z,7E,22E)-(1S,3R,24R)*-1,3,24-trihydroxy-9,10-secocholesta-5,7,10(19)20,22-pentaène-25-carboxylate d'éthyle
*(5Z,7E,22E)-(1S,3R,24S)* -1,3,24-trihydroxy-9, 10-secocholesta-5,7,10(19)20,22-pentaène-25-carboxylate d'éthyle
*(5Z,7E,22E)-(1S,3R,24R)*-1,3,24-trihydroxy-9,10-secocholesta-5,7,10(19)20,22-pentaène-25-carboxylate de propyle
*(5Z,7E,22E)-(1S,3R,24S)*-1,3,24-trihydroxy-9,10-secocholesta-5,7,10(19)20,22-pentaène-25-carboxylate de propyle
*(5Z,7E,22E)-(1S,3R,24R)*-1,3,24-trihydroxy-9,10-secocholesta-5,7,10(19)20,22-pentaène-25-carboxylate de 1-méthyléthyle
*(5Z,7E,22E)-(1S,3R,24S)*-1,3,24-trihydroxy-9,10-secocholesta-5,7,10(19)20,22-pentaène-25-carboxylate de 1-méthyléthyle
*(5Z,7E,22E)-(1S,3R,24R)*-1,3,24-trihydroxy-9,10-secocholesta-5,7,10(19)20,22-pentaène-25-carboxylate de butyle
*(5Z,7E,22E)-(1S,3R,24S)*-1,3,24-trihydroxy-9,10-secocholesta-5,7,10(19)20,22-pentaène-25-carboxylate de butyle
*(5Z,7E,22E)-(1S,3R,24R)*-20,21-méthylène-1,3,24-trihydroxy-9,10-secocholesta-5,7,10(19)22-tétraène-25-carboxylate de méthyle
*(5Z,7E,22E)-(1S,3R,24S)*-20,21-méthylène-1,3,24-trihydroxy-9,10-secocholesta-5,7,10(19)22-tétraène-25-carboxylate de méthyle
*(5Z,7E,22E)-(1S,3R,24R)*-20,21-méthylène-1,3,24-trihydroxy-9,10-secocholesta-5,7,10(19)22-tétraène-25-carboxylate d'éthyle

*(5Z,7E,22E)-(1S,3R,24S)*-20,21-méthylène-1,3,24-trihydroxy-9,10-secocholesta-5,7,10(19)22-tétraène-25-carboxylate d'éthyle
*(5Z,7E,22E)-(1S,3R,24R)*-20,21-méthylène-1,3,24-trihydroxy-9,10-secocholesta-5,7,10(19)22-tétraène-25-carboxylate de propyle
*(5Z,7E,22E)-(1S,3R,24S)*-20,21-méthylène-1,3,24-trihydroxy-9,10-secocholesta-5,7,10(19)22-tétraène-25-carboxylate de propyle
*(5Z,7E,22E)-(1S,3R,24R)*-20,21-méthylène-1,3,24-trihydroxy-9,10-secocholesta-5,7,10(19)22-tétraène-25-carboxylate de 1-méthyléthyle
*(5Z,7E,22E)-(1S,3R,24S)*-20,21-méthylène-1,3,24-trihydroxy-9,10-secocholesta-5,7,10(19)22-tétraène-25-carboxylate de 1-méthyléthyle
*(5Z,7E,22E)-(1S,3R,24R)*-20,21-méthylène-1,3,24-trihydroxy-9,10-secocholesta-5,7,10(19)22-tétraène-25-carboxylate de butyle
*(5Z,7E,22E)-(1S,3R,24S)*-20,21-méthylène-1,3,24-trihydroxy-9,10-secocholesta-5,7,10(19)22-tétraène-25-carboxylate de butyle
*(7E,22E)-(1R,3R,24R)*-1,3,24-trihydroxy-19-nor-9,10-secocholesta-5,7,22-triène-25-carboxylate de méthyle
*(7E,22E)-(1R,3R,24S)*-1,3,24-trihydroxy-19-nor-9,10-secocholesta-5,7,22-triène-25-carboxylate de méthyle
*(7E,22E)-(1R,3R,24R)*-1,3,24-trihydroxy-19-nor-9,10-secocholesta-5,7,22-triène-25-carboxylate d'éthyle
*(7E,22E)-(1R,3R,24S)*-1,3,24-trihydroxy-19-nor-9,10-secocholesta-5,7,22-triène-25-carboxylate d'éthyle
*(7E,22E)-(1R,3R,24R)*-1,3,24-trihydroxy-19-nor-9,10-secocholesta-5,7,22-triène-25-carboxylate de propyle
*(7E,22E)-(1R,3R,24S)*-1,3,24-trihydroxy-19-nor-9,10-secocholesta-5,7,22-triène-25-carboxylate de propyle
*(7E,22E)-(1R,3R,24R)*-1,3,24-trihydroxy-19-nor-9,10-secocholesta-5,7,22-triène-25-carboxylate de 1-méthyléthyle
*(7E,22E)-(1R,3R,24S)*-1,3,24-trihydroxy-19-nor-9,10-secocholesta-5,7,22-triène-25-carboxylate de 1-méthyl-éthyle
*(7E,22E)-(1R,3R,24R)*-1,3,24-trihydroxy-19-nor-9,10-secocholesta-5,7,22-triène-25-carboxylate de butyle
*(7E,22E)-(1R,3R,24S)*-1,3,24-trihydroxy-19-nor-9,10-secocholesta-5,7,22-triène-25-carboxylate de butyle
*(7E,22E)-(1R,3R,24R)*-1,3,24-trihydroxy-19-nor-9,10-secocholesta-5,7,20,22-tétraène-25-carboxylate de méthyle
*(7E,22E)-(1R,3R,24S)*-1,3,24-trihydroxy-19-nor-9,10-secocholesta-5,7,20,22-tétraène-25-carboxylate de méthyle
*(7E,22E)-(1R,3R,24R)*-1,3,24-trihydroxy-19-nor-9,10-secocholesta-5,7,20,22-tétraène-25-carboxylate d'éthyle
*(7E,22E)-(1R,3R,24S)*-1,3,24-trihydroxy-19-nor-9,10-secocholesta-5,7,20,22-tétraène-25-carboxylate d'éthyle
*(7E,22E)-(1R,3R,24R)*-1,3,24-trihydroxy-19-nor-9,10-secocholesta-5,7,20,22-tétraène-25-carboxylate de propyle
*(7E,22E)-(1R,3R,24S)*-1,3,24-trihydroxy-19-nor-9,10-secocholesta-5,7,20,22-tétraène-25-carboxylate de propyle
*(7E,22E)-(1R,3R,24R)*-1,3,24-trihydroxy-19-nor-9,10-secocholesta-5,7,20,22-tétraène-25-carboxylate de 1-méthyléthyle
*(7E,22E)-(1R,3R,24S)*-1,3,24-trihydroxy-19-nor-9,10-secocholesta-5,7,20,22-tétraène-25-carboxylate de 1-méthyléthyle
*(7E,22E)-(1R,3R,24R)*-1,3,24-trihydroxy-19-nor-9,10-secocholesta-5,7,20,22-tétraène-25-carboxylate de butyle
*(7E,22E)-(1R,3R,24S)*-1,3,24-trihydroxy-19-nor-9,10-secocholesta-5,7,20,22-tétraène-25-carboxylate de butyle
*(7E,22E)-(1R,3R)*-1,3-dihydroxy-24-oxo-19-nor-9,10-seco-cholesta-5,7,22-triène-25-carboxylate de méthyle
*(7E,22E)-(1R,3R)*-1,3-dihydroxy-24-oxo-19-nor-9,10-seco-cholesta-5,7,22-triène-25-carboxylate d'éthyle
*(7E,22E)-(1R,3R)*-1,3-dihydroxy-24-oxo-19-nor-9,10-seco-cholesta-5,7,22-triène-25-carboxylate de propyle
*(7E,22E)-(1R,3R)*-1,3-dihydroxy-24-oxo-19-nor-9,10-seco-cholesta-5,7,22-triène-25-carboxylate de 1-méthyléthyle
*(7E,22E)-(1R,3R)*-1,3-dihydroxy-24-oxo-19-nor-9,10-seco-cholesta-5,7,22-triène-25-carboxylate de butyle
*(7E,22E)-(1R,3R)*-1,3-dihydroxy-24-oxo-19-nor-9,10-seco-cholesta-5,7,20,22-tétraène-25-carboxylate de méthyle
*(7E,22E)-(1R,3R)*-1,3-dihydroxy-24-oxo-19-nor-9,10-seco-cholesta-5,7,20,22-tétraène-25-carboxylate d'éthyle
*(7E,22E)-(1R,3R)*-1,3-dihydroxy-24-oxo-19-nor-9,10-seco-cholesta-5,7,20,22-tétraène-25-carboxylate de propyle

*(7E,22E)-(1R,3R)*-1,3-dihydroxy-24-oxo-19-nor-9,10-seco-cholesta-5,7,20,22-tétraène-2 5-carboxylate de 1-méthyléthyle

*(7E,22E)-(1R,3R)*-1,3-dihydroxy-24-oxo-19-nor-9,10-seco-cholesta-5,7,20,22-tétraène-25-carboxylate de butyle

*(7E,22E)-(1R,3R,205,24R)* -20-fluoro-1,3,24-trihydroxy-19-nor-9,10-secocholesta-5,7,22-triène-25-carboxylate d'éthyle

*(7E,22E)-(1R,3R,20S,24S)*-20-fluoro-1,3,24-trihydroxy-19-nor-9,10-secocholesta-5,7,22-triène-25-carboxylate d'éthyle

*(7E,22E)-(1R,3R,20R,24R)*-20-fluoro-1,3,24-trihydroxy-19-nor-9,10-secocholesta-5,7,22-triène-25-carboxylate d'éthyle

*(7E,22E)-(1R,3R,20R,24S)*-20-fluoro-1,3,24-trihydroxy-19-nor-9,10-secocholesta-5,7,22-triène-25-carboxylate d'éthyle

*(7E,22E)-(1R,3R,20S,24R)*-20-fluoro-1,3,24-trihydroxy-19-nor-9,10-secocholesta-5,7,22-triène-25-carboxylate de 1-méthyléthyle

*(7E,22E)-(1R,3R,20S,24S)*-20-fluoro-1,3,24-trihydroxy-19-nor-9,10-secocholesta-5,7,22-triène-25-carboxylate de 1-méthyléthyle

*(7E,22E)-(1R,3R,20R,24R)*-20-fluoro-1,3,24-trihydroxy-19-nor-9,10-secocholesta-5,7,22-triène-25-carboxylate de 1-méthyléthyle

*(7E,22E)-(1R,3R,20R,24S)*-20-fluoro-1,3,24-trihydroxy-19-nor-9,10-secocholesta-5,7,22-triène-25-carboxylate de 1-méthyléthyle

*(5Z,7E,22E)-(1S,3R,24R)*-1,3,24-trihydroxy-9,10--secocholesta-5,7,10(19)22-tétraène-25-carboxylate de butyle

*(5Z,7E,22E)-(1S,3R,24S)*-1,3,24-trihydroxy-9,10-secocholesta-5,7,10(19)22-tétraène-25-carboxylate de 1-butyle

*(5Z,7E,22E)-(1S,3R,24R)*-1,3,24-trihydroxy-9,10-seco-cholesta-5,7,10(19)22-tétraène-25-carboxylate de 2-méthyl-propyle

*(5Z,7E,22E)-(1S,3R,24S)*-1,3,24-trihydroxy-9,10-seco-cholesta-5,7,10(19)22-tétraène-25-carboxylate de 2-méthyl-propyle

*(5Z,7E,22E)-(1S,3R,24R)*-1,3,24-trihydroxy-9,10-seco-cholesta-5,7,10(19)22-tétraène-25-carboxylate de pentyle

*(5Z,7E,22E)-(1S,3R,24S)*-1,3,24-trihydroxy-9,10-seco-cholesta-5,7,10(19)22-tétraène-25-carboxylate de pentyle

*(5Z,7E,22E)-(1S,3R)*-1,3-dihydroxy-24-oxo-9,10-seco-cholesta-5,7,10(19)22-tétraène-25-carboxylate de méthyle

*(5Z,7E,22E)-(1S,3R)*-1,3-dihydroxy-24-oxo-9,10-seco-cholesta-5,7,10(19)22-tétraène-25-carboxylate d'éthyle

*(5Z,7E,22E)-(1S,3R)*-1,3-dihydroxy-24-oxo-9,10-seco-cholesta-5,7,10(19)22-tétraène-25-carboxylate de propyle

*(5Z,7E,22E)-(1S,3R)*-1,3-dihydroxy-24-oxo-9,10-seco-cholesta-5,7,10(19)22-tétraène-25-carboxylate de 1-méthyléthyle

*(5Z,7E,22E)-(1S,3R)*-1,3-dihydroxy-24-oxo-9,10-seco-cholesta-5,7,10(19)22-tétraène-25-carboxylate de butyle

*(5Z,7E,22E)-(1S,3R)*-1,3-dihydroxy-24-oxo-9,10-seco-cholesta-5,7,10(19)22-tétraène-25-carboxylate de 2-méthylpropyle

*(5Z,7E,22E)-(1S,3R)*-1,3-dihydroxy-24-oxo-9,10-seco-cholesta-5,7,10(19)22-tétraène-25-carboxylate de pentyle

*(5Z,7E,22E)-(1S,3R)*-1,3-dihydroxy-24-oxo-9,10-seco-cholesta-5,7,10(19)22-tétraène-25-carboxylate d'éthyle

*(5Z,7E,22E)-(1S,3R)*-1,3-dihydroxy-24-oxo-9,10-seco-cholesta-5,7,10(19)22-tétraène-25-carboxylate de 1-méthyléthyle

*(5Z,7E,22E)-(1S,3R,24R)*-1,3,24-trihydroxy-26a,27-cyclo-26a-homo-9,10-secocholesta-5,7,10(19)22-tétraène-25-carboxylate d'éthyle

*(5Z,7E,22E)-(1S,3R,24S)*-1,3,24-trihydroxy-26a,27-cyclo-26a-homo-9,10-secocholesta-5,7,10(19)22-tétraène-25-carboxylate d'éthyle

*(5Z,7E,22E)-(1S,3R,24R)*-1,3,24-trihydroxy-26a,27-cyclo-26a-homo-9,10-secocholesta-5,7,10(19)22-tétraène-25-carboxylate de 1-méthyléthyle

*(5Z,7E,22E)-(1S,3R,24S)*-1,3,24-trihydroxy-26a,27-cyclo-26a-homo-9,10-secocholesta-5,7,10(19)22-tétraène-25-carboxylate de 1-méthyléthyle

**23.** Préparations pharmaceutiques contenant au moins un composé de formule générale I selon l'une des revendications 1 à 22, ainsi qu'un support pharmaceutique acceptable.

**24.** Utilisation des composés de formule générale I selon les revendications 1 à 22 pour la préparation de médicaments.